(19) Europäisches Patentamt / European Patent Office / Office européen des brevets

(11) EP 3 152 219 B1

(12) EUROPÄISCHE PATENTSCHRIFT

(45) Veröffentlichungstag und Bekanntmachung des Hinweises auf die Patenterteilung:
**09.01.2019 Patentblatt 2019/02**

(21) Anmeldenummer: **15726612.3**

(22) Anmeldetag: **03.06.2015**

(51) Int Cl.:
***C07H 15/04*** *(2006.01)* ***C11B 1/10*** *(2006.01)*
***C11B 3/06*** *(2006.01)* ***C11B 3/16*** *(2006.01)*
***C11B 7/00*** *(2006.01)* ***C11B 11/00*** *(2006.01)*
***C11C 1/00*** *(2006.01)*

(86) Internationale Anmeldenummer:
**PCT/EP2015/062465**

(87) Internationale Veröffentlichungsnummer:
**WO 2015/185675 (10.12.2015 Gazette 2015/49)**

(54) **VERFAHREN ZUR GEWINNUNG VON GLYCOGLYCEROLIPIDEN UND GLYCOSPHINGOLIPIDEN AUS LIPOIDEN PHASEN**

METHOD FOR OBTAINING GLYCOGLYCEROLIPIDS AND GLYCOSPHINGOLIPIDS FROM LIPOID PHASES

PROCÉDÉS D'OBTENTION DE GLYCOGLYCÉROLIPIDES ET GLYCOSPHINGOLIPIDES À PARTIR DE PHASES LIPOÏDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.06.2014 DE 102014210662**

(43) Veröffentlichungstag der Anmeldung:
**12.04.2017 Patentblatt 2017/15**

(73) Patentinhaber:
- **Nanoscience for life GmbH & Co. KG**
  **65193 Wiesbaden (DE)**
- **GEA Westfalia Separator Group GmbH**
  **59302 Oelde (DE)**

(72) Erfinder:
- **DIETZ, Ulrich**
  **65193 Wiesbaden (DE)**
- **HRUSCHKA, Steffen**
  **59302 Oelde (DE)**

(74) Vertreter: **Arth, Hans-Lothar**
**ABK Patent Attorneys**
**Jasminweg 9**
**14052 Berlin (DE)**

(56) Entgegenhaltungen:
**EP-A1- 2 175 012** **EP-A1- 2 389 816**
**EP-A1- 2 735 605** **EP-A2- 0 730 033**
**WO-A1-2007/069733** **DE-A1- 19 652 813**
**JP-A- 2002 294 274** **US-A- 5 972 928**

- **FANNY ADAM ET AL: "Solvent-free ultrasound-assisted extraction of lipids from fresh microalgae cells: A green, clean and scalable process", BIORESOURCE TECHNOLOGY, ELSEVIER BV, GB, Bd. 114, 18. Februar 2012 (2012-02-18), Seiten 457-465, XP028422603, ISSN: 0960-8524, DOI: 10.1016/J.BIORTECH.2012.02.096 [gefunden am 2012-03-07]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 152 219 B1

## Beschreibung

Die vorliegende Erfindung betrifft ein Verfahren zum Abtrennen von Glycoglycerolipiden als auch von Glycoglycerolipiden und Glycosphingolipiden aus einer lipoiden Phase, welche Glycoglycerolipide und Acylglyceride oder Glycoglycerolipide und Glycosphingolipide und Acylglyceride enthält.

## Hintergrund der Erfindung

Glycolipide, Glycoglycerolipide, Glycosphingolipide und Phospholipide sind biogene Lipide, die als Membranbestandteile in fast allen biologischen Systemen vorkommen und als solche amphiphile Eigenschaften, d.h. eine hydrophile Kopfgruppe und eine hydrophobe bzw. lipoide Schwanzgruppe, besitzen. Das ubiquitäre Vorkommen von Glycolipiden, Glycoglycerolipiden, Glycosphingolipiden und Phospholipiden in praktisch allen Lebewesen erklärt auch, dass aus diesen Lebewesen gewonnenen Lipidextrakte (z.B. Pflanzenöle oder tierische Öle) unvermeidlich auch Glycolipide und Phospholipide enthalten.
Ihre amphiphilen Eigenschaften verleihen den Glycoglycerolipiden und Glycosphingolipiden eine besondere Bedeutung bei der Lösungsvermittlung von hydrophilen Molekülen in Lipidphasen wie z.B. Ölen.
Glycolipide, Glycoglycerolipide, Glycosphingolipide und Glycophospholipide sind aufgrund ihrer amphiphilen Eigenschaften hervorragende Bio-Emulgatoren mit einer hohen Emulsionsleistung. Dies erklärt aber auch, warum sich derartige Lipide mit standardmäßigen wässrigen Extraktionsverfahren nicht oder nur zu einem geringen Anteil abtrennen lassen. Dabei sind Glycoglycerolipide und Glycosphingolipide aufgrund ihrer Eignung als Bio-Emulgatoren bzw. Bio-Tenside von enormem wirtschaftlichem Potenzial. In der praktischen Anwendung betrifft dies insbesondere die Verwendung als Reinigungsmittel zur Entfernung öliger oder fettiger Rückstände. Glycoglycerolipide und Glycosphingolipide eignen sich aber auch für die Emulgierung von lipophilen Wirkstoffen wie z.B. für pharmazeutische Formulierungen oder Pflanzenschutzmitteln, da sie eine Verbesserung der Aufnahme dieser Wirkstoffe durch den Zielorganismus bewirken können. Darüber hinaus bestehen Belege für immunmodulatorische Effekte verschiedener Glycoglycerolipide und Glycosphingolipide, die Bestandteil menschlicher Zellmembranen sind. Ferner werden manchen Glycoglycerolipide und Glycosphingolipide auch anti-bakterielle und fungizide Eigenschaften zugeschrieben. Die emulgierenden Eigenschaften der Glycoglycerolipide und Glycosphingolipide bewirken auch bessere Interaktion lipophiler und hydrophiler Komponenten in Backwaren.

Andererseits haben Glycoglycerolipide und Glycosphingolipide aufgrund ebendieser Eigenschaft als Bio-Emulgator auch ein enormes wirtschaftliches Potenzial. In der praktischen Anwendung betrifft dies insbesondere die Verwendung als Reinigungsmittel zur Entfernung öliger oder fettiger Rückstände.

Folglich ist wirtschaftlich die Gewinnung von Glycoglycerolipiden und Glycosphingolipiden aus biogenen Lipidfraktionen bzw. Ölen von Interesse, weil sie vielfältige Verwendungen in der Lebensmittelindustrie und vor allem in Backwaren und Süßigkeiten haben.

Obwohl davon ausgegangen werden kann, dass in praktisch allen lipoiden Phasen, die aus biogenen Materialien gewonnen werden können, Glycoglycerolipide und Glycosphingolipide unterschiedlicher Art vorliegen, existieren in der wissenschaftlichen Literatur nur wenige Untersuchungen hierzu. Insbesondere sind die Zusammensetzungen in derartigen lipoiden Phasen sowie die Auswirkungen dieser Verbindungen auf die Lösungsvermittlung anderer Verbindungen, die ebenfalls hierin gelöst sind, im Wesentlichen unklar.

Glycoglycerolipide und Glycosphingolipide haben trotz ihres amphiphilen Charakters aufgrund ihrer langen Fettsäurereste eine starke Affinität zu Lipidfraktionen. Daher ist die Verteilung in wässrigen Extraktionsmedien aus dem Stand der Technik nur gering. Flüssigextraktionen wurden hingegen erfolgreich mit Gemischen aus organischen Lösungsmitteln bewerkstelligt. Hierbei ist der Einsatz von Alkoholen entscheidend, um eine hohe Effizienz der Abtrennung herzustellen. Im Labormaßstab gelingt die Abtrennung von Glycoglycerolipiden und Glycosphingolipiden durch Chromatographie mit anschließender Lösungsmittelextraktion der adsorbierten Glycolipide. Solche adsorptiven Verfahren eignen sich aus ökonomischen und ökologischen Gründen jedoch nicht für einen industriellen Maßstab. Das Patent US6953849 beschreibt die Extraktion von Glycoglycerolipiden aus Reiskeim-Öl mittels heißem Wasserdampf. Hierdurch werden Zuckerreste abgespalten, wodurch sich Teile dieser Verbindungen mit der Wasserdampffraktion antrennen lassen. Nachteilig ist z.B. bei einer derartigen Behandlung von Speiseölen, dass es bei den hierbei angewandten Dampftemperaturen und der Dauer dieser Dampfexposition zu einem erhöhten Anteil an trans-Fettsäuren in dem Speiseöl kommen kann, wodurch derartig gewonnene Speiseöle gesundheitsschädlich werden können. Ferner sind im Stand der Technik enzymatische Methoden zur Entfernung von Glycoglycerolipiden und Glycosphingolipiden aus Lipidphasen bekannt. Hierdurch werden die Glycoglycerolipide und Glycosphingolipide jedoch in ihrer Struktur verändert, was die spätere Nutzung der abgetrennten Glycolipidfraktion stark einschränkt oder sie für weitere Anwendungen unbrauchbar macht. Folglich existiert bisher kein Verfahren, mit dem eine kontinuierliche und schonende Gewinnung größerer Mengen an biogenen Glycoglycerolipiden und Glycosphingolipiden möglich ist.

In lipoiden Phasen, die als solche durch ein Raffinationsverfahren von Begleitstoffen befreit werden sollen, ist

die Abtrennung der Glycoglycerolipide und Glycosphingolipide per se zwar nicht erforderlich, da diese in der Regel als unpolare lipophile Verbindungen zu keiner relevanten Qualitätseinschränkung (Farbe, Geruch, Transparenz) der raffinierten lipoiden Phasen bei einem Verbleib führen, sofern es sich um kleine Mengenanteile handelt. Störend ist jedoch ihr starkes Bindungsvermögen für Wasser, Erdalkalimetall- und Metallionen, deren Anwesenheit in einer raffinierten lipoiden Phase, z. B. einem Pflanzenöl nicht erwünscht ist. Auch die Abreicherung der o.g. Stoffe ist aus einer lipoiden Phase, die stark mit Glycoglycerolipiden und Glycosphingolipiden belastet ist, mit den konventionellen Techniken problematisch.

Nach dem Stand der Technik werden lipoide Phasen zum Zwecke einer technischen Raffination zumeist einem soganannten Entschleimungsverfahren (Degumming) unterzogen, um hydratisierbare Verbindungen in eine Wasserphase zu überführen oder über eine Verseifung von Fettsäuren eine Aggregation zu bewirken, wodurch sich die gelösten oder aggregierten Verbindungen mit Verfahren zur Phasenseparation abtrennen lassen. Durch diese Verfahren wird der größte Teil hydratisierbarer und ein Teil nicht hydratisierbarer Phospholipide abgetrennt. Glycoglycerolipide und Glycosphingolipide werden dabei teilweise durch eine Hydrolyse degradiert und mit der Phospholipidfraktion entfernt. Bei lipoiden Phasen, die nach Standard-Degummingverfahren aufgereinigt wurden, kann durch eine intensive Einmischung einer Wasserphase je nach Gehalt an Glycolipidverbindungen eine Emulsion bewirkt werden, die eine anschließende Phasenseparation mittels Zentrifugalkraft nur noch teilweise oder gar nicht mehr erlaubt. Es konnte gezeigt werden, dass ein weiterer Aufreinigungsschritt unter Normalbedingungen (Raumtemperatur und Normaldruck) entweder mit einer Alklalilauge oder einer Säure (Zitronen- oder Phosphorsäure) auch nach intensivem Mischeintrag zu keiner relevanten Entfernung weiterer Glycoglycerolipide und Glycosphingolipide in der Lage ist, wenn eine erneute Abtrennung mittels eines Separators im Anschluss erfolgt.

Aus den bekannten Eigenschaften der Glycoglycerolipide und Glycosphingolipide kann allerdings angenommen werden, dass es zu einer Wasserbindung an den OH-Gruppen der Zuckerreste kommt, wodurch auch kleinste Mengen an Wasser ohne Ausbildung von Micellen gebunden werden können. Über den gleichen Mechanismus werden Erdalkalimetall- und Metallionen gebunden. Dabei ist anzunehmen, dass Glycoglycerolipide und Glycosphingolipide mit mehreren und komplexen Zuckereinheiten mehr und komplexer Wasser und Metallionen binden können. Es ist ferner anzunehmen, dass auch Glycoglycerolipide und Glycosphingolipide in einer lipoiden Phase dazu tendieren, micellare Strukturen auszubilden. Sofern Wasser und Metallionen an Zuckerresten gebunden sind, wird eine Herauslösung dieser Stoffe durch ein wässriges Medium weitgehend dadurch verhindert, dass lange unpolare Fettsäurereste ein Eindringen von Wasser an diese Strukturen stark erschweren und durch die elektrostatischen Wechselkräfte mit den OH-Gruppen der Zuckereste ein "Herausspülen" der Ionen verhindert wird. Dies erklärt, warum es nach dem Stand der Technik bisher erforderlich ist, die Fraktion der Glycoglycerolipide und Glycosphingolipide mittels chemischer oder enzymatischer Hydrolyse oder destillativer Verfahren zu entfernen, wodurch sich dann auch der Gehalt an in einer lipoiden Phase noch gebundenem Wasser sowie Erdalkalimetall- und Metallionen auf das erforderliche Maß reduzieren lässt. Daher ist es umso überraschender, dass die stark hydrophilen Salz-Verbindungen, die in der erfindungsgemäßen Applikationsform mit einer Wasserhülle vorliegen, eine Hydophilisierung von Glycoglycerolipiden und Glycosphingolipiden bewirkt, die eine Abtrennung in ein wässriges Medium ermöglicht. Darüber hinaus zeigte sich unerwartet und überraschend, dass es sich bei den abgetrennten Galaktosydiglyceriden um Verbindungen handelte, die aufgrund ihrer hydrophilen-lipophilen Balance eine hohe Affinität zum lipoiden Medium, in dem sie sich befunden haben, hatte. Es ist daher sehr wahrscheinlich, dass durch den erfindungsgemäßen Eintrag der wässrig gelösten Salze und dem hiermit erfolgten Wassereintrag ein Zusammenschluss von Glycoglycerolipiden und Glycosphingolipiden als micellare Strukturen ermöglicht wird, wodurch sich diese mittels einer gravitativen Separation aus einer lipoiden Phase abtrennen lassen. Dies erklärt allerdings nicht vollständig die unerwartete erhebliche Steigerung der Extraktionsleistung des erfindungsgemäßen Verfahrens bei Verwendung eines Intensivmischeintrages der erfindungsgemäßen wässrigen Medien.

Glycoglycerolipide und Glycosphingolipide, die aus liopiden Phasen durch extraktive Prozesse gewonnen wurden, weisen zumeist stark unterschiedliche Strukturen und Zusammensetzungen auf. Häufig bleiben sie mit anderen Strukturen über hydrophobe oder hydrophile Wechselwirkungen verbunden, für die sie als "Lösungsvermittler" in der lipoiden Phase gedient haben. Dies könnte begründen, warum sich Teile der Glycolipidfrakion erst durch organische Lösungsmittel von Strukturen, an die sie elektrostatisch gebunden sind, lösen lassen oder eine Herauslösung der Glycoglycerolipide und Glycosphingolipide zusammen mit den elektrostatisch gebundenen Strukturen erfolgt. Hierdurch ließe sich auch erklären, warum in manchen erfindungsgemäß hergestellten glycolipidreichen Extraktionsphasen eine große Anzahl von bisher nicht aufgeklärten Verbindungen gefunden werden konnte. Eine dieser nicht-Glycolipidverbindungen, die mit dem erfindungsgemäßen Verfahren abgetrennt werden, sind z.B. Phorbolester.

Aus den vorgenannten Aspekten ist es um so erstaunlicher, dass es mit dem erfindungsgemäßen Intensiveintrag der Salzlösungen möglich wird, die in den lipoden Phasen enthaltenen Glycolipidfraktionen bereits mit einem einzigen wässrigen Extraktionsschritt zu entfernen oder bei lipoiden Phasen, die einen sehr hohen Gehalt an Glycoglycerolipiden und Glycosphingolipiden aufweisen, diese mit weniger Extrationsschritten entfernen zu können als dies der Fall ist bei einem niedrigenergetischen Eintrag der wässrigen Lösungen in die lipoiden Phasen. Als weiterer unerwarteter Effekt zeigte sich, dass die Entfernung der Glycolipidfraktion in einer besonders vorteilhaften Reduktion des Bindungsvermögens von Wasser und Elektrolyten in der so behandelten lipoiden Phase resultiert. Ferner kommt es als besonders vorteilhafter Effekt bei subsequent durchgeführten Abtrennungen der lipoiden Phasen mit wässrigen Medien praktisch zu keiner Schaumbildung. Auch zeigte sich überraschenderweise, dass wässrige Lösungen von Guanidin- oder Amidinverbindungen, die mit einer erfindungsgemäß behandelten lipoiden Phase gemischt werden, sich von dieser deutlich besser durch eine zentrifugale Separation abtrennen lassen, wenn dies im Anschluss an die erfindungsgemäße Abtrennung von Glycoglycerolipiden und Glycosphingolipiden mit einen Intensiveintrag der wässrigen Salz-Lösungen erfolgt.

Im Stand der Technik wird beispielsweise in WO 2012/109642 A1 die Abtrennung von Lipiden aus Ölen mittels einer Natriumchloridlösung offenbart. Ungeachtet der unerwünschten korrosiven Eigenschaften von Chloridsalzen wie z.B. Natriumchlorid, welches die verarbeitenden Vorrichtungen angreift und korrodiert, konnten die Erfinder belegen, dass nur eine spezielle Auswahl an Anionen wie hierin offenbart für die erfindungsgemäße Abtrennung von Glycoglycerolipiden und Glycosphingolipiden eingesetzt werden kann und Anionen wie z.B. Chlorid, Bromid, Iodid, Nitrat, Nitrit, Sulfat, Phosphat und viele andere nicht in der Lage sind, die erfindungsgemäße Aufgabe zu lösen.
EP 2 735 605 A1 beschreibt die Abtrennung von Rhamnolipiden durch Extraktion mittels eines organischen Lösungsmittels. Bei diesem Verfahren werden die Rhamnolipide aus einer wässrigen Phase in eine organische Phase überführt und nicht aus einer lipoiden Phase abgetrennt. Zudem finden bei der Abtrennung keine Salze Anwendung.

Es ist deshalb Aufgabe der vorliegenden Erfindung, eine Vorrichtung und ein Verfahren zum Abtrennen von Glycoglycerolipiden oder Glycoglycerolipiden und Glycosphingolipiden aus einer lipoiden Phase, welche unter anderem Glycoglycerolipide und Acylglyceride oder welche Glycoglycerolipide und Glycosphingolipide und Acylglyceride enthält, bereitzustellen.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

**Beschreibung**

Überraschenderweise wurde jetzt gefunden, dass es möglich ist, mit wässrigen Lösungen, enthaltend mindestens ein in Wasser bei 20°C gut lösliches Salz, welches bei der Dissoziation in Wasser Carbonat- ($CO_3^{2-}$), Hydrogencarbonat- ($HCO_3^-$), Metasilicat-($SiO_3^{2-}$), Orthosilicat- ($SiO_4^{4-}$), Disilicat- ($Si_2O_5^{2-}$), Trisilicat- ($Si_3O_7^{2-}$) , Acetat- ($CH_3COO^-$), Borat- ($BO_3^{3-}$) oder Tartrationen ($C_4H_4O_6^{2-}$) bildet und welche unter intensivem Mischungseintrag mit einer lipoiden Phase in Kontakt gebracht wurden, Glycoglycerolipide in ein wässriges Medium überführbar zu machen und dabei ihre chemische und strukturelle Integrität zu bewahren.
"Gut wasserlöslich" beeutet in diesem Zusammenhang vorzugsweise eine Löslichkeit von mindestens 30 g/L in Wasser bei 20°C. Der zur Abtrennung von Glycoglycerolipiden und Glycosphingolipiden erfoderliche Eintrag einer wässrigen Lösung mit den oben genannten Verbindungen konnte bereits mittels einer Rührvorrichtung vorgenommen werden. Dabei zeigte sich, dass in Abhängigkeit der Rührdauer, ohne oder mit nur einer geringen Erwärmung der Suspension oder Emulsion, es zu einer spontanen Phasentrennung kommt, wobei in der Wasserphase Trübstoffe gelöst sind. Während nach Abtrennung einer so behandelten lipoiden Phase durch einen erneuten Rühreintrag einer wässrigen Lösung mit den oben genannten Verbindungen sich praktisch keine Trübstoffabtrennung mehr erzeugen ließ, konnte überraschenderweise durch eine Intensivmischung der so vorbehandelten lipoiden Phasen mit Lösungen der oben genannten Verbindungen eine erhebliche Abtrennung von Trübstoffen in die Wasserphase bewirkt werden. Bei einer Wiederholung eines intensiven Rühreintrags einer Lösung mit den oben genannten Verbindungen in die derartig vorbehandelte lipoide Phase ließen sich praktisch keine Nicht-Triglyceride mehr abtrennen. Wenn die so vorbehandelte lipoide Phase erneut mit Wasser intensiv gemischt wurde, kam es ferner zu keiner oder nur einer sehr geringen Emulsionsbildung der lipoiden Phasen. Somit konnte zum ersten Mal gezeigt werden, dass durch einen intensiven Mischeintrag einer wässrigen Lösung der oben genannten Verbindungen in lipoide Phasen, bei denen eine weitgehende Entfernung von hydratisierbaren Phospholipiden und freien Fettsäuren bereits erfolgt ist, sich signifikante Mengen an hierin verbliebenen Glycoglycerolipiden und Glycosphingolipiden herauslösen und mittels zentrifugaler Verfahren separieren lassen
Darüber hinaus zeigte sich völlig unerwartet, dass es bei einer mittels Intensivmischung mit Salzen, welche in Wasser bei 20°C eine Löslichkeit von vorzugsweise mindestens 30 g/L besitzen und bei der Dissoziation in Wasser Carbonat- ($CO_3^{2-}$), Hydrogencarbonat-($HCO_3^-$), Metasilicat- ($SiO_3^{2-}$), Orthosilicat- ($SiO_4^{4-}$), Disilicat- ($Si_2O_5^{2-}$), Trisilicat- ($Si_3O_7^{2-}$), Acetat- ($CH_3COO^-$), Borat- ($BO_3^{3-}$) oder Tartrationen ($C_4H_4O_6^{2-}$) bilden gewonnenen glycoglycerolipidhaltigen wässrigen Fraktionen zu keiner relevanten Mitabtrennung von Triacylglyceriden kommt, wie sich bei dünnschichtchromatographischen Analysen herausstellte. Dies ist umso überraschender, als dass sich mit der so abgetrennten glycoglycerolipidhaltigen Fraktion durch Hinzugabe von Wasser eine stabile nicht ölige Emulsion herstellen lässt, die ein sehr großes Emulgiervermögen gegenüber einer aufgereinigten Triayclglyceridphase aufweist. Somit kann erstmals ein wässriges Abtrennverfahren bereitgestellt werden, mit dem sich eine weitestgehend vollständige Separation von nicht hydrolysierten Glycoglycerolipiden aus lipoiden Phasen bewerkstelligen lässt.

Die erfindungsgemäße Zusammenbringung der wässrigen Phasen mit hierin gelösten Salzen in Verbindung mit einem intensiven Eintrag, der eine Separation der Glycoglycerolipide, die an andere Strukturen elektrostatisch gebunden sind, bewirkt, kann auch durch einen geringen Mischeintrag, der aber bei erhöhter Temperatur der lipoiden Phase erfolgt, erreicht werden. Hierdurch kommt es allerdings zu einem raschen hydrolytischen Abbau der Glycoglycerolipide, was für die Gewinnung und Verwertung dieser Fraktion nicht gewünscht ist. Durch die Verwendung des erfindungsgemäßen Intensiveintrages der gelösten Salze können weitgehend alle in einer lipoiden Phase befindlichen Glycoglycerolipide herausgelöst werden, ohne dass hierfür eine Erhöhung der Temperatur der lipoiden Phase erforderlich ist. Hierdurch wird die Gewinnung von strukturell nicht veränderten Glycoglycerolipiden ermöglicht.

Da nach der technischen Lehre dieser Erfindung lange Kontaktzeiten der abzutrennenden Glycoglycerolipide mit den wässrigen Medien eine Hydrolyse begünstigt und hierdurch auch höhere Prozesskosten zu erwarten sind, ist die besonders bevorzugte Ausführungesform für die Zusammenbringung der wässrigen und der lipoiden Phasen die Anwendung eines Intensivmischers, der innerhalb kurzer Zeit eine Intensivmischung bewerkstelligen kann. Der technischen Lehre kann auch entnommen werden, dass es im Rahmen einer solchen Intensivmischung zu einem Eintrag von Luft oder Gasen (z. B. durch Entmischungsprozesse) kommen kann, die dann zu einer sehr stabilen Emulsionsbildung führt, die eine Separation der Glycoglycerolipide und Glycosphingolipide weitgehend behindert, da eine Phasentrenung einer Luft/Gas enthaltenden Emulsion durch Zentrifugation nicht möglich ist. Es ist anzunehmen, dass die Glycoglycerolipide selbst zu der Entstehung von stabilen Grenzflächen zwischen einer Gas- und einer Flüssigkeitsphase beitragen, da eine derartige Emulsionsbildung nach Abtrennung der Glycolipidfraktion nicht oder nur in geringem Ausmaß beobachtet werden kann. Um also den besonders vorteilhaften Effekt, den ein intensiver Mischvorgang mit den erfindungsgemäßen wässrigen Salzlösungen auf die Abtrennbarkeit von Glycoglycerolipiden und Glycosphingolipiden hat, auszunutzen, ohne dabei auf ein möglicht hydrolysefreies verwertbares Glycolipidgemisch verzichten zu müssen bei gleichzeitig ökonomischem Prozessablauf, ist die vorliegende Anmeldung auf die Verwendung einer Abtrennvorrichtung gezielt, die sowohl bei der Intensivmischung der liopiden Phasen und der erfindungsgemäßen wässrigen Lösungen als auch während des anschließenden Phasenseparationsvorgangs ein Ausschluss eines Lufteintrags oder einer Gasbildung gewährleistet.

Daher ist es besonders vorteilhaft wenn der erfindungsgemäße Intensiveintrag der wässrigen Lösungen in eine lipoide Phase unter Ausschluss eines Luft-/Gaseintrags erfolgt.
Ferner ist es besonders vorteilhaft, wenn ein Separator die mit dem erfindungsgemäßen Verfahren hergestellten Gemische, bestehend aus einer lipoiden Phase und einer erfindungsgemäßen wässrigen Lösung, unter Ausschluss eines Luft-/Gaseintrags voneinander trennt.

Die vorliegende Offenbarung betrifft eine Vorrichtung zum Abtrennen von Glycoglycerolipiden aus einer lipoiden Phase, welche Glycoglycerolipide und Acylglyceride enthält, wobei die Vorrichtung einen Intensivmischer zur Aufnahme der lipoiden Phase, eine Kavität mit Zuleitung zum Intensivmischer zur Aufnahme einer wässrigen Phase, enthaltend Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO4^{4-}$), Disilicat ($Si_2O_5^{2-}$), Trisilicat ($Si_3O_7^{2-}$), Acetat ($CH_3COO^-$), Borat ($BO_3^{3-}$) und/oder Tartrat ($C_4H_4O_6^{2-}$) bildet sowie eine Zentrifuge mit Zuleitung zum Intensivmischer umfasst.

Die wässrige Phase enthält natürlich auch Kationen, so dass die vorliegende Offenbarung eine Vorrichtung zum Abtrennen von Glycoglycerolipiden aus einer lipoiden Phase betrifft, welche Glycoglycerolipide und Acylglyceride enthält, wobei die Vorrichtung einen Intensivmischer zur Aufnahme der lipoiden Phase, eine Kavität mit Zuleitung zum Intensivmischer zur Aufnahme einer wässrigen Phase enthaltend Kationen und Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO4^{4-}$), Disilicat ($Si_2O_5^{2-}$), Trisilicat ($Si_3O_7^{2-}$), Acetat ($CH_3COO^-$), Borat ($BO_3^{3-}$) und/oder Tartrat ($C_4H_4O_6^{2-}$) bildet sowie eine Zentrifuge mit Zuleitung zum Intensivmischer umfasst.

Sollte die lipoide Phase neben den Glycoglycerolipiden auch noch Glycosphingolipide enthalten, so können diese zusammen mit den Glycoglycerolipiden aus der lipoiden Phase abgetrennt werden, ohne dass es dafür einer anderen Vorrichtung bedarf. In einem solchen Falle betrifft die vorliegende Offenbarung eine Vorrichtung zum Abtrennen von Glycoglycerolipiden und Glycosphingolipiden aus einer lipoiden Phase, welche Glycoglycerolipide, Glycosphingolipide und Acylglyceride enthält, wobei die Vorrichtung einen Intensivmischer zur Aufnahme der lipoiden Phase, eine Kavität mit Zuleitung zum Intensivmischer zur Aufnahme einer wässrigen Phase enthaltend Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$) , Acetat ($CH_3COO^-$), Borat ($BO_3^{3-}$) und/oder Tartrat ($C_4H_4O_6^{2-}$) bildet sowie eine Zentrifuge mit Zuleitung zum Intensivmischer umfasst.

Entsprechend betrifft die vorliegende Offenbarung eine Vorrichtung zum Abtrennen von Glycoglycerolipiden und Glycosphingolipiden aus einer lipoiden Phase, welche Glycoglycerolipide, Glycosphingolipide und Acylglyceride enthält, wobei die Vorrichtung einen Intensivmischer zur Aufnahme der lipoiden Phase, eine Kavität mit Zuleitung zum

Intensivmischer zur Aufnahme einer wässrigen Phase enthaltend Kationen und Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO4^{4-}$), Disilicat ($Si_2O_5^{2-}$), Trisilicat ($Si_3O_7^{2-}$), Acetat ($CH_3COO^-$), Borat ($BO_3^{3-}$) und/oder Tartrat ($C_4H_4O_6^{2-}$) bildet sowie eine Zentrifuge mit Zuleitung zum Intensivmischer umfasst.

Mischen und Homogenisieren

Wie die technische Lehre dieser Anmeldung zeigt, ist die Effizienz des Verfahrens zur Abtrennung von Glycoglycerolipiden aus einer lipoiden Phase auch davon abhängig, wie vollständig die Durchmischung letzterer mit den in der Wasserphase gelösten Verbindungen ist. Da die Bereitstellung der wässrigen Salzlösungen bereits in einem äquimolaren Verhältnis mit den Glycoglycerolipiden ausreichen kann, um letztere aus der lipoiden Phase zu extrahieren und um den Abtrennprozess technisch einfach zu gestalten, ist die Zugabe einer geringen Wassermenge, die die Verbindungen enthält, ausreichend. Da die zu mischenden Flüssigkeiten stark gegensätzliche Eigenschaften aufweisen (hydrophil - hydrophob), ist zu Herstellung möglichst großer Grenzflächen zwischen beiden Flüssigkeiten ein erheblicher Energieaufwand notwendig. Nach dem Stand der Technik stehen hierfür verschiedene Techniken zur Verfügung: dynamische Mischverfahren, die auf laminaren oder turbulenten Strömungen der Mischkomponenten basieren, oder statische Verfahren, bei denen lokale Druck-/Spannungsgradienten erzeugt werden, die zu einer Grenzflächenausbildung führen. Aus der Literatur ist bekannt, dass die kritische Weber-Zahl in laminaren Dehn- und Scherströmungen und Mischströmungen vom Viskositätsverhältnis $\lambda$ zwischen disperser und kontinuierlicher Phase für einzelne Tropfen abhängig ist. Hieraus folgt, dass bei den üblicherweise hochviskosen lipoiden Phasen eine auf einer laminaren Strömung basierende Mischung nicht geeignet ist. Bei turbulenten Strömungen erfolgt der Srömungsfortschritt unstetig, scheinbar regellos, zufällig und chaotisch, somit ist eine zeitliche und örtliche Auflösung nicht vorhersehbar. Aufbauend auf dem Modell von Kolmogorov (1949) wurden unterschiedliche Modelle entwickelt, um die Grenzflächeninteraktionen in turbulenten Strömungen am einzelnen Tropfen zu simulieren [Rodriguez-Rodriguez et al., 2006; Gordillo et al., 2006] und im Kollektiv zu beschreiben [Hinze, 1955; Davies, 1985; Vankova et al., 2007]. Die Modelle unterscheiden sich hauptsächlich in den Geräten und Stoffsystemen, für die sie entworfen wurden, und damit verbunden in den Annahmen über die turbulente Strömung. Die Variation des Aufbruchmechanismus wurde erreicht durch das gezielte Einstellen von unterschiedlichen Reynolds-Zahlen, Viskositäten der Phasen, Dichten der Phasen, Grenzflächenspannungen und Dispersphasenanteilen. Da mit dem Kolmogorov-Modell aber nur vorhergesagt werden kann, welcher Tropfen nicht mehr aufgebrochen wird, nicht aber wie klein die Tropfen beim Aufbruch werden, kann mit diesem Modell nur eine obere Partikelgröße berechnet werden.

Kavitation entsteht in Flüssigkeiten durch die Erzeugung von Blasen, die anschließend wieder kollabieren. Generell werden die drei Kavitationsarten Dampfkavitation (harte Kavitation), Gaskavitation (weiche Kavitation) und Pseudokavitation unterschieden [Riedel, 1973]. Bei der harten Kavitation entstehen die Blasen durch Absinken des statischen Drucks unter den Dampfdruck, wodurch das Fluid teilweise verdampft und sich Dampfblasen bilden. Bei der weichen Kavitation wird durch Absenken des statischen Drucks die Löslichkeit von Gasen gesenkt, so dass diese Blasen bilden. Wenn in einer Flüssigkeit bereits Blasen vorliegen, führt das Absinken des Drucks zum Wachstum dieser Blasen, was es als Pseudokavitation bezeichnet wird. Sobald der Druck wieder über den Dampfdruck steigt, kommt es zu einem schlagartigen Kondensieren der Flüssigkeit und somit im Extremfall zum Kollabieren der Blasen, was zu hohen Druckschwankungen führt. Welche Spannungen aus der Kavitation resultieren und welcher Mechanismus somit zum Tropfenaufbruch führt, ist bis heute nicht endgültig geklärt. Somit stehen zwar verschiedene Methoden, die einen intensven Mischeintrag ermöglichen, zur Verfügung, für die Mischung der Fluiden Phasen ist aber weder eine Berechnung noch eine Annahme über die Effektivität derartiger Verfahren für Extrahierbarkeit gelöster Glycoglycerolipide und Glycosphingolipide möglich und daher nur empirisch eruierbar.

Die Verfahren, die für Erzeugung von Grenzflächen zwischen zwei Fluiden geeignet sind, können in die vier Obergruppen Rotor-Stator-, Hochdruck-, Ultraschall- und Membransysteme unterteilt werden [Schubert, 2005]. Die einfachste Variante eines Rotor-Stator-Systems ist der Rührer in einem Behälter. Weiterentwicklungen der Rotor-Stator-Systeme sind Zahnkranzdispergiermaschinen und Kolloidmühlen, die sich dadurch auszeichnen, dass sie deutlich definierte Beanspruchungen ermöglichen. Ein Nachteil von Rotor-Stator-Systemen besteht darin, dass die Energie häufig stark inhomogen eingetragen wird, was zu breiten Tropfengrößenverteilungen oder langen Prozesszeiten führt. Weiterhin sind häufig nur niedrige spezifische Energieeinträge realisierbar. Hochdruckhomogenisatoren werden insbesondere dann eingesetzt, wenn sehr hohe spezifische Energieeinträge benötigt werden. Hochdruckhomogenisatoren bestehen im Wesentlichen aus einer Hochdruckpumpe und einer Zerkleinerungseinheit. Als Hochdruckpumpen werden üblicherweise Kolbenpumpen eingesetzt, die einen Homogenisierdruck zwischen 50 und 10.000 bar erzeugen. Die Zerkleinerungseinheit kann aus Ventilen oder Blenden bestehen, durch die die mit Druck beaufschlagten Fluide gepresst werden. Die hierdurch erzeugten Spannungen zwischen den Fluiden sind für eine Tropfenentstehung und Tropfendeformation sowie -zerkleinerung verantwortlich. Die resultierenden Effekte auf diese Eigenschaften werden von den Stoffeigenschaften der Fluide (wie Viskositäten der Phasen, Grenzflächenaufbau, Aktivität des grenzflächenaktiven Materials) sowie dem Druckgradienten und der Geometrie des Zerkleinerungsgeräts bestimmt. Deformation und Aufbruch werden maßgeblich vom Viskositätsverhältnis λ zwischen der dispersen und der kontinuierlichen Phase bestimmt [Walstra, 1998; Kaufmann, 2002; Aguilar et al., 2004]. Insbesondere für höhere Viskositätsverhältnisse λ ist die Dehnströmung im Ventileinlauf vorteilhaft, da die aus der Turbulenz und der Kavitation resultierenden Spannungen an den Filamenten besser wirken und somit feine Tropfen bei möglichst geringem Energieeintrag hergestellt werden können.

Bei Membranen und mikrostrukturierten Systemen werden zumeist vorgemischte fluide Phasen verwendet, bei denen durch den Porendurchtritt die Tropfen aufgebrochen werden, womit eine noch engere Tropfengrößenverteilung herstellt werden kann als bei Hochdruckhomogenisierungsgeräten, jedoch sind bis heute keine hohen Volumenströme bei vertretbaren Kosten erreichbar.

Aus dem Stand der Technik sind somit verschiedene Verfahren und Vorrichtungen bekannt, die eine intensive Mischung von Fluiden ermöglichen. Da das Mischergebnis von einer großen Anzahl von Einflussparametern abhängt, lassen sich das Mischergebnis und die damit verbundenen Auswirkungen auf die chemischen und physikalischen Interaktionen der hierin enthaltenen Verbindungen nicht vorhersagen. Es war daher überraschend, dass mit einem Zahnkranzdispersionswerkzeug eine sehr viel größere Menge an Glycoglycerolipiden aus einer lipoiden Phase extrahiert werden konnte als mittels eines Rotorsystems. Dieser deutliche Unterschied konnte allerdings erst durch den Ausschluss einer Luft-/Gasblasenbildung erzielt werden.

Es ist daher auch die Aufgabe ein Misch- und Separatorsystem bereitzustellen, mit dem ohne einen Luft-/Gaseintrag eine hydrolysearme oder hydrolysefreie Intensivmischung der wässrigen Salzlösungen mit einer lipoiden Phase hergestellt werden kann. Als geeignete Intensivmischer können vor allem solche Intensivmischer genannt werden, die nach dem Hochdruck- oder Rotor-Stator-Homogenisierungprinzip arbeiten.

Die wässrige Phase, in der die oben genannten Salze bzw. Anionen in gelöster Form vorliegen, ist in einer Kavität oder einem Vorratsbehälter enthalten, der mit einer Zuleitung mit dem Intensivmischer verbunden ist, so dass über diese Zuleitung eine definierte Menge bzw. ein definiertes Volumen der wässrigen Phase in den Intensivmischer eingeleitet werden kann.

In dem Intensivmischer findet dann eine intensive Durchmischung der lipoiden Phase und der wässrigen Phase statt. Die intensive Durchmischung findet bei Atmosphärendruck und einer Temperatur im Bereich von 10°C bis 90°C, bevorzugt 15°C bis 70°C, weiter bevorzugt 20°C bis 60°C und insbesondere bevorzugt 25°C bis 50°C, statt. Daher erfolgt die Durchmischung und vorzugsweise intensive Durchmischung bei niedriger Temperatur von vorzugsweise unterhalb 70°C, weiter bevorzugt von unterhalb 65°C, weiter bevorzugt von unterhalb 60°C, weiter bevorzugt von unterhalb 55°C, noch weiter bevorzugt von unterhalb 50°C, noch weiter bevorzugt von unterhalb 45°C, statt. Schutzgas, Unterdruck oder Überdruck oder auch Lichtausschluss sind weder bei der Durchmischung noch bei der anschließenden Aufarbeitung erforderlich. Die niedrigen Temperaturen bei der Durchmischung als auch bei der nachfolgenden Trennung beispielsweise mittels Zentrifugation und der nachfolgenden Aufarbeitung sorgen dafür, dass keine Hydrolyse stattfindet. Somit ist die vorliegende Erfindung auch auf ein hydrolysefreies oder zumindest hydrolysearmes Verfahren zur Abtrennung von Glycoglycerolipiden und Glycosphingolipiden aus lipoiden Phasen gerichtet.

Unter dem Begriff "hydrolysefrei" wird eine Hydrolyse der Glycoglycerolipide und Glycosphingolipide in der lipoiden Phase von weniger als 1,0 Gew.-%, bevorzugt von weniger als 0,5 Gew.-%, bezeichnet.

Unter dem Begriff "hydrolysearm" wird eine Hydrolyse der Glycoglycerolipide und Glycosphingolipide in der lipoiden Phase von weniger als 10,0 Gew.-%, bevorzugt von weniger als 5,0 Gew.-%, weiter bevorzugt von weniger als 3,0 Gew.-%, bezeichnet. Natürlich gewährleistet diese schonende Abtrennung der Fraktion, enthaltend die Glycoglycerolipide oder die Glycoglycerolipide und Glycosphingolipide, auch, dass die anderen Bestandteile der lipoiden Phase wie beispielsweise die Glycolipide, Phospholipide und Triacylglyceride, Diacylglyceride und Monoacylglyceride nicht hydrolysiert werden.

Daher ist insbesondere bevorzugt, wenn das gesamte erfindungsgemäße Verfahren vorzugsweise einschließlich der optionalen Schritte bei Temperaturen im Bereich von 10°C bis 90°C, bevorzugt 13°C bis 80°C, bevorzugt 15°C bis 70°C, weiter bevorzugt 18°C bis 65°C, weiter bevorzugt 20°C bis 60°C, weiter bevorzugt 22°C bis 55°C und insbesondere bevorzugt 25°C bis 50°C oder 25°C bis 45°C, durchgeführt wird.

Die intensiv durchmischte lipoide Phase und wässrige Phase werden dann in eine Zentrifuge überführt und in eine abzutrennende wässrige glycoglycerolipidreiche Phase und eine lipoide glycoglycerolipidarme Phase getrennt. Wässrige glycoglycerolipidreiche Phase und lipoide glycoglycerolipidarme Phase werden dann voneinander separiert. Unter dem Begriff "intensiv durchmischt" wird eine mechanische/physikalische Durchmischung mit einem Intensivmischer verstanden oder eine derartige Durchmischung, dass lipoide Phase und wässrige Phase eine homogene Emulsion oder Dispersion bilden.

In einer bevorzugten Ausführungsform kann die lipoide glycoglycerolipidarme Phase erneut mit einer wässrigen Phase, enthaltend mindestens eine Verbindung, welche mindestens eine Amidinogruppe und/oder mindestens eine Guanidinogruppe aufweist, versetzt und erneut durchmischt werden, falls die lipoide glycoglycerolipidarme Phase Fettsäuren oder Carbonsöuren enthält, welche abgetrennt werden sollen.

Die wässrige Phase, enthaltend mindestens eine Verbindung, welche mindestens eine Amidinogruppe und/oder mindestens eine Guanidinogruppe aufweist, wird aus einem Vorratsbehälter oder einer Kavität, die mit einer Zuleitung verbunden ist, zugeführt. Nach Durchmischung erfolgt erneut die Überführung des Gemisches in eine Zentrifuge, wo die wässrige Phase (d.h. die fettsäurenreiche oder carbonsäurenreiche Phase) von der lipoiden glycoglycerolipidarmen Phase (d.h. die fettsäurenarme oder carbonsäurenarme Phase) getrennt und separiert wird, um eine weiter carbonsäurearme und glycoglycerolipidarme lipoide Phase zu erhalten.

Die (schematisch dargestellte) Vorrichtung gemäß Fig. 3 weist ein Aufnahmegefäß 1 für die Aufnahme der wässrigen Phase bzw. der Salzlösung der hierin beschriebenen Salze auf. Von dem Aufnahmegefäß 1 führt eine Leitung 2 (in welche hier eine Pumpe 14 geschaltet ist) zu einem Behälter 3. Dieser Behälter 3 ist vorzugsweise als Konstantdruck-Pufferbehälter ausgelegt. Hierzu kann der Behälter 3 eine Überlaufrückführung 4 aufweisen, die dazu dient, beim Überschreiten eines Überlaufpegels Flüssigkeit aus dem Behälter 2 in das Aufnahmegefäß 1 zurückzuleiten. Der Behälter 3 weist ferner eine Ablaufleitung 5 auf (vorzugsweise an seinem unteren Ende), in welche hier ein Ventil 6 geschaltet ist. Mit dem Ventil 6 kann der Volumenstrom in der Ablaufleitung 5 gesteuert werden. Die Ablaufleitung mündet in einen Mischer 7. In den Mischer 7 führt zudem eine Zuleitung 8, in welche eine Pumpe 13 geschaltet sein kann. Durch die Zuleitung 8 kann eine weitere Phase, vorzugsweise die lipoidhaltige (lipide) Phase in den Mischer 7 geleitet werden. Der Mischer 7 weist ferner eine Ablaufleitung 9 auf, welche in einen Zulauf einer Zentrifuge 10 mündet. In dem Mischer 7 werden die beiden zugeleiteten Phasen vermischt.

In der Zentrifuge 10 erfolgt eine zentrifugale Trennung in zwei Phasen unterschiedlicher Dichte, die durch zwei Abläufe 11 und 12 aus der Zentrifuge abfließen.

Der Mischer 7 kann auf verschiedene Weise ausgelegt sein. So kann ein statischer Mischer oder ein dynamischer Mischer eingesetzt werden. Geeignet sind auch Sonderformen wie ein High-Shear-Mischer oder ein Nanoreaktor.

Denkbar ist es auch, als Mischer die Zentrifuge selbst zu verwenden. In diesem Fall werden die lipoide Phase und die Salzlösung (wäßrige Lösung) durch getrennte Zuleitungen in die Zentrifuge geleitet, wo - beispielsweise in einem Verteiler 15 der Zentrifugentrommel - die Mischung dieser beiden Phasen erfolgt. Derartige Verteiler sind an sich bekannt und dienen zu Überleitung des zulaufenden Produktes in die rotierende Trommel.

Als Zentrifuge wird vorzugsweise ein Trenn-Separator mit vertikaler Drehachse eingesetzt, der dazu ausgelegt ist, zwei Flüssigkeitsphasen unterschiedlicher Dichte zu trennen.

Die Vorrichtung kann auch dazu ausgelegt sein, unter Druck p betrieben zu werden, der höher als der Atmosphärendruck ist. Vorzugseise gilt: $1\ \text{bar} \leq p < 10\ \text{bar}$. Der Ablaufdruck in den Abläufen 11 und 12 sollte höher sein als der Zulaufdruck in der Zuleitung zur Zentrifuge. Vermieden werden soll vorzugsweise ein Lufteintrag im Zulauf, um zu vermeiden, dass sich im Mischer und/oder in der Zentrifugentrommel in störendem Maße eine Emulsion ausbildet.

Es konnte gezeigt werden, dass mit dieser Vorrichtung sich eine Emulsionsbildung vermeiden lässt, was zur Folge hat, dass einerseits die separierte Fraktion, enthaltend Glycoglycerolipide und Glycosphingolipide, besser abtrennbar ist, weil eine bessere Phasentrennung erfolgt, und zum anderen die Abreicherung der lipoiden Phase vollständiger ist als mit einem Misch- und Separationssystem, das den Ausschluss einer Luft/-Gaseintragung nicht verhindert. Zur Realisierung der großtechnischen Gewinnung einer hydrolysearmen und besonders reinen Fraktion an Glycoglycerolipiden und Glycosphingolipiden aus lipoiden Phasen ist daher die Vorrichtung besonders geeignet.

Diese Vorrichtungen sind zur Ausführung der im Folgenden beschriebenen erfindungsgemäßen Verfahren konzipiert.

Daher betrifft die vorliegende Erfindung ein Verfahren zum hydrolysearmen Abtrennen von Glycoglycerolipiden aus einer lipoiden Phase, welche Glycoglycerolipide und Acylglyceride enthält, umfassend die Schritte:

A1) Bereitstellung einer lipoiden Phase, enthaltend Glycoglycerolipide und Acylglyceride,

B1) Versetzen der lipoiden Phase mit einer wässrigen Phase, enthaltend Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$) , Acetat ($CH_3COO^-$), Borat ($BO_3^{3-}$) und/oder Tartrat ($C_4H_4O_6^{2-}$) bildet,

C1) Durchmischung der lipoiden Phase und der wässrigen Phase,

D1) und Abtrennung der wässrigen glycoglycerolipidreichen Phase und Erhalt einer lipoiden glycoglycerolipidarmen Phase.

Das oder die eingesetzten Salze bestehen natürlich nicht nur aus Anionen sondern auch aus Kationen, so dass die vorliegende Erfindung auch ein Verfahren zum hydrolysearmen Abtrennen von Glycoglycerolipiden aus einer lipoiden Phase betrifft, welche Glycoglycerolipide und Acylglyceride enthält, umfassend die Schritte:

A1) Bereitstellung einer lipoiden Phase, enthaltend Glycoglycerolipide und Acylglyceride,

B1) Versetzen der lipoiden Phase mit einer wässrigen Phase, enthaltend Kationen und Anionen mindestens eines Salzes, welches in Wasser bei 20°C eine Löslichkeit von mindestens 30 g/L besitzt und bei der Dissoziation in

Wasser Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$) , Acetat ($CH_3COO^-$), Borat ($BO_3^{3-}$) und/oder Tartrat ($C_4H_4O_6^{2-}$) bildet,
C1) Durchmischung der lipoiden Phase und der wässrigen Phase,
D1) und Abtrennung der wässrigen glycoglycerolipidreichen Phase und Erhalt einer lipoiden glycoglycerolipidarmen Phase.

Erfindungsgemäß werden in den hierin offenbarten Verfahren die Verfahrensschritte A1) bis D1) auch in der angegebenen Reihenfolge A1) => B1) => C1) => D1) durchgeführt oder sofern zusätzliche Verfahrenschritte hinzukommen sollten wie z.B. die Schritte A2), A2'), D2) und E1), so ist die Schrittfolge wie hierin angegeben und Schritt D2) folgt nach Schritt D1), Schritt E1) folgt nach Schritt D1) oder sofern Schritt D2) vorhanden ist, folgt Schritt E1) nach Schritt D2) und Schritt A2) folgt nach Schritt A1) oder alternativ zu Schritt A2) kann Schritt A2') nach Schritt A1) folgen.
Es ergeben sich somit folgende mögliche Schrittabfolgen:

A1) => B1) => C1) => D1)
A1) => B1) => C1) => D1) => D2)
A1) => B1) => C1) => D1) => E1)
A1) => B1) => C1) => D1) => D2) => E1)
A1) => A2) => B1) => C1) => D1)
A1) => A2) => B1) => C1) => D1) => D2)
A1) => A2) => B1) => C1) => D1) => E1)
A1) => A2) => B1) => C1) => D1) => D2) => E1)
A1) => A2') => B1) => C1) => D1)
A1) => A2') => B1) => C1) => D1) => D2)
A1) => A2') => B1) => C1) => D1) => E1)
A1) => A2') => B1) => C1) => D1) => D2) => E1)

Sollte die lipoide Phase neben den Glycoglycerolipiden auch noch Glycosphingolipide enthalten, so können diese zusammen mit den Glycoglycerolipiden aus der lipoiden Phase abgetrennt werden. In einem solchen Falle gestaltet sich das erfindungsgemäße Verfahren wie folgt:
Verfahren zum hydrolysearmen Abtrennen von Glycoglycerolipiden und Glycosphingolipiden aus einer lipoiden Phase, welche Glycoglycerolipide und Glycosphingolipide und Acylglyceride enthält, umfassend die Schritte:

A1) Bereitstellung einer lipoiden Phase, enthaltend Glycoglycerolipide, Glycosphingolipide und Acylglyceride,
B1) Versetzen der lipoiden Phase mit einer wässrigen Phase, enthaltend Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO4^{4-}$), Disilicat ($Si_2O_5^{2-}$), Trisilicat ($Si_3O_7^{2-}$) , Acetat ($CH_3COO^-$), Borat ($BO_3^{3-}$) und/oder Tartrat ($C_4H_4O_6^{2-}$) bildet,
C1) Durchmischung der lipoiden Phase und der wässrigen Phase,
D1) und Abtrennung der wässrigen glycoglycerolipidreichen und/oder glycosphingolipidreichen Phase und Erhalt einer lipoiden glycoglycerolipidarmen und/oder glycosphingolipidarmen Phase.

Entsprechend betrifft die vorliegende Erfindung ein Verfahren zum hydrolysearmen Abtrennen von Glycoglycerolipiden und Glycosphingolipiden aus einer lipoiden Phase, welche Glycoglycerolipide und Glycosphingolipide und Acylglyceride enthält, umfassend die Schritte:

A1) Bereitstellung einer lipoiden Phase, enthaltend Glycoglycerolipide, Glycosphingolipide und Acylglyceride,
B1) Versetzen der lipoiden Phase mit einer wässrigen Phase, enthaltend Kationen und Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$) , Acetat ($CH_3COO^-$), Borat ($BO_3^{3-}$) und/oder Tartrat ($C_4H_4O_6^{2-}$) bildet,
C1) Durchmischung der lipoiden Phase und der wässrigen Phase,
D1) und Abtrennung der wässrigen glycoglycerolipidreichen und/oder glycosphingolipidreichen Phase und Erhalt einer lipoiden glycoglycerolipidarmen und/oder glycosphingolipidarmen Phase.

Als Kation des Salzes oder als Kationen des Gemsiches von Salzen werden folgende bevorzugt: $Na^+$, $K^+$, $Li^+$, $Mg^{2+}$, $Ca^{2+}$, $Ti^{2+}$, $Ti^{4+}$, $Co^{2+}$, $Co^{3+}$, $Ni^{2+}$, $Cu^{2+}$, $Zn^{2+}$, $Sn^{2+}$ und/oder $Sn^{4+}$. Insbesondere bevorzugt sind $Na^+$ und $K^+$.
Als Acylglyceride werden Monoacylglyceride, Diacylglyceride und Triacylglyceride bezeichnet. Als Triacylglyceride werden Verbindungen bezeichnet, bei denen drei Acylreste (Ay, Ay', Ay") über eine Esterbindung an Glycerin

gebunden sind. Eine allgemeine Formel für Triacylglyceride ist unten gezeigt. Entsprechend sind bei Diacylglyceriden zwei Acylreste (Ay, Ay') über eine Esterbindung an Glycerin gebunden und bei Monoacylglyceriden ist ein Acylrest (Ay) über eine Esterbindung an Glycerin gebunden.

Unter Glycoglycerolipiden werden Verbindungen verstanden, bei denen zwei Acylreste (Ay, Ay') über eine Esterbindung an Glycerin gebunden sind und an die dritte Hydroxygruppe (bzw. das dritte Sauerstoffatom) des Glycerins ein Saccharid vorzugsweise über das anomere Kohlenstoffatom gebunden ist. Eine allgemeine Formel für Glycoglycerolipide ist unten gezeigt.

Erfindungsgemäß werden die Acylglyceride nicht in die wäßrige glycoglycerolipidreiche Phase überführt und verbleiben in der lipoiden glycoglycerolipidarmen Phase. Der Begriff "glycoglycerolipidreiche Phase" bezeichnet somit die Fraktion der eingesetzten lipoiden Phase, welche aus Glycoglycerolipiden besteht oder worin die Glycoglycerolipide angereichert sind. Entsprechend bezeichnet der Begriff "glycoglycerolipidarme Phase" die Fraktion der eingesetzten lipoiden Phase, worin die Glycoglycerolipide vermindert oder aus der die Glycoglycerolipide entfernt wurden. Sofern die eingesetzte lipoide Phase auch Glycosphingolipide neben den Glycoglycerolipiden enthält, so bezeichnet der Begriff "glycoglycerolipidarme Phase" entsprechend die Fraktion der eingesetzten lipoiden Phase, worin die Glycosphingolipide und die Glycoglycerolipide vermindert oder entfernt wurden und der Begriff "glycoglycerolipidreiche Phase" bezeichnet somit die Fraktion der eingesetzten lipoiden Phase, welche aus Glycoglycerolipiden und Glycosphingolipiden besteht oder worin die Glycoglycerolipide und die Glycosphingolipide angereichert sind.

Glycolipide bezeichnen hingegen Substanzen, bei denen ein Acylrest (Ay) an eine Hydroxygruppe eines Saccharids und vorzugsweise an der Hydroxygruppe am anomeren Kohlenstoffatom des Saccharids gebunden ist. Eine allgemeine Formel für Glycolipide und Glycoglycerolipide ist im Folgenden gezeigt.

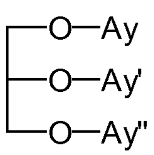
Triacylglyceride

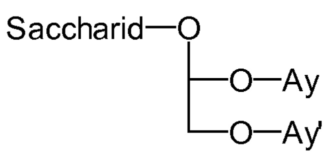
Glycoglycerolipide

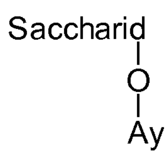
Glycolipide

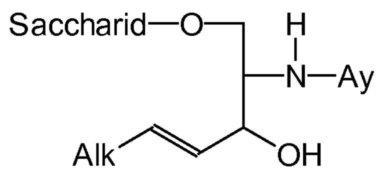
Glycosphingolipide

Die Bezeichnung "Saccharid" steht für einen Zuckerrest, wobei es sich bei dem Zuckerrest um ein Monosaccharid, Oligosaccharid oder Polysaccharid handeln kann. Bei den Acylresten (Ay, Ay' und Ay") handelt es sich vorzugsweise um Fettsäurereste. Die Lipide und Fettsäurereste werden weiter unten noch eingehender behandelt.

Glycosphingolipide bestehen aus einem Saccharidrest, der an einen Ceramidrest gebunden ist. Die Bezeichnung "Alk" steht für einen Alkylrest und vorzugsweise einen langkettigen Alkylrest mit mehr als 10 Kohlenstoffatomen. Handelt es sich bei dem Saccharidrest um Galaktose, so bezeichnet man diese Monoglycosylceramide als Cerebroside. Ein Beispiel ist im Folgenden gezeigt:

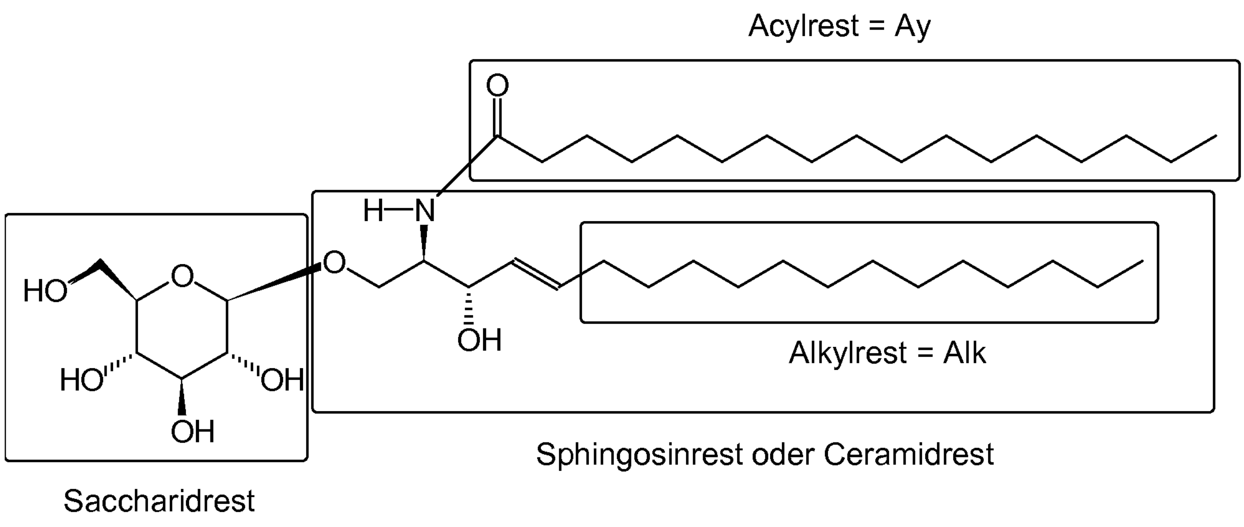


Im Folgenden wird als Beispiel für ein Glycolipid ein Diglycosyllipid und als Beispiel für ein Glycoglycerolipid ein Monoglycosylglycerolipid gezeigt.

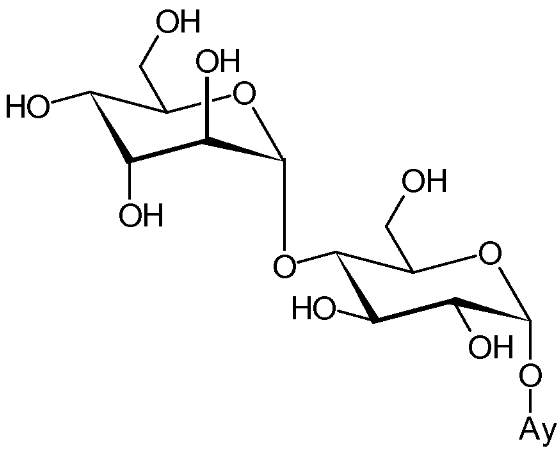

Diglycolipid oder Diglycosyllipid

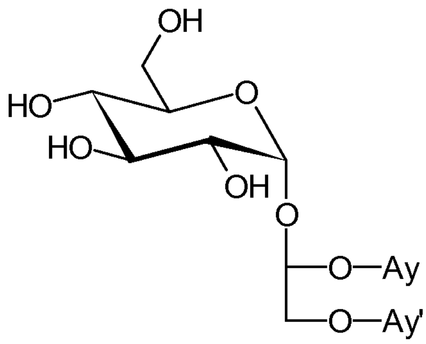

Monoglycoglycerolipide oder Monoglycosylglycerolipide

Die erfindungsgemäßen Verfahren sind insbesondere geeignet, um Glycoglycerolipide oder Gemische aus Glycoglycerolipiden und Glycosphingolipiden aus den lipoiden Phasen abzutrennen. Sollten die lipoiden Phasen neben den Glycoglycerolipiden oder neben den Glycoglycerolipiden und Glycosphingolipiden auch noch Glycolipid und/oder Glycophospholipide enthalten, so lassen sich vor allem die Glycolipide aber auch die Glycophospholipide und insbesondere die neutralen Glycophospholipide zusammen mit den Glycoglycerolipiden oder den Glycoglycerolipiden und Glycosphingolipiden aus den lipoiden Phasen abtrennen. Insbesondere sind die erfindungsgemäßen Verfahren geeignet, um Monoglycosylsphingolipide, Diglycosylsphingolipide, Triglycosylsphingolipide, Tetraglycosylsphingolipide, Pentaglycosylsphingolipide, Monoacylmonoglycosyl-sphingolipide, Monoacyldiglycosylsphingolipide, Monoacyltriglycosylsphingolipide, Monoacyltetraglycosylsphingolipide, Monoacylpentaglycosylsphingolipide, Diacyldi-glycosylsphingolipide, Diacyltriglycosylsphingolipide, Diacyltetraglycosylsphingolipide, Diacylpentaglycosylsphingolipide, Triacyltriglycosylsphingolipide, Triacyltetraglycosylsphingolipide, Triacylpentaglycosylsphingolipide, Tetraacyltetraglycosylsphingolipide, Tetraacylpentaglycosylsphingolipide, Pentaacylpentaglycosylsphingolipide, Monoglycosylglycerolipide, Diglycosylglycerolipide, Triglycosylglycerolipide, Tetraglycosylglycerolipide, Pentaglycosylglycerolipide, Hexaglycosylglycerolipide, Heptaglycosylglycerolipide, Octaglycosylglycerolipide, Nonaglycosylglycerolipide, Decaglycosylglycerolipide, Monoacyldiglycosylglycerolipide, Monoacyltriglycosylglycerolipide, Monoacyltetraglycosylglycerolipide, Monoacylpentaglycosylglycerolipide, Monoacylhexaglycosylglycerolipide, Monoacylheptaglycosylglycerolipide, Monoacyloctaglycosylglycerolipide, Monoacylnonaglycosylglycerolipide, Monoacyldecaglycosylglycerolipide, Diacyltriglycosylglycerolipide, Diacyltetraglycosylglycerolipide, Diacylpentaglycosylglycerolipide, Diacylhexaglycosylglycerolipide, Diacylheptaglycosylglycerolipide, Diacyloctaglycosylglycerolipide, Diacylnonaglycosylglycerolipide, Diacyldecaglycosylglycerolipide, Triacyltetraglycosylglycerolipide, Triacylpentaglycosylglycerolipide, Triacylhexaglycosylglycerolipide, Triacylheptaglycosylglycerolipide, Triacyloctaglycosylglycerolipide, Triacylnonaglycosylglycerolipide, Triacyldecaglycosylglycerolipide, Tetraacylpentaglycosylglycerolipide, Tetraacylhexaglycosylglycerolipide, Tetraacylheptaglycosylglycerolipide, Tetraacyloctaglycosylglycerolipide, Tetraacylnonaglycosylglycerolipide, Tetraacyldecaglycosylglycerolipide, Pentaacylhexaglycosylglycerolipide, Pentaacylheptaglycosylglycerolipide, Pentaacyloctaglycosylglycerolipide, Pentaacylnonaglycosylglycerolipide, Pentaacyldecaglycosylglycerolipide, Hexaacylheptaglycosylglycerolipide, Hexaacyloctaglycosylglycerolipide, Hexaacylnonaglycosylglycerolipide, Hexaacyldecaglycosylglycerolipide, Heptaacyloctaglycosylglycerolipide, Heptaacylnonaglycosylglycerolipide, Heptaacyldecaglycosylglycerolipide, Octaacylnonaglycosylglycerolipide, Octaacyldecaglycosylglycerolipide und/oder Nonaacyldecaglycosylglycerolipide aus der lipoiden Phase abzutrennen.

Es war sehr überraschend, dass mit den erfindungsgemäßen Verfahren die eher lipoiden Glycoglycerolipide und Glycosphingolipide in die wässrige Phase überführt werden konnten. Bei den lipohilen Glycoglycerolipiden und Glycosphingolipiden handelt es sich um Verbindungen, die nicht oder nur schlecht durch eine Extraktion mit Wasser oder hydrophile Verbindungen in die wässrige Phase überführt werden können. Der Begriff "schlecht" bedeutet in diesem Zusammenhang, dass pro Extraktionsschritt mit Wasser nur weniger als 10 Gew.% der Gesamtmenge an Glycoglycerolipiden oder Glycoglycerolipiden und Glycosphingolipiden aus der lipoiden Phase abgetrennt werden können. Daher ist auch bevorzugt, dass die Glycoglycerolipide keine Carboxylat-, Sulfat-, Sulfonat- oder Phosphatgruppe(n) enthalten. Ebenfalls ist bevorzugt, dass die Glycosphingolipide keine Carboxylat-, Sulfat-, Sulfonat- oder Phosphatgruppe(n) enthalten.

Somit ist die vorliegende Erfindung insbesondere auf Verfahren zum Abtrennen von Glycoglycerolipiden gerichtet, wobei es sich bei den Glycoglycerolipiden um lipophile Glycoglycerolipide mit einem Lipophilitätsindex GL von 1,0 ≤ GL ≤ 6,0 handelt, wobei sich der Lipophilitätsindex GL gemäß folgender Formel berechnet:

$$GL = \frac{\text{Summe der Kohlenstoffatome der Acylreste}}{\text{Summe der Hydroxy- und Aminogruppen}}$$

Darin bedeutet die "Summe der Kohlenstoffatome der Acylrest" die Summe aller Kohlenstoffatome aller Acylreste. Zwei Acylreste (Ay und Ay') befinden sich am Glycerinrest und eine oder mehrere weitere Acylreste können sich an den Saccharidresten befinden. Möglich ist aber auch, dass sich an einem Acylrest ein weiterer Acylrest befindet. Bei den Acylresten wird auch das Carbonylkohlenstoffatom mitgezählt.
Die "Summe der Hydroxy- und Aminogruppen" meint sämtliche Hydroxygruppen und Aminogruppen im Molekül einschließlich der Hydroxy- und Aminogruppen, welche sich an einem Acylrest befinden. Beispielsweise besitzt folgendes Diglycosylglycerolipid, worin R einen Alkylrest mit 16 Kohlenstoffatomen bedeutet, einen Lipophilitätsindex GL von 4,9.

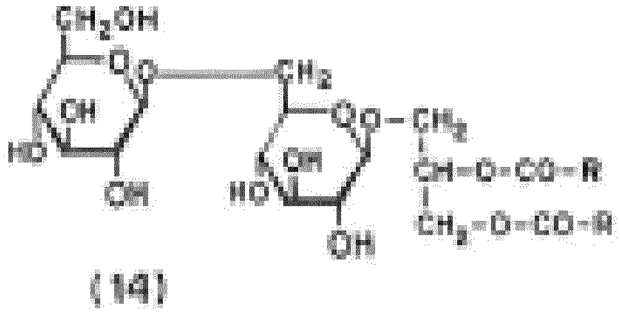

(14)

Sofern in der lipoiden Phase auch Glycosphingolipide anwesend sind, bezieht sich die vorliegende Erfindung vorzugsweise auf Verfahren zum Abtrennen von Glycoglycerolipiden und Glycosphingolipide, wobei es sich bei den Glycosphingolipiden um lipophile Glycosphingolipide mit einem Lipophilitätsindex SL von 1,0 ≤ SL ≤ 7,0 handelt, wobei sich der Lipophilitätsindex SL gemäß folgender Formel berechnet:

$$SL = \frac{\text{Summe der Kohlenstoffatome des Ceramidrestes}}{\text{Summe der Hydroxy- und Amino- und Amidgruppen}}$$

Darin bedeutet die "Summe der Kohlenstoffatome des Ceramidrestes" die Summe aller Kohlenstoffatome im Ceramidrest einschließlich der Kohlenstoffatome des Acylrestes im Ceramidrest. Falls weitere Acylreste an den Saccharidresten oder an dem Acylrest im Ceramidrest gebunden sein sollten, so werden die Kohlenstoffatome dieser Acylreste zu der Summe der Kohlenstoffatome des Ceramidrestes hinzu addiert. Bei den Acylresten wird auch das Carbonylkohlenstoffatom als auch im Ceramidrest das Amidkohlenstoffatom mitgezählt. Die "Summe der Hydroxy- und Amino- und Amidgruppen" meint sämtliche Hydroxygruppen, Aminogruppen und Amidgruppen im Molekül einschließlich der Hydroxy- und Aminogruppen, welche sich an einem Acylrest befinden. Als Hydroxygruppe wird die -OH Gruppe, als Aminogruppe die $-NH_2$ Gruppe und als Amidgruppe die -NH-CO- oder die -CO-NH- Gruppe bezeichnet. Beispielsweise besitzt folgendes Glycosphingolipid, worin R einen Alkenylrest mit 15 Kohlenstoffatomen bedeutet, einen Lipophilitätsindex von 4,7.

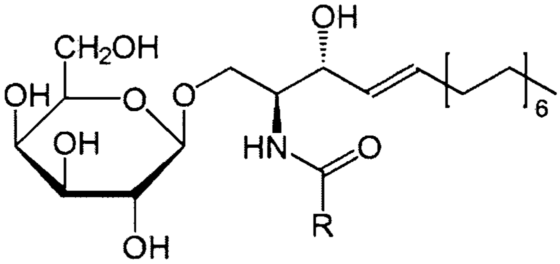

Alternativ zum Lipophilitätsindex kann auch die HLB-Lipophilitätsskala verwendet werden, welche in Figur 2 gezeigt ist und innerhalb eines Bereichs von 0 bis 20 im unteren Bereich die lipophilen Substanzen, im oberen Bereich die hydrophilen Substanzen und im Bereich um 10 die equi-amphiphilen Substanzen (gleichermaßen lipophil wie hydrophil) ansiedelt und insbesondere für Emulgiermittel gedacht ist.

Da die erfindungsgemäßen Verfahren zur Abtrennung von Glycoglycerolipiden aus lipoiden Phasen dienen, ist bevorzugt, wenn die erfindungsgemäßen Verfahren zur Abtrennung von Glycoglycerolipiden nach Schritt D1) den folgenden Schritt D2) umfassen:

D2) Gewinnung der Glycoglycerolipide aus der abgetrennten wässrigen Phase.

Für den Fall, dass die lipoide Phase neben den Glycoglycerolipiden auch Glycosphingolipide enthält, ist bevorzugt, wenn die erfindungsgemäßen Verfahren zur Abtrennung von Glycoglycerolipiden und Glycosphingolipiden nach Schritt D1) den folgenden Schritt D2) umfassen:

D2) Gewinnung der Glycoglycerolipide und der Glycosphingolipide aus der abgetrennten wässrigen Phase.

Für den Fall, dass die lipoide Phase neben den Glycoglycerolipiden oder neben den Glycoglycerolipiden und Glycosphingolipiden auch noch Glycolipide und/oder Glycophospholipide enthält, ist bevorzugt, wenn die erfindungsgemäßen Verfahren zur Abtrennung von Glycoglycerolipiden und Glycosphingolipiden nach Schritt D1) den folgenden Schritt D2) umfassen:

D2) Gewinnung der Glycoglycerolipide und Glycolipide und/oder Glycophospholipide oder Gewinnung der Glycoglycerolipide und Glycosphingolipide und Glycolipide und/oder Glycophospholipide und der aus der abgetrennten wässrigen Phase.

Aufgrund ihrer vielfältigen Eignung als Biotenside ist ein Ziel der vorliegenden Erfindung, aus den lipoiden Phasen Glycoglycerolipide oder Gemische, enthaltend Glycoglycerolipide und Glycophospholipide, zu isolieren und weiteren Verwendungen zuzuführen. Je nach Zusammensetzung und Inhaltsstoffen der lipoiden Phase, welche zum überwiegenden Anteil (vorzugsweise > 80 Gew.-%) aus Acylglyceriden und insbesondere aus Triacylglyceriden besteht, können aus der lipoiden Phase Fraktionen, enthaltend Glycophospholipide oder Fraktionen, enthaltend Glycosphingolipide oder Fraktionen, enthaltend Sterylglycoside oder Fraktionen, enthaltend Glycophospholipide und Glycosphingolipide oder Fraktionen, enthaltend Glycophospholipide und Sterylglycoside oder Fraktionen, enthaltend Glycosphingolipide und Sterylglycoside oder Fraktionen, enthaltend Glycophospholipide und Sterylglycoside und Glycosphingolipide abgetrennt und die Glycophospholipide, Glycosphingolipide, Sterylglycoside oder die Gemische der vorgenannten Stoffe aus diesen Fraktionen gewonnen werden. Somit betrifft die vorliegende Offenbarung auch wässrige glycoglycerolipidreiche Phasen, wässrige sterylglycosidreiche Phasen sowie lipoide glycoglycerolipidarme Phasen, erhältlich oder erhalten nach einem der hierin offenbarten Verfahren.

Der Begriff Glycophospholipide bezeichnet ein Glycerophospholipid, wobei an der Phosphatgruppe ein Saccharidrest gebunden ist wie z.B. beim Phosphatidylinositol.

Bei vorgenannter Nomenklatur bezeichnet z.B. "Monoacyltetraglycosylglycerolipide", dass ein Acylrest sich an einem der 4 Saccharidreste befindet. Dabei befindet sich der Acylrest vorzugsweise nicht an dem Saccharidrest, an dem sich der Diacylglycerinrest befindet. Falls unter den Saccharidresten ein Aminozucker ist, so kann sich der Acylrest auch an der Aminogruppe des Aminozuckers befinden. Somit bezeichnet der Begriff "Triacylhexaglycosylglycerolipide" ein Hexasaccharid, wobei an drei Hyxdroxygruppen des Hexasaccharids sich ein Acylrest befindet. Bei den Acylresten kann es sich natürlich um unterschiedliche Acylreste handeln. Es ist nicht erforderlich und zudem auch eher die Ausnahme, dass es sich um gleiche Acylreste handelt. Auch die Acylreste am Glycerinrest sind zumeist nicht identisch und sind zudem in der Regel andere Acylreste, als die Acylreste, welche direkt an die Saccharidreste gebunden sind. Beim vorgenannten Beispiel besteht das Glycoglycerolipid aus einem Diacylglycerinrest und einem Hexasaccharid, wobei an das Hexasaccharid noch drei Acylreste gebunden sind. Hier ist bevorzugt, wenn nicht mehr als ein Acylrest pro Saccharidrest vorhanden ist und sich an dem Saccharidrest kein Acylrest befindet, an dem der Diacylglycerinrest gebunden ist. Sollten sich im dem Hexesaccharid Aminozucker befinden, so kann an der Aminogruppe des Aminozuckers der Acylrest gebunden sein.

Des weiteren fallen unter den Begriff der Glycoglycerolipide auch solche, welche an dem Glycerinrest anstelle von einem Acylrest einen Alkylrest, Alkenylrest oder Alkinylrest tragen und solche, welche an dem Glycerinrest anstelle von beiden Acylresten zwei Reste ausgewählt aus der Gruppe, bestehend aus Alkylrest, Alkenylrest und Alkinylrest, haben. Diese Verbindungen können durch die folgenden allgemeinen Formeln dargestellt werden, worin Alk und Alk' unabhängig voneinander jeweils einen Alkylrest, Alkenylrest oder Alkinylrest bedeuten:

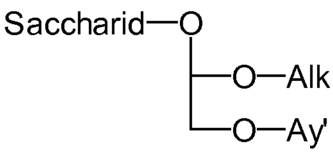

Glycosylacylalkylglycerine

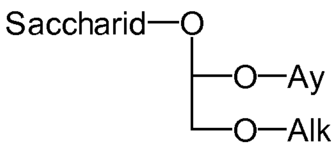

Glycosylacylalkylglycerine

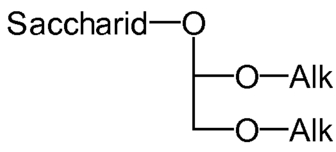

Glycosyldialkylglycerine

Somit betrifft die vorliegende Erfindung vorzugsweise Verfahren zum Abtrennen von Glycosyldiacylglycerinen, Glycosylacylalkylglycerinen und Glycosyldialkylglycerinen aus einer lipoiden Phase und noch bevorzugter von Monoglycosylsphingolipiden, Diglycosylsphingolipiden, Triglycosylsphingolipiden, Tetraglycosylsphingolipiden, Pentaglycosylsphingolipiden, Monoglycosylglycerolipiden, Diglycosylglycerolipiden, Triglycosylglycerolipiden, Tetraglycosylglycerolipiden, Pentaglycosylglycerolipiden, Hexaglycosylglycerolipiden, Heptaglycosylglycerolipiden, Monoacyldiglycosylglycerolipiden, Monoacyltriglycosylglycerolipiden, Monoacyltetraglycosylglycerolipiden, Monoacylpentaglycosylglycerolipiden, Monoacylhexaglycosylglycerolipiden und Monoacylheptaglycosylglycerolipiden.

Bei den Acylresten handelt es sich vorzugsweise um Acylreste von Fettsäuren und bei den Alk-Resten vorzugsweise um Alkylreste, Alkenylreste oder Alkinylreste von Fettsäuren. Dabei umfasst der Begriff "Alkenylrest" nicht nur monoolefinische Reste, sondern auch ditri- und polyolefinische Reste sowie Kohlenstoffreste mit mindestens einer Doppelbindung und mindestens einer Dreifachbindung. Der Begriff "Alkinylrest" umfasst Kohlenstoffreste mit einer, zwei, drei oder mehr Dreifachbindungen.

Beispiele für bevorzugte Acylreste sind:
Dodecanoyl, Hexadecanoyl, Octadecanoyl, Eicosanoyl, Docosanoyl, Tetracosanoyl, cis-9-Tetradecenoyl, cis-9-Hexadecenoyl, cis-6-Octadecenoyl, cis-9-Octadecenoyl, cis-11-Octadecenoyl, cis-9-Eicosenoyl, cis-11-Eicosenoyl, cis-13-Docosenoyl, cis-15-Tetracosenoyl, 9,12-Octadecadienoyl, 6,9,12-Octadecatrienoyl, 8,11,14-Eicosatrienoyl, 5,8,11,14-Eicosatetraenoyl, 7,10,13,16-Docosatetraenoyl, 4,7,10,13,16-Docosapentaenoyl, 9,12,15-Octadecatrienoyl, 6,9,12,15-Octadecatetraenoyl, 8,11,14,17-Eicosatetraenoyl, 5,8,11,14,17-Eicosapentaenoyl, 7,10,13,16,19-Docosapentaenoyl, 4,7,10,13,16,19-Docosahexaenoyl, 5,8,11-Eicosatrienoyl, 1,2-Dithiolan-3-pentanoyl, 6,8-Dithianoctanoyl, Docosaheptadecanoyl, Eleostearoyl, Calendoyl, Catalpoyl, Taxoleoyl, Pinolenoyl, Sciadonoyl, Retinoyl, 14-Methylpentadecanoyl, Pristanoyl, Phytanoyl, 11,12-Methyleneoctadecanoyl, 9,10-Methylenehexadecanoyl, 9,10-Epoxystearoyl, 9,10-Epoxyoctadec-12-enoyl, 6-Octadecinoyl, t11-Octadecen-9-inoyl, 9-Octadecinoyl, 6-Octadecen-9-inoyl, t10-Heptadecen-8-inoyl, 9-Octadecen-12-inoyl, t7,t11-Octadecadiene-9-inoyl, t8,t10-Octadecadiene-12-inoyl, 5,8,11,14-Eicosatetrainoyl, 2-Hydroxytetracosanoyl, 2-Hydroxy-15-tetracosenoyl, 12-Hydroxy-9-octadecenoyl und 14-Hydroxy-11-eicosenoyl.

Als Alkylreste, Alkenylreste oder Alkinylreste sind insbesondere die Kohlenstoffreste (d.h. ohne die COOH-Gruppe) der folgenden Säuren bevorzugt: Hexansäure, Octansäure, Decansäure, Dodecansäure, Tetradecansäure, Hexadecansäure, Heptadecansäure, Octadecansäure, Eicosansäure, Docosansäure, Tetracosansäure, cis-9-Tetradecensäure, cis-9-Hexadecensäure, cis-6-Octadecensäure, cis-9-Octadecensäure, cis-11-Octadecensäure, cis-9-Eicosensäure, cis-11-Eicosensäure, cis-13-Docosensäure, cis-15-Tetracosensäure, t9-Octadecensäure, t11-Octadecensäure, t3-Hexadecensäure, 9,12-Octadecadiensäure, 6,9,12-Octadecatriensäure, 8,11,14-Eicosatriensäure, 5,8,11,14-Eicosatetraensäure, 7,10,13,16-Docosatetraensäure, 4,7,10,13,16-Docosapentaensäure, 9,12,15-Octadecatriensäure, 6,9,12,15-Octadecatetraensäure, 8,11,14,17-Eicosatetraensäure, 5,8,11,14,17-Eicosapentaensäure, 7,10,13,16,19-Docosapentaensäure, 4,7,10,13,16,19-Docosahexaensäure, 5,8,11-Eicosatriensäure, 9c11t13t-Eleostearinsäure, 8t10t12c-Calendulasäure, 9c11t13c-Catalpinsäure, 4,7,9,11,13,16,19-Docosaheptadecansäure, Taxoleinsäure, Pinolensäure, Sciadonsäure, 6-Octadecinsäure, t11-Octadecen-9-insäure, 9-Octadecinsäure, 6-Octadecen-9-insäure, t10-Heptadecen-8-insäure, 9-Octadecen-12-insäure, t7,t11-Octadecadien-9-insäure, t8,t10-Octadecadien-12-insäure, 5,8,11,14-Eicosatetrainsäure, Retinsäure, Isopalmitinsäure, Pristansäure, Phytansäure, 11,12-Methylen-Octadecansäure, 9,10-Methylen-Hexadecansäure, Coronarinsäure, (R,S)-Liponsäure, (S)-Liponsäure, (R)-Liponsäure, 6,8(methylsulfanyl)-Oktansäure, 4,6-bis(methylsulfanyl)-Hexansäure, 2,4-bis(methylsulfanyl)-Butansäure, 1,2-Dithiolan-Carboxylsäure, (R,S)-6,8-Dithian-Oktansäure, (S)-6,8-Dithian-Oktansäure, Taririnsäure, Santalbinsäure, Stearolsäure, 6,9-Octadeceninsäure, Pyrulinsäure, Crepeninsäure, Heisterinsäure, t8,t10-Octadecadien-12-insäure, ETYA, Cerebronsäure, Hydroxynervonsäure, Ricinoleinsäure, Lesquerolinsäure, Brassylinsäure und Thapsinsäure.

Ein weiterer Aspekt der vorliegenden Offenbarung ist auf die abgetrennten Stoffe gerichtet, so dass die vorliegende Offenbarung auch die Gemische, enthaltend die Glycoglycerolipide oder die Glycoglycerolipide und Glycosphingolipide oder die Glycoglycerolipide und Glycosphingolipide und die Glycolipide und/oder die Glycophospholipide, betrifft. Diese Stoffgemische aus verschiedenen Glycoglycerolipiden oder aus verschiedenen Glycoglycerolipiden und Glycosphingolipiden oder aus verschiedenen Glycoglycerolipiden und Glycosphingolipiden und Glycolipiden und/oder Glycophospholipiden können gemäß den erfindungsgemäßen Verfahren und insbesondere durch Extraktion aus der abgetrennten wässrigen Phase gewonnen werden.

Die Glycoglycerolipide und sofern in der lipoiden Phase vorhanden, die Glycosphingolipide, werden aus der

lipoiden Phase durch Versetzen der lipoiden Phase mit einer wässrigen Phase, enthaltend Anionen mindestens eines Salzes, gewonnen, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$) , Acetat ($CH_3COO^-$), Borat ($BO_3^{3-}$) und/oder Tartrat ($C_4H_4O_6^{2-}$) bildet, gefolgt von vorzugsweise intensiver Durchmischung beider Phasen und Trennung und Abtrennung der wässrigen Phase durch vorzugsweise Zentrifugation. Der Begriff "mindestens eines Salzes", wie hierin verwendet, soll verdeutlichen, dass natürlich auch mehrere Salze, d.h. Gemische von Salzen eingesetzt werden können. Dabei können Salze gleicher Anionen und unterschiedlicher Kationen wie z.B. Natriumacetat und Kaliumacetat als auch Salze gleicher Kationen und unterschiedlicher Anionen wie z.B. Natriumhydrogencarbonat und Natriumsilikat eingesetzt werden. Selbstverständlich können auch Gemische von Salzen unterschiedlicher Kationen und unterschiedlicher Anionen wie z.B. Natriumcarbonat und Kaliumtartrat eingesetzt werden. Nicht üblich ist hingegen die Verwendung von Salzen mit verschiedenen Anionen und/oder verschiedenen Kationen in einem Salz wie z.B. Natriumkaliumcarbonat.

Als Salze, welche in Wasser die vorgenannten Anionen bilden, eignen sich vorzugsweise $Na_2CO_3$, $K_2CO_3$, $NaHCO_3$, $KHCO_3$, $Na_2SiO_3$, $K_2SiO_3$, $Na_4SiO_4$, $K_4SiO_4$, $Na_2Si_2O_5$, $K_2Si_2O_5$, $Na_2Si_3O_7$, $K_2Si_3O_7$, $NaOOCCH_3$, $KOOCCH_3$, $Cu(OOCCH_3)_2$, $Na_2C_4H_4O_6$, $K_2C_4H_4O_6$, $Na_3BO_3$ und $K_3BO_3$. Diese Salze werden bezogen auf die Glycoglycerolipide mindestens in stöchiometrischen Mengen zugesetzt. Sofern neben den Glycoglycerolipiden auch Glycosphingolipide in der lipoiden Phase enthalten sind, werden diese Salze, bezogen auf die Gesamtmenge der Glycoglycerolipide und Glycosphingolipide, mindestens in stöchiometrischen Mengen zugesetzt. Ferner sollte zumindest ein Überschuss von 0,2 bis 1,0 Moläquivalenten angewendet werden. Ein Überschuss von 1,0 Moläquivalenten entspricht einem 100%igen Überschuss. Bevorzugt ist in de Regel ein Überschuss von 1,0 Moläquivalenten bis 10,0 Moläquivalenten, weiter bevorzugt von 2,0 Moläquivalenten bis 9,0 Moläquivalenten, weiter bevorzugt von 3,0 Moläquivalenten bis 8,0 Moläquivalenten, weiter bevorzugt von 4,0 Moläquivalenten bis 7,0 Moläquivalenten.

Erfindungsgemäß werden die Glycoglycerolipide und sofern in der lipoiden Phase vorhanden, die Glycosphingolipide aus einer lipoiden Phase und nicht aus einer wäßrigen Phase mittels der hierin offenbarten Salze in die wäßrige Phase überführt und lassen sich durch Abtrennung der wässrigen Phase von der lipoiden Phase abtrennen und danach aus der wässrigen Phase gewinnen. Bei dem erfindungsgemäßen Verfahren befinden sich die abzutrennenden Stoffe immer in einer lipoiden Phase. Diese lipoide Phase wird mit einer wässrigen Lösung eines oder mehrerer Salze versetzt, welche(s) als Anion(en) in Wasser Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$) , Acetat ($CH_3COO^-$), Borat ($BO_3^{3-}$) und/oder Tartrat ($C_4H_4O_6^{2-}$) bildet/bilden und die Glycoglycerolipide und sofern vorhanden, die Glycosphingolipide, werden durch dieses Anion oder durch diese Anionen in die wässrige Phase überführt. Bei diesem Extraktionsschritt finden vorzugsweise keine zusätzlichen organsichen Lösungsmittel Anwendung. Somit kann der zusätzliche Einsatz von organischen Lösungsmitteln auch ausgeschlossen werden.

Zudem haben die Erfinder eine Vielzahl an Salzen mit verschiedenen Anionen getestet, und erfindungsgemäß sind nur die hierin offenbarten Anionen in der Lage, die Glycoglycerolipide und Glycosphingolipide in die wässrige Phase zu überführen. Anionen wie z.B. Chlorid, Bromid, Iodid, Nitrat, Nitrit, Nitrid, Sulfat, Sulfit, Sulfid, Phosphat und viele andere sind dazu nicht in der Lage und daher erfindungsgemäß nicht einsetzbar.

In einer bevorzugten Ausführungsform der vorliegenden Erfindung enthält die in Schritt B1) zugesetzte wässrige Phase die oben genannten Anionen in Form ihrer Natriumsalze.

In einer bevorzugten Ausführungsform der vorliegenden Erfindung enthält die in Schritt B1) zugesetzte wässrige Phase neben den oben genannten Anionen außer Chlorid- und/oder Bromidionen keine weiteren Anionen.

Es wird daher erfindungsgemäß bevorzugt, wenn die unter Schritt B1) zugesetzte wässrige Phase abgesehen von Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$) , Acetat ($CH_3COO^-$), Borat ($BO_3^{3-}$) oder Tartrat ($C_4H_4O_6^{2-}$) keine weiteren Anionen, d.h. kein Phosphat, kein Iodid, kein Fluorid, kein Nitrit, kein Nitrat, kein Hydrogenphosphat, kein Dihydrogenphosphat und kein Cyanid enthält. Als Kationen enthält die wässrige Phase vorzugsweise $Na^+$, $K^+$, $Li^+$, $Mg^{2+}$, $Ca^{2+}$, $Ti^{2+}$, $Ti^{4+}$, $Co^{2+}$, $Co^{3+}$, $Ni^{2+}$, $Cu^{2+}$, $Zn^{2+}$, $Sn^{2+}$ und/oder $Sn^{4+}$ und insbesondere bevorzugt nur $Na^+$ und/oder $K^+$.

Dem Fachmann ist jedoch bewusst, dass je nach Quelle und Qualität des verwendeten Wassers für die Herstellung der unter Schritt B1) als auch unter Schritt A2) oder Schritt A2') oder Schritt E1) zugesetzten wässrigen Phase unvermeidbare Verunreinigungen in Form anderer Kationen und/oder Anionen vorhanden sein können.

Des Weiteren ist bevorzugt, wenn in der zugesetzten wässrigen Phase eines oder mehrere der folgenden Kationen anwesend ist/sind: $Li^+$, $Mg^{2+}$, $Ca^{2+}$, $Ti^{2+}$, $Ti^{4+}$, $Co^{2+}$, $Co^{3+}$, $Ni^{2+}$, $Cu^{2+}$, $Zn^{2+}$, $Sn^{2+}$ oder $Sn^{4+}$. Vorzugsweise wird daher in Schritt B1) die lipoide Phase mit einer wässrigen Phase versetzt, welche Kationen eines Salzes enthält, welches in Wasser bei 20°C eine Löslichkeit von mindestens 30 g/L besitzt und bei der Dissoziation in Wasser $Li^+$, $Mg^{2+}$, $Ca^{2+}$, $Ti^{2+}$, $Ti^{4+}$, $Co^{2+}$, $Co^{3+}$, $Ni^{2+}$, $Cu^{2+}$, $Zn^{2+}$, $Sn^{2+}$ oder $Sn^{4+}$ Ionen bildet. Natürlich versteht sich von selbst, dass diese wässrigen Phasen auch Aniopnen zu den vorgenannten Kationen enthalten wie z.B. Sulfat, Bromid, Acetat. Die Zugabe von Eisenionen sollte vermieden werden.

Geeignete Salze, welche der zuzusetzenden wässrigen Phase zugegeben werden können, sind beispielsweise:

LiOAc, LiBr, $MgBr_2$, $CaBr_2$, $Mg(OAc)_2$, $Ca(OAc)_2$, $MgCO_3$, $CaCO_3$, $Ti(OAc)_2$, $Ti(OAc)_4$, $Ni(OAc)_2$, $TiBr_2$, $TiBr_4$, $CoBr_2$, $CoBr_3$, $NiBr_2$, $CuBr_2$, $Cu(OAc)_2$, $ZnBr_2$, $Zn(OAc)_2$, $Sn(OAc)_2$, $SnBr_2$, $Sn(OAc)_2$ oder $SnBr_4$.
Die Verwendung von Chloridsalzen wie beispielweise NaCl sollte vermieden werden, weil Chloridsalze bekanntermaßen ein korrosives Verhalten aufweisen und die verarbeitende Vorrichtung angreifen, welche vorzugsweise aus Stahl gefertigt sind. Somit ist der Einsatz von Chloridsalzen wie z.B. LiCl, $MgCl_2$, $CaCl_2$, NaCl, $TiCl_2$, $TiCl_4$, $CoCl_2$, $CoCl_3$, $NiCl_2$, $CuCl_2$, KCl, $ZnCl_2$, $SnCl_2$ und $SnCl_4$ zu vermeiden. Vorzugsweise ist der Einsatz von Chloridsalzen erfindungsgemäß ausgeschlossen.

Die in Schritt B1) zuzusetzende wässrige Phase hat vorzugsweise einen pH-Wert im Bereich von 7,0 bis 13,5, weiter bevorzugt von 7,5 bis 12,0, weiter bevorzugt von 8,0 bis 11,0. Der pH-Wert kann, falls erforderlich, mittels Zugabe z. B. von Essigsäure eingestellt werden. Der pH-Wert hängt natürlich auch von dem zugesetzten Salz ab. Bei der Verwendung von Metasilicat (MS) liegt der pH-Wert vorzugsweise im Bereich von 12,0 bis 13,5, vorzugsweise 12,5 bis 13,5.
Bei der Verwendung von Carbonat (Natriumcarbonat: NC) liegt der pH-Wert vorzugsweise im Bereich von 10,0 bis 12,0, vorzugsweise 10,5 bis 11,5.
Bei der Verwendung von Acetat (Ac; Natriumacetat: NAc) liegt der pH-Wert vorzugsweise im Bereich von 7,0 bis 9,0, vorzugsweise 7,5 bis 8,5.
Bei der Verwendung von Hydrogencarbonat (HC; Natriumhydrogencarbonat: NHC) liegt der pH-Wert vorzugsweise im Bereich von 7,0 bis 9,0, vorzugsweise 7,5 bis 8,5.

Die in Schritt E1) zuzusetzende wässrige Phase hat vorzugsweise einen pH-Wert im Bereich von 10,0 bis 14,0, weiter bevorzugt von 11,0 bis 13,7, weiter bevorzugt von 12,0 bis 13,5. Der pH-Wert kann, falls erforderlich, mittels Zugabe von z. B. Essigsäure eingestellt werden.

Die Zugabe der wässrigen Phase zur lipoiden Phase erfolgt vorzugsweise bei Raumtemperatur oder bei einer Temperatur im Bereich von 10°C bis 50°C.

Die Durchmischung findet bei Atmosphärendruck und einer Temperatur im Bereich von 10°C bis 90°C, bevorzugt 15°C bis 70°C, weiter bevorzugt 20°C bis 60°C und insbesondere bevorzugt 25°C bis 50°C statt. Die Abtrennung der wässrigen Phase nach Durchmischung erfolgt vorzugsweise bei Atmosphärendruck und einer Temperatur im Bereich von 10°C bis 90°C, bevorzugt 15°C bis 70°C, weiter bevorzugt 20°C bis 60°C und insbesondere bevorzugt 25°C bis 50°C.

Da die erfindungsgemäße Abtrennung von Glycoglycerolipiden und Glycosphingolipiden aus lipoiden Phasen erfolgen kann, in denen aus vorgenannten Gründen auch hydratisierbare leicht wasserlösliche Verbindungen enthalten sein können, kann es von Interesse sein, diese Verbindungen separat abzutrennen. Hierzu kann vor Zugabe der in den vorherigen Absätzen bezeichneten wässrigen Phase gemäß Schritt B1) nach Schritt A1) noch folgender Schritt A2) folgen:

A2) Versetzen der lipoiden Phase mit Wasser als wässrige Phase, nachfolgendes Durchmischen der lipoiden Phase und der wässrigen Phase und Abtrennung der wässrigen Phase.

Somit ist ein Aspekt der vorliegenden Erfindung auf ein Verfahren gerichtet, welches nach Schritt A1) und vor Schritt B1) den folgenden Schritt A2) umfasst:

A2) Versetzen der lipoiden Phase mit Wasser als wässrige Phase, nachfolgendes Durchmischen der lipoiden Phase und der wässrigen Phase und Abtrennung der wässrigen Phase.

Anstelle von Wasser als wässriger Phase oder neutraler wässriger Phase kann auch eine saure wässrige Phase eingesetzt werden, welches beispielsweise Zitronensäure, Phosphorsäure, Essigsäure, Ameisensäure oder Oxalsäure enthält. Daher ist eine weitere mögliche Variante der vorliegenden Erfindung auf ein Verfahren gerichtet, welches nach Schritt A1) und vor Schritt B1) den folgenden Schritt A2') umfasst:

A2') Versetzen der lipoiden Phase mit einer wässrigen Carbonsäure-Lösung oder einer wässrigen Lösung einer anorganischen Säure mit einem pH-Wert zwischen 3,0 bis 5,0 als wässrige Phase, nachfolgendes Durchmischen der lipoiden Phase und der wässrigen Phase und Abtrennung der wässrigen Phase.

Als anorganische Säuren eignen sich beispielsweise Phosphorsäure, Schwefelsäure als auch Salzsäure. In einigen Ausführungsformen der vorliegenden Erfindung kann es von Vorteil und somit bevorzugt sein, wenn als erster Schritt nach der Bereitstellung einer lipoiden Phase enthaltend Acylglyceride, Glycolipide, Glycoglycerolipide, Glycophospholipide, Phospholipide und freien Fettsäuren der Schritt A2) oder A2') durchgeführt wird. Dies gilt besonders für lipoide Phasen, die neben Acylglyceriden, Glycolipiden, Glycoglycerolipiden, Glycophospholipiden besonders viele Phospholipide enthalten und welche gut durch einen Schritt A2) oder A2') abgetrennt werden können. Nach Durchmischen der lipoiden Phase mit einer wässrigen Phase in Form von destilliertem Wasser bzw. einer schwachen Säure,

wird die resultierende wässrige Phase von der lipoiden Phase abgetrennt und kann verworfen oder zur weiteren Verwendung gesammelt werden. Hierdurch können hydrophile Substanzen wie Salze, aber auch leicht hydrolysierbare Phospholipide (z.B. Phosphatidylcholin, auch als Lecithin bezeichnet) oder Glycolipide mit Carboxylat-, Sulfat- und/oder Sulfonatgruppe(n) abgetrennt werden. Fettsäuren, Glycoglycerolipide und Glycophospholipide werden hierdurch gar nicht oder nur in einem sehr geringen Maße abgetrennt.
Durch die Abtrennung von hydratisierbaren Verbindungen aus einer lipoiden Phase wird die Gewinnung einer reineren Form von Glycoglycerolipiden und Glycophospholipiden ermöglicht, was die weitere Aufbereitung der abgetrennten Fraktion der Glycoglycerolipide und Glycophospholipide wesentlich erleichtert. Daher sind die Verfahrensschritte A1) und A2) besonders bevorzugte Ausführungsformen um eine reinere Form von Glycoglycerolipiden und Glycophospholipiden mit den erfindungsgemäßen Verfahrensschritten B1) und B2) zu erhalten. Somit betrifft die vorliegende Offenbarung auch Gemische, enthaltend Glycoglycerolipide, Glycosphingolipide, Sterylglycoside oder Kombinationen der vorgenannten Stoffe wie z.B. Glycoglycerolipide und Glycosphingolipide oder Glycoglycerolipide und Glycosphingolipide und Sterylglycoside, erhältlich nach einem der Verfahren wie hierin offenbart.
Mit den Verfahrensschritten gemäß A), A1) und A2) werden allerdings neben Glycoglycerolipiden und Glycosphingolipiden auch keine anderen nicht hydratisierbaren Verbindungen wie freie Fettsäuren oder Carbonsäuren aus den lipoiden Phasen entfernt. Bei einer Abtrennung der Glycoglycerolipide und Glycosphingolipide gemäß den Verfahrensschritten B), B1) und B2) kann es zu der Mitabtrennung von freien Fettsäuren und Phospholipiden bzw. Glycophospholipiden kommen, was die Qualität der abgetrennten Glycoglycerolipide und Glycosphingolipide ungünstig beeinträchtigt. Es konnte gezeigt werden, dass insbesondere die Mitabtrennung von freien Fettsäuren, Carbonsäuren und Phospholipiden durch eine geeignete Auswahl an Prozessparametern gesteuert werden kann. Dies betrifft insbesondere die Einstellung des pH-Werts der beschriebenen wässrigen Salzlösungen. So konnte gezeigt werden, dass auch bei einem hohen Gehalt an freien Fettsäuren in der lipoiden Phase, die mit den Verfahrensschritten B), B1) und B2) durchgeführten Abtrennungen der enthaltenen Glycoglycerolipiden und Glycosphingolipiden der Gehalt der freien Fettsäuren und Phospholiden bzw. Glycophospholipiden im Wesentlichen unverändert bleibt, wenn der pH-Wert der wässrigen Lösung auf ein neutrales Niveau eigestellt wurde. Hierdurch ist die Gewinnung einer besoders vorteilhaften und reinen Fraktion an Glycoglycerolipiden und Glycosphingolipiden mit einem wässrigen Abtrennungsverfahren erstmalig möglich, sodass die eine besonders bevorzugte Ausführungsform der Verfahrensschritte B), B1) und B2) mit wässrigen Lösungen der hierin genannten Anionen oder Salze mit einem neutralen oder um den Neurtalbereich liegenden pH-Wert ist.

Die lipoiden Phasen, die mit den offenbarten Vorrichtungen und Verfahren zur Gewinnung einer hydrolysearmen und reinen Fraktion von Glycoglycerolipiden und Glycosphingolipiden behandelt werden können, sind bei einigen großtechnischen Anwendungen auch Gemische, die selbst von wirtschaftlichem Interesse sind. Dies betrifft insbesondere Pflanzenöle. Es konnte nun erstmalig gezeigt werden, dass die Entfernung von Glycoglycerolipiden und Glycophospholipiden sich relevant auf die Weiterverarbeitung dieser lipoiden Phasen auswirkt. So konnte bisher mit noch keinem wässrigen Extraktionsverfahren eine Abreicherung von freien Fettsäuren aus lipoiden Phasen, die zu >90 Gew.-%, bevorzugt zu >95 Gew.-% und weiter bevorzugt zu >98 Gew.-% aus Triacylglycerolen bestehen, auf werten < 0,1 Gew.-% gezeigt worden.

Überraschenderweise konnte nunmehr gefunden werden, dass durch eine im Anschluss an die wässrige Extraktion von Glycoglycerolipiden und Glycosphingolipiden gemäß den Schritten B1) bis D1) durchgeführte Extraktion mit einer wässrigen Lösung von Guanidino- oder Amidinoverbindungen eine praktisch vollständige Entfernung noch in einem Alkan- oder Triglygeridgemisch verbliebenen freien Fettsäuren und Phospholipiden möglich ist. Eine derartige Reduktion von Fettsäuren und Phospholipiden konnte mit den gleichen Amidino- oder Guanidinoverbindungen, die im Anschluss an ein klassisches Verfahren aus dem Stand der Technik mit dem Alkan- oder Triglyceridgemisch in Verbindung gebracht wurden, nicht erreicht werden. Daher stellt die Kombination einer erfindungsgemäßen wässrigen Extraktion gemäß den Schritten B1) bis D1) und der zusätzlichen wässrigen Extraktion mit einer Amidino- oder Guanidinoverbindung ein besonders vorteilhaftes Verfahren zur Erlangung einer optimalen Reduktion von Fettsäuren und Phospholipiden dar.
Gleichzeitig kann hierdurch eine äußerst vorteilhafte weitere Reduktion der mit den erfindungsgemäßen Vorrichtungen und Verfahren behandelten lipoiden Phase ermöglicht werden, sodass die Erfindung auch auf die Gewinnung einer hochveredelten glycoglycerolipidarmen lipoiden Phase gerichtet ist.
Hierdurch können Triglycerid- und Alkangemische erhalten werden, die Restgehalte von freien Fettsäuren und Phospholipiden aufweisen, die deutlich unter den aktuellen DIN-Anforderungen an z.B. die Qualität von biogenen Kraftstoffen wie z.B. Biodiesel liegen. Dies gilt auch für die Maximalwerte von Erdalkalimetallen und Metallionen, die allerdings bereits nach dem Extraktionsverfahren gemäß den Schritten B1) bis D1) reduziert sind. Gleichwohl ist eine weitere Reduktion durch die zusätzliche wässrige Extraktion mit einer Amidino- oder Guanidinverbindung möglich.

Eine insbesondere bevorzugte Ausführungsform der vorliegenden Erfindung betrifft daher Verfahren, welche nach Schritt D1) den folgenden Schritt E1) umfassen:

E1) Versetzen der glycoglycerolipidarmen lipoiden Phase mit einer wässrigen Phase, enthaltend mindestens eine

Verbindung, welche mindestens eine Amidinogruppe und/oder mindestens eine Guanidinogruppe aufweist, nachfolgendes Durchmischen der glycoglycerolipidarmen lipoiden Phase und der wässrigen Phase und Abtrennung der wässrigen Phase.

Sofern das erfindungsgemäße Verfahren den Schritt D2) umfasst, ist eine insbesondere bevorzugte Ausführungsform der vorliegenden Erfindung auf Verfahren gerichtet, welche nach Schritt D2) den folgenden Schritt E1) umfassen:

E1) Versetzen der glycoglycerolipidarmen lipoiden Phase mit einer wässrigen Phase, enthaltend mindestens eine Verbindung, welche mindestens eine Amidinogruppe und/oder mindestens eine Guanidinogruppe aufweist, nachfolgendes Durchmischen der glycoglycerolipidarmen lipoiden Phase und der wässrigen Phase und Abtrennung der wässrigen Phase.

Beispiele für geeignete Verbindungen mit mindestens einer Guanidinogruppe (auch Guanidinoverbindungen genannt) und/oder mit mindestens einer Amidinogruppe (auch Amidinoverbindungen genannt) sind eingehend in der internationalen Patentanmeldung WO 2011160857 A2 offenbart. Als Guanidinogruppe wird der chemische Rest $H_2N$-C(NH)-NH- sowie dessen cyclische Formen bezeichnet und als Amidinogruppe der chemische Rest $H_2N$-C(NH)- sowie dessen cyclische Formen (s. Beispiele unten). Bevorzugt sind Guanidinoverbindungen, welche zusätzlich zur Guanidinogruppe mindestens eine Carboxylatgruppe (-COOH) aufweisen. Ferner ist bevorzugt, wenn die Carboxylatgruppe(n) durch mindestens ein Kohlenstoffatom von der Guanidinogruppe im Molekül getrennt sind. Bevorzugt sind auch Amidinoverbindungen, welche zusätzlich zur Amidinogruppe mindestens eine Carboxylatgruppe (-COOH) aufweisen. Ferner ist bevorzugt, wenn die Carboxylatgruppe(n) durch mindestens ein Kohlenstoffatom von der Amidinogruppe im Molekül getrennt sind.

Diese Guanidinoverbindungen und Amidinoverbindungen haben vorzugsweise einen Verteilungskoeffizienten $K_{OW}$ zwischen n-Octanol und Wasser von keiner 6,3 ($K_{OW} < 6,3$).

Insbesondere bevorzugt sind Argininderivate. Argininderivate sind definiert als Verbindungen, welche eine Guanidinogruppe und eine Carboxylatgruppe oder eine Amidinogruppe und eine Carboxylatgruppe aufweisen, wobei Guanidinogruppe und Carboxylatgruppe oder Amidinogruppe und Carboxylatgruppe durch mindestens ein Kohlenstoffatom voneinander entfernt sind, d.h. sich zumindest eine der folgenden Gruppen zwischen der Guanidinogruppe oder der Amidinogruppe und der Carboxylatgruppe befindet: $-CH_2-$, -CHR-, -CRR'-, worin R und R' unabhängig voneinander beliebige chemische Reste darstellen. Natürlich kann der Abstand zwischen der Guanidinogruppe und der Carboxylatgruppe oder der Amidinogruppe und der Carboxylatgruppe auch mehr als ein Kohlenstoffatom betragen, beispielweise bei folgenden Gruppen $-(CH_2)_n-$, $-(CHR)_n-$, $-(CRR')_n-$, mit n = 2, 3, 4, 5, 6, 7, 8 oder 9 wie es z.B. bei Amidinopropionsäure, Amidinobuttersäure, Guanidinopropionsäure oder Guanidinobuttersäure der Fall ist. Verbindungen mit mehr als einer Guanidinogruppe und mehr als einer Carboxylatgruppe sind beispielsweise Oligoarginin und Polyarginin.

Im Folgenden werden Beispiele für bevorzugte Verbindungen mit einer Guanidinogruppe oder einer Amidinogruppe und einer Carboxylatgruppe gezeigt.

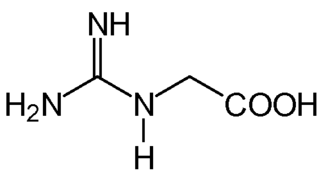

Guanidinoessigsäure

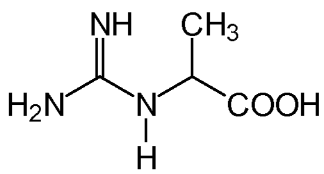

Creatin

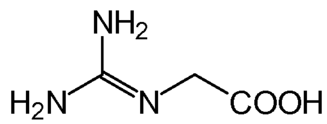

Glycocyamin

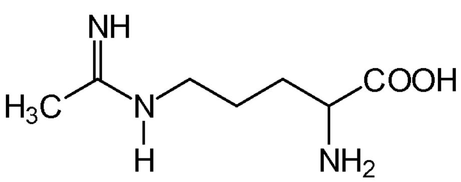

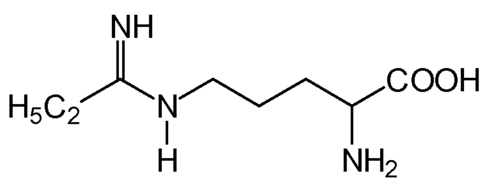

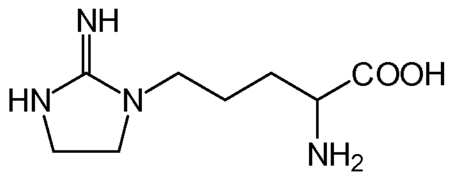

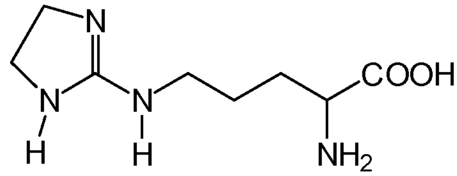

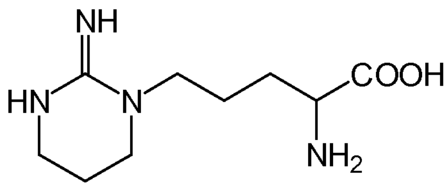

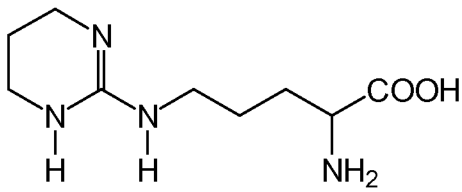

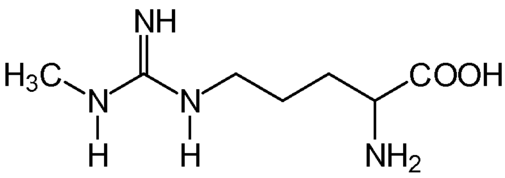

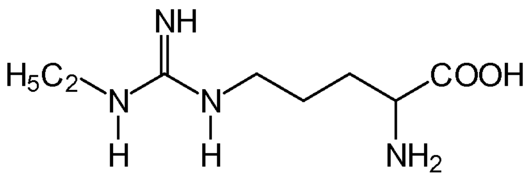

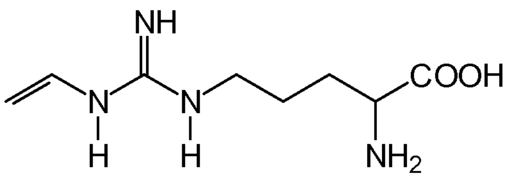

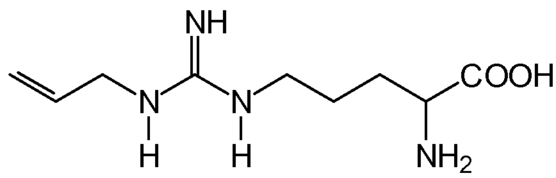

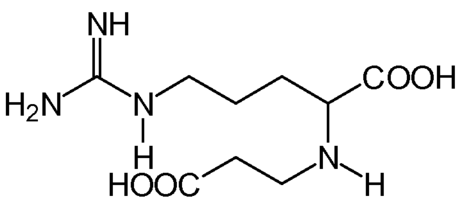

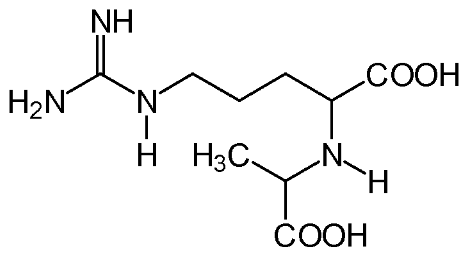

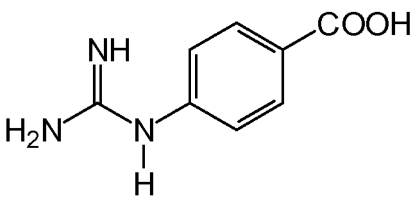

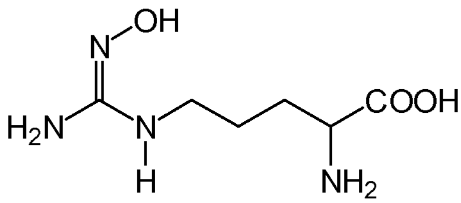

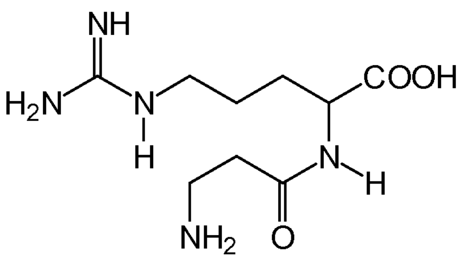

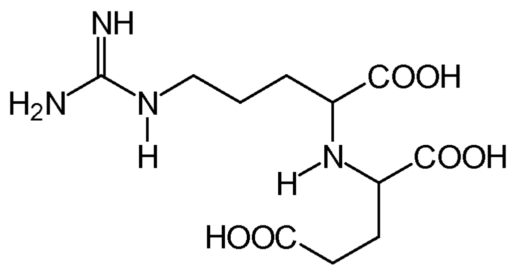

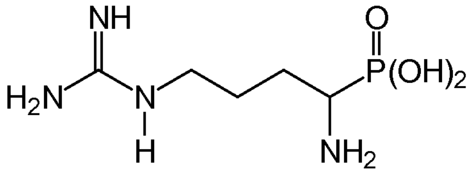

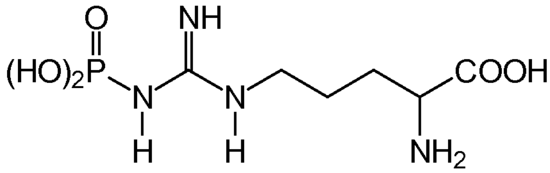

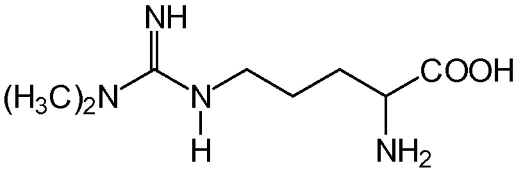

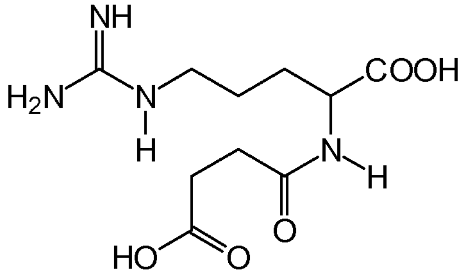

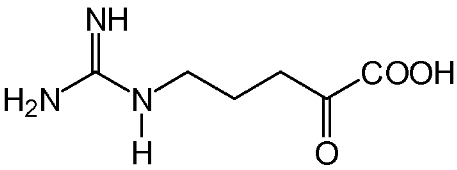

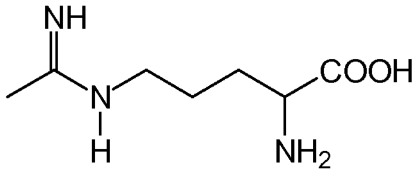

L-NIL

Bevorzugte Argininderivate sind Verbindungen der folgenden allgemeinen Formel (I) oder (II)

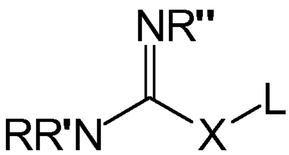

**(I)**

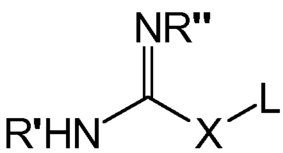

**(II)**

worin

R’, R", R’" und R"" unabhängig voneinander bedeuten: -H, -OH, -CH=$CH_2$, -$CH_2$-CH=$CH_2$, -C($CH_3$)=$CH_2$, -CH=CH-$CH_3$, -$C_2H_4$-CH=$CH_2$, -$CH_3$, -$C_2H_5$, -$C_3H_7$, -CH($CH_3$)$_2$, -$C_4H_9$, -$CH_2$-CH($CH_3$)$_2$, -CH($CH_3$)-$C_2H_5$, -C($CH_3$)$_3$, -$C_5H_{11}$, -CH($CH_3$)-$C_3H_7$, -$CH_2$-CH($CH_3$)-$C_2H_5$, -CH($CH_3$)-CH($CH_3$)$_2$, -C($CH_3$)$_2$-$C_2H_5$, -$CH_2$-C($CH_3$)$_3$, -CH($C_2H_5$)$_2$, -$C_2H_4$-CH($CH_3$)$_2$, -$C_6H_{13}$, -$C_7H_{15}$, cyclo-$C_3H_5$, cyclo-$C_4H_7$, cyclo-$C_5H_9$, cyclo-$C_6H_{11}$, -$PO_3H_2$, -$PO_3H^-$, -$PO_3^{2-}$, -$NO_2$, -C≡CH, -C≡C-$CH_3$, -$CH_2$-C≡CH, -$C_2H_4$-C≡CH, -$CH_2$-C≡C-$CH_3$,

oder R’ und R" zusammen eine der folgenden Gruppen bilden: -$CH_2$-$CH_2$-, -CO-$CH_2$-, -$CH_2$-CO-, -CH=CH-, -CO-CH=CH-, -CH=CH-CO-, -CO-$CH_2$-$CH_2$-, -$CH_2$-$CH_2$-CO-, -$CH_2$-CO-$CH_2$- or -$CH_2$-$CH_2$-$CH_2$-;

X für -NH-, -NR""-, -O-, -S-, -$CH_2$-, -$C_2H_4$-, -$C_3H_6$-, -$C_4H_8$- oder -$C_5H_{10}$-steht oder für eine C1 bis C5 Kohlenstoffkette, welche mit einem oder mehreren der folgenden Reste substituiert sein kann: -F, -Cl, -OH, -$OCH_3$, -$OC_2H_5$, -$NH_2$, -$NHCH_3$, -NH($C_2H_5$), -N($CH_3$)$_2$, -N($C_2H_5$)$_2$, -SH, -$NO_2$, -$PO_3H_2$, -$PO_3H$ , -$PO_3^2$, -$CH_3$, -$C_2H_5$, -CH=$CH_2$, -C=CH, -COOH, -$COOCH_3$, -$COOC_2H_5$, -$COCH_3$, -$COC_2H_5$, -O-$COCH_3$, -O-$COC_2H_5$, -CN, -$CF_3$, -$C_2F_5$, -$OCF_3$, -$OC_2F_5$;

L einen hydrophilen Substituenten bedeutet ausgewählt aus der Gruppe bestehend aus:

-$NH_2$, -OH, -$PO_3H_2$, -$PO_3H$ , -$PO_3^{2-}$, -$OPO_3H_2$, -$OPO_3H$ , -$OPO_3^{2-}$, -COOH, -$COO^-$, -CO-$NH_2$, -$NH_3^+$, -NH-CO-$NH_2$, -N($CH_3$)$_3^+$, -N($C_2H_5$)$_3^+$, -N($C_3H_7$)$_3^+$, -NH($CH_3$)$_2^+$, -NH($C_2H_5$)$_2^+$, -NH($C_3H_7$)$_2^+$, -$NHCH_3$, -$NHC_2H_5$, -$NHC_3H_7$, -$NH_2CH_3^+$, -$NH_2C_2H_5^+$, -$NH_2C_3H_7^+$, -$SO_3H$, -$SO_3^-$, -$SO_2NH_2$, -CO-COOH, -O-CO-$NH_2$, -C(NH)-$NH_2$, -NH-C(NH)-$NH_2$, -NH-CS-$NH_2$, -NH-COOH,

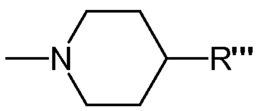 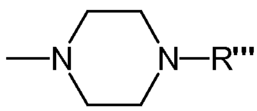 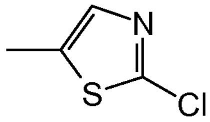 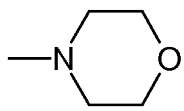

Der Verfahrensschritt E1) eignet sich insbesondere, um weiter aufbereitete lipoide glycolipid- und carbonsäurearme Phasen zu erhalten, welche gleichzeitig nur noch minimale Restmengen an Kalium, Phosphor, Eisen, Calcium, freien Fettsäuren, Glycoglycerolipiden und Glycosphingolipiden aufweisen. Somit ist ein weiterer Aspekt der vorliegenden Erfindung auf cabonsäurearme lipoide glycolipid- und carbonsäurearme Phasen gerichtet, welche nach einem erfindungsgemäßen Verfahren erhalten werden.

Der Schritt E1) kann auch als Schritt A2) anstelle des Waschschrittes mit Wasser oder des Waschschrittes mit einer wässrigen Carbonsäure-Lösung oder einer wässrigen Phosphorsäurelösung durchgeführt werden oder als Schritt A3) nach einem Waschschritt mit Wasser [Schritt A2)] oder als Schritt A3) nach einem Waschschritt mit wässriger Carbonsäure-Lösung oder mit wässriger Phosphorsäurelösung, Schwefelsäurelösung oder Salzsäurelösung [Schritt A2')].'Die Anwendung von Schritt E1) ist insbesondere nach dem initial erfolgten Schritt A2) besonders vorteilhaft, da hiermit, die zur Gewinnung einer besonders vorteilhaften phospholipid- und hydrolysearmen Glycoglycerolipid- und Glycosphingolipidfraktion eingetragenen Säuren, unter besonders schonenden Bedingungen und vollständig entfernt werden können, so dass hierdurch gleichzeitig unter besonders schonenden Bedingungen eine carbonsäurearme lipoide Phase gewonnen werden kann.

Erfindungsgemäß ist daher bevorzugt, einen Schritt A2) oder A2') durchzuführen, um leicht wasserlösliche Bestandteile der lipoiden Phase zu entfernen, d.h. Substanzen mit einem HLB-Wert von vorzugsweise >18, bevorzugt >16, weiter bevorzugt >15, und danach den erfindungsgemäßen Schritt B1) durchzuführen, um eine wässrige Phase mit einem möglichst hohen Reinheitsgrad der gewinnbaren Glycoglycerolipide und Glycosphingolipide zu erhalten.

Sofern erwünscht, sind die Glycoglycerolipide und Glycosphingolipide weiter in solche mit geladenen Gruppen, wie z.B. Phosphat, Sulfonat, Sulfat und solche ohne geladene Gruppe (also z.B. ohne Phosphat, Sulfonat, Sulfat) zu separieren, so wird vorzugsweise Schritt B1) mittels einer wässrigen Phase, enthaltend Anionen mindestens eines Salzes, durchgeführt, welches in Wasser bei 20°C eine Löslichkeit von mindestens 30 g/L besitzt und bei der Dissoziation in Wasser Carbonat ($CO_3^{2-}$), Metasilicat ($SiO_3^{2-}$), Orthosilicat ($SiO_4^{4-}$), Disilicat ($Si_2O_5^{2-}$), oder Trisilicat ($Si_3O_7^{2-}$) bildet, und danach die erhaltene wässrige Phase, enthaltend die Glycoglycerolipide und Glycosphingolipide, mittels geeigneter organischer Lösungsmittel wie z.B. Dimethyleter oder Chloroform extrahiert. Gegebenenfalls kann es hilfreich sein, zusätzlich polarere Lösungsmittel zur Separation einzusetzen, wie z. B. Methanol. Bei diesem weiteren Trennschritt bleiben die Glycoglycerolipide und Glycosphingolipide mit geladenen Gruppen wie z.B. Phosphatgruppen, Sulfonatgruppen oder Sulfatgruppen in der wässrigen Phase, und die Glycoglycerolipide und Glycosphingolipide ohne ionische Gruppen gehen in die organische Phase über.

Möchte man hingegen bereits aus der lipoiden Phasen eine Fraktion mit einem möglichst hohen Anteil an Glycoglycerolipiden und Glycosphingolipiden ohne ionische Gruppen abtrennen, so wird vorzugsweise vor Schritt B1) ein Waschschritt mittels Wasser [Schritt A2)] oder Säurelösung [Schritt A2')] durchgeführt.

Erfindungsgemäß lassen sich vorzugsweise die folgenden Glycoglycerolipide aus einer lipoiden Phase gewinnen:

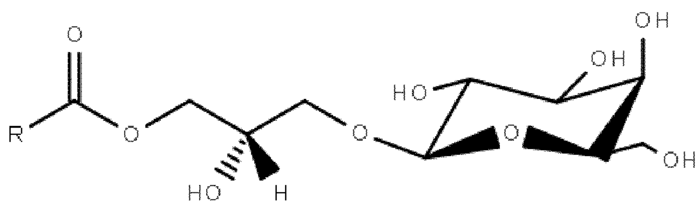

1-acyl-3-O-β-D-galactosyl-sn-glycerol

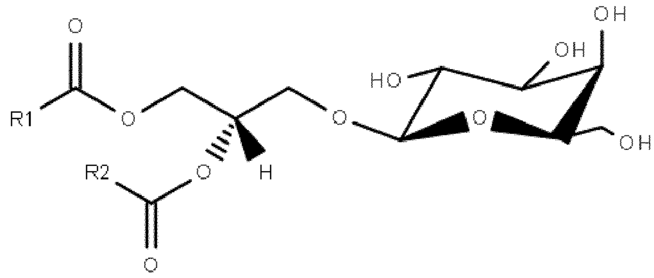

1,2 diacyl-3-O-β-D-galactosyl-sn-glycerol

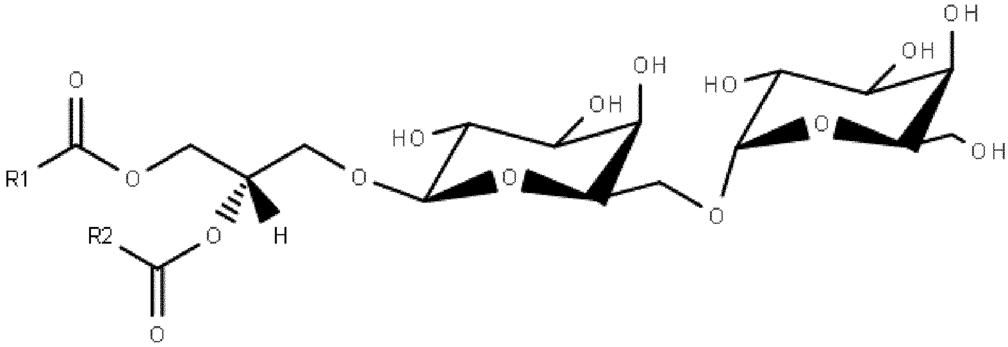

1,2 diacyl-3-O-(α-D-galactosyl1-6)-β-D-galactosyl-sn-glycerol

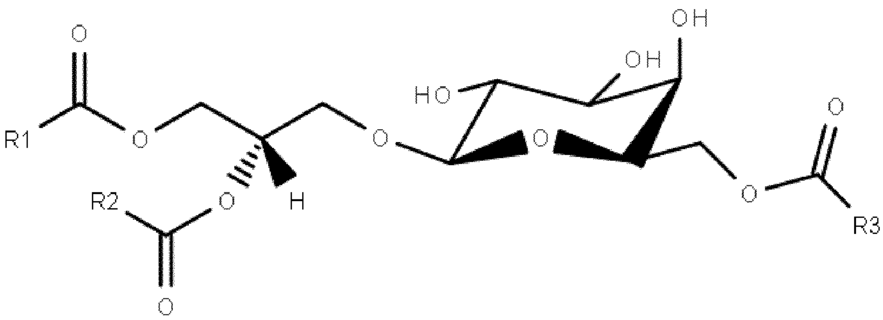

1,2-diacyl-3-O-(acyl1-6)-β-D-galactosyl-sn-glycerol

Die Reste R, R1 und R2 stehen dabei für die Kohlenstoffreste von Fettsäuren, wobei die Formeln RCOOH, R1COOH und R2COOH die entsprechenden Fettsäuren definieren. Insbesondere sind Fettsäurereste (RCOO-, R1COO- und R2COO-) mit 14 bis 24 Kohlenstoffatomen, bevorzugt 16 bis 22 Kohlenstoffatomen und weiter bevorzugt 18 bis 20 Kohlenstoffatomen bevorzugt. Zudem sind Fettsäurereste mit einer geraden Anzahl an Kohlenstoffatomen bevorzugt.

Beispiele für erfindungsgemäß aus lipoiden Phasen gewinnbare Glycoglycerolipid ohne ionische Gruppen sind beispielsweise

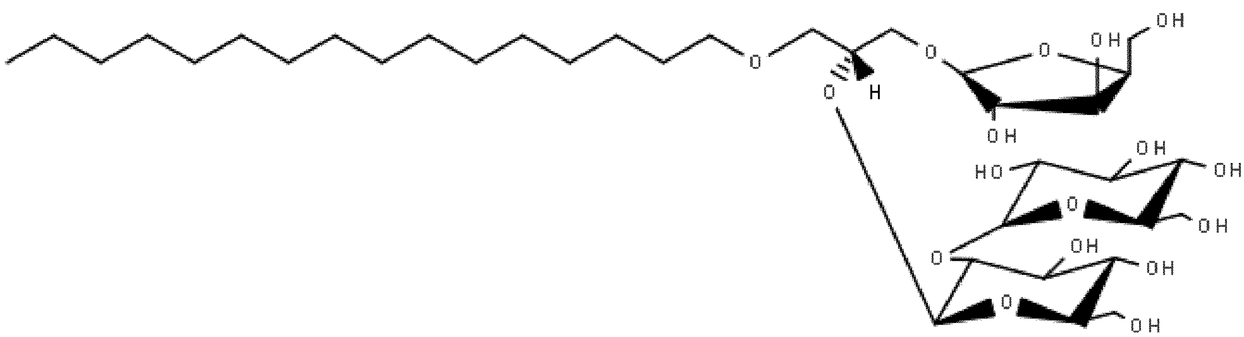

1-hexadecanyl-2-((2'-α-glucosyl)-β-glucosyl)-3-β-xylosyl-sn-glycerol

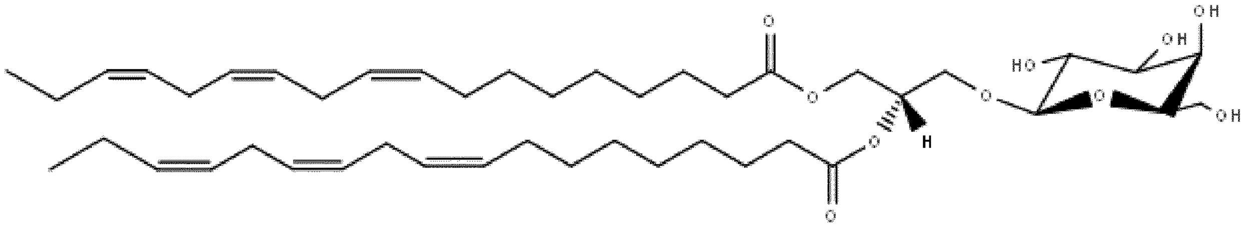

1,2 di-(9Z,12Z,15Z-octadecatrienoyl)-3-O-β-D-galactosyl-sn-glycerol

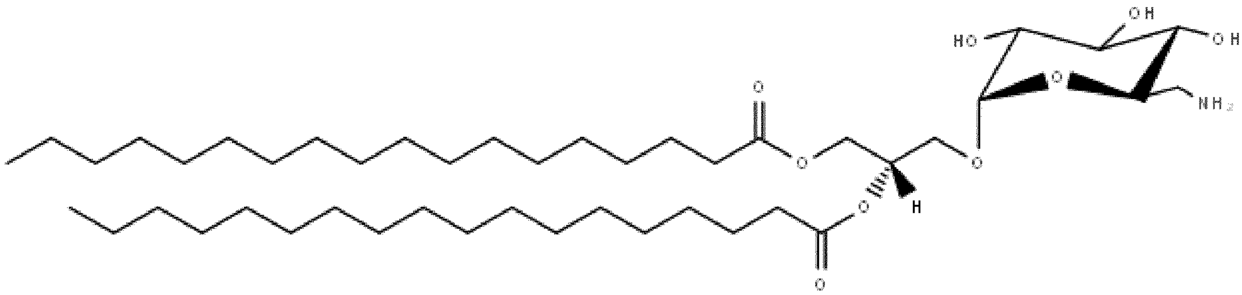

1,2-dioctadecanoyl-3-O-(6-deoxy-6-amino-α-D-glucosyl)-sn-glycerol

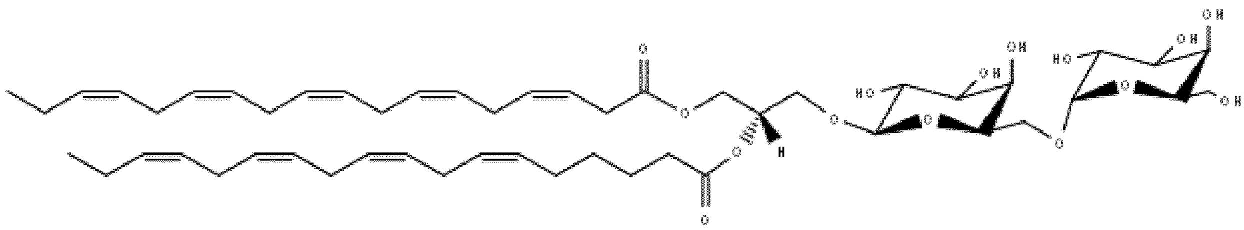

1-(3Z,6Z,9Z,12Z,15Z-octadecapentaenoyl)-2-(6Z,9Z,12Z,15Z-octadecatetraenoyl)-3-O-(6'-O-α-D-galactosyl-β-D-galactosyl)-sn-glycerol

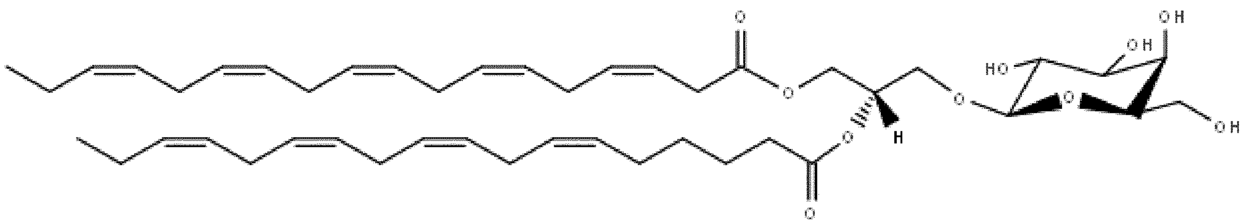

1-(3Z,6Z,9Z,12Z,15Z-octadecapentaenoyl)-2-(6Z,9Z,12Z,15Z-octadecatetraenoyl)-3-O-β-D-galactosyl-sn-glycerol

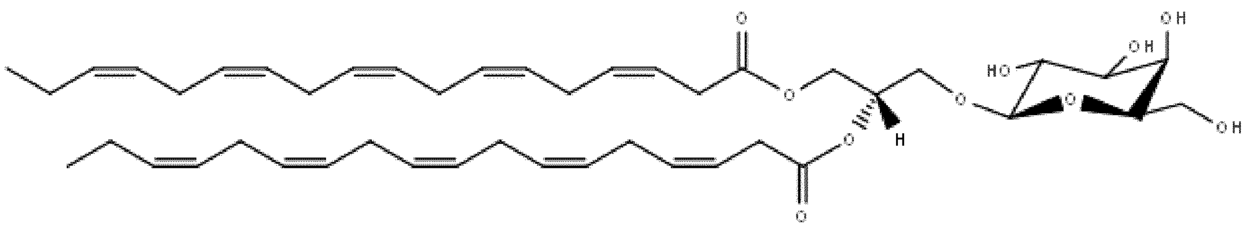

1,2-di-(3Z,6Z,9Z,12Z,15Z-octadecapentaenoyl)-3-O-β-D-galactosyl-sn-glycerol

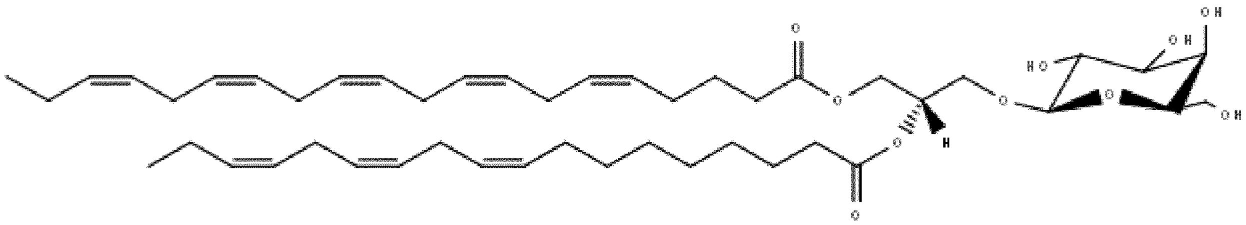

1-(5Z,8Z,11Z,14Z,17Z-eicosapentaenoyl)-2-(9Z,12Z,15Z-octadecatrienoyl)-3-O-β-D-galactosyl-sn-glycerol

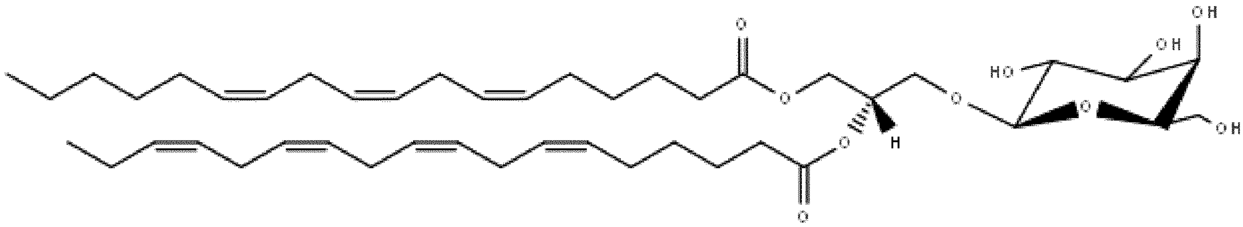

1-(9Z,12Z,15Z-octadecatrienoyl)-2-(6Z,9Z,12Z,15Z-octadecatetraenoyl)-3-O-β-D-galactosyl-sn-glycerol

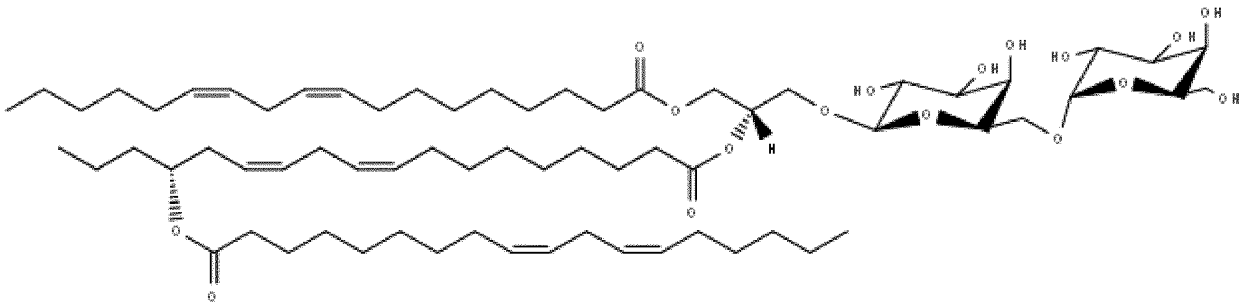

1-(9Z,12Z-octadecadienoyl)-2-(15R-[9Z,12Z-octadecadienoyloxy]-9Z,12Z-octadecadienoyl)-3-(α-D-galactosyl-1-6-β-D-galactosyl)-sn-glycerol

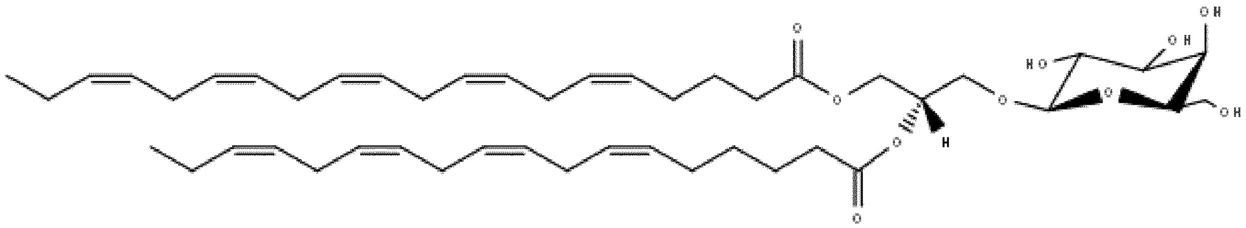

1-(5Z,8Z,11Z,14Z,17Z-eicosapentaenoyl)-2-(6Z,9Z,12Z,15Z-octadecatetraenoyl)-3-O-β-D-galactosyl-sn-glycerol

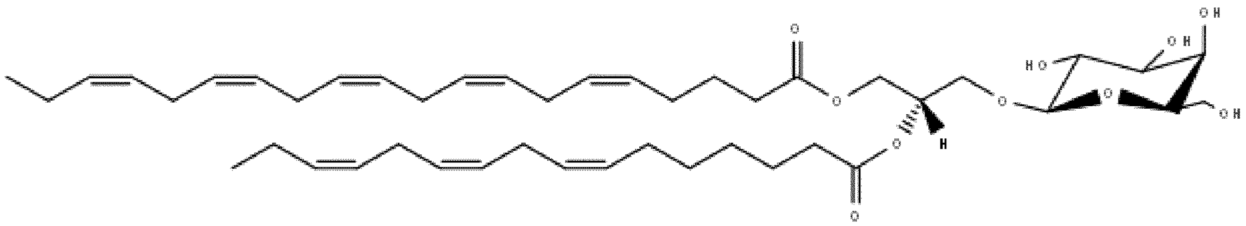

1-(5Z,8Z,11Z,14Z,17Z-eicosapentaenoyl)-2-(7Z,10Z,13Z-hexadecatrienoyl)-3-O-β-D-galactosyl-sn-glycerol

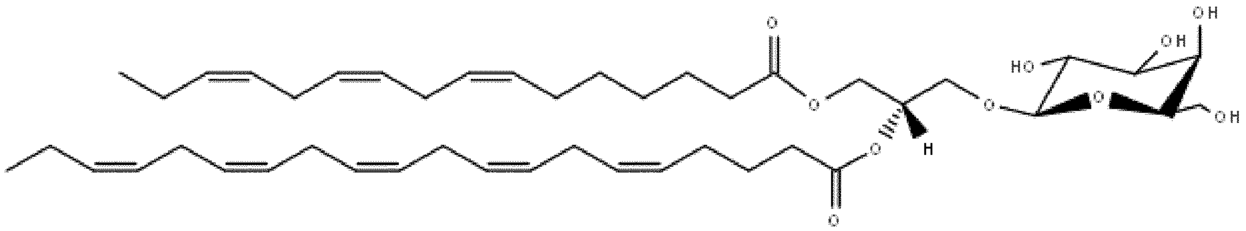

1-(7Z,10Z,13Z-hexadecatrienoyl)-2-(5Z,8Z,11Z,14Z,17Z-eicosapentaenoyl)-3-O-β-D-galactosyl-sn-glycerol

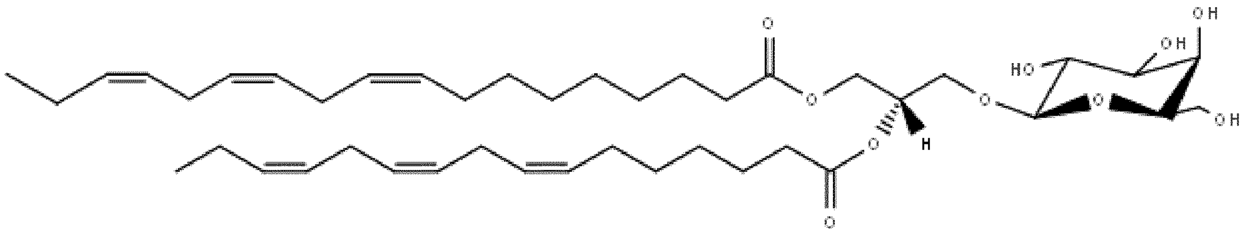

1-(9Z,12Z,15Z-octadecatrienoyl)-2-(7Z,10Z,13Z-hexadecatrienoyl)-3-O-β-D-galactosyl-sn-glycerol

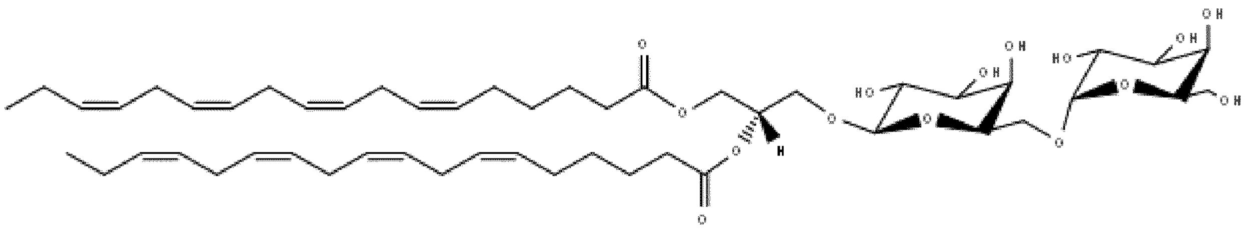

1,2-di-(6Z,9Z,12Z,15Z-octadecatetraenoyl)-3-O-β-D-galactosyl-sn-glycerol

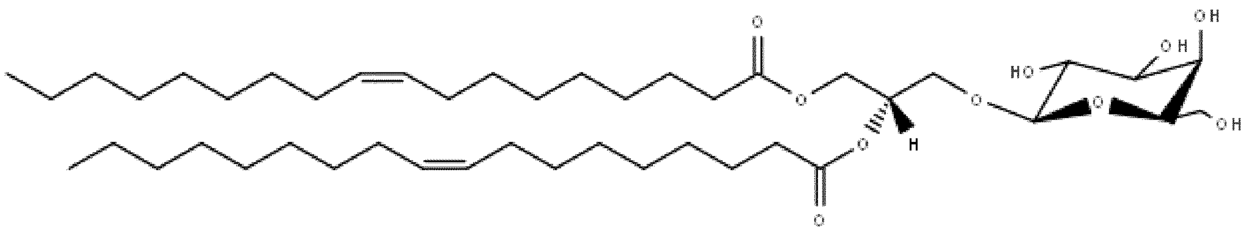

1,2 di-(9Z-octadecenoyl)-3-O-β-D-galactosyl-sn-glycerol

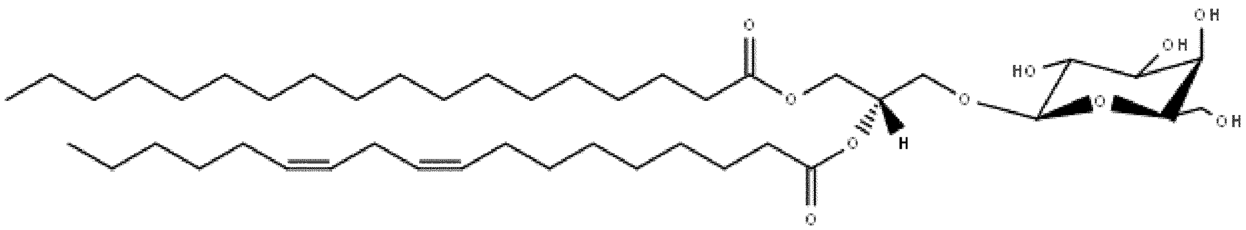

1-octadecanoyl-2-(9Z,12Z-octadecadienoyl)-3-O-β-D-galactosyl-sn-glycerol

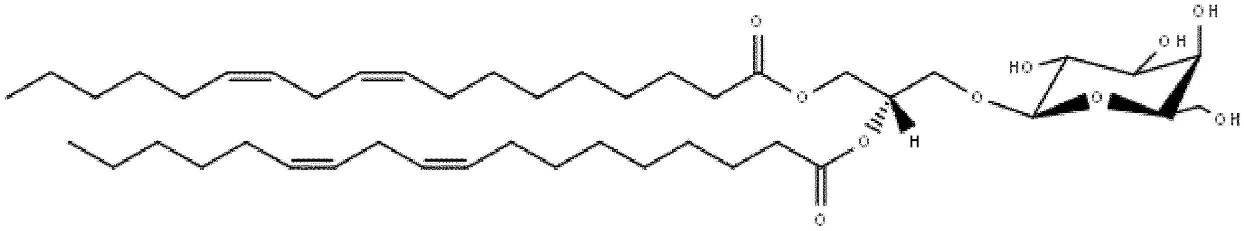

1,2-di-(9Z,12Z-octadecadienoyl)-3-O-β-D-galactosyl-sn-glycerol

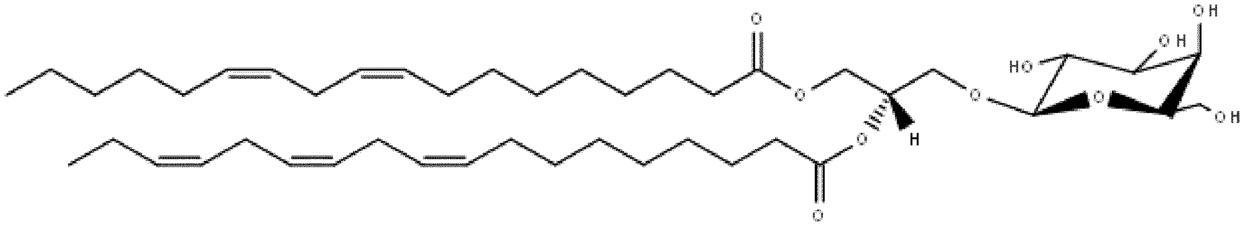

1-(9Z,12Z-octadecadienoyl)-2-(9Z,12Z,15Z-octadecatrienoyl)-3-O-β-D-galactosyl-sn-glycerol

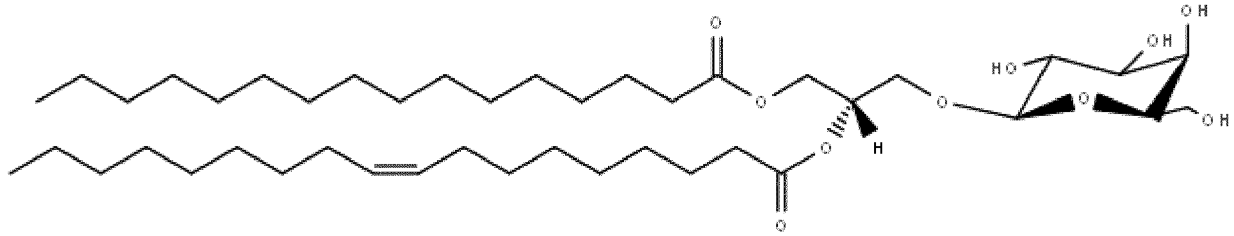

1-hexadecanoyl-2-(9Z-octadecenoyl)-3-O-β-D-galactosyl-sn-glycerol

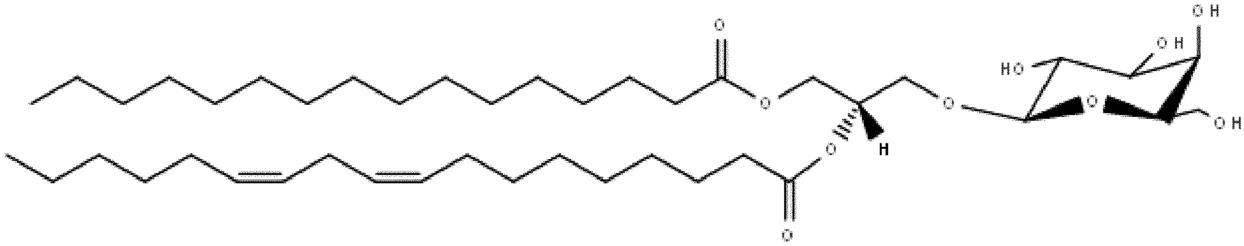

1-hexadecanoyl-2-(9Z,12Z-octadecadienoyl)-3-O-β-D-galactosyl-sn-glycerol

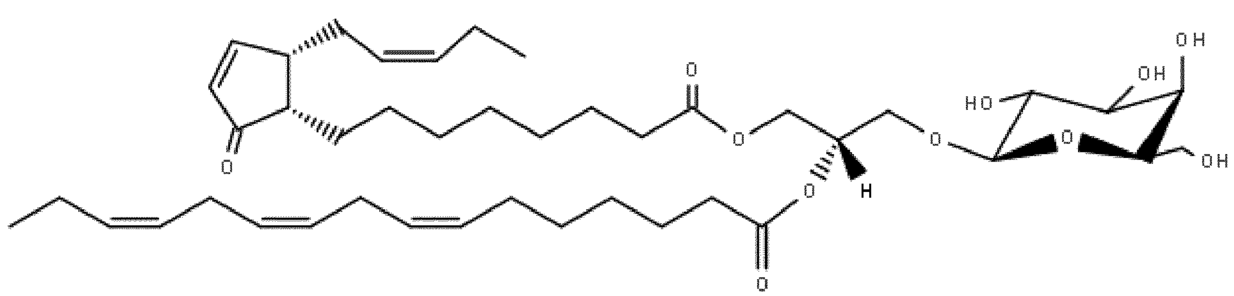

1-(9S,13S-12-oxo-11,15Z-phytodienoyl)-2-(7Z,10Z,13Z-hexadecatrienoyl)-3-O-(β-D-galactosyl)-sn-glycerol

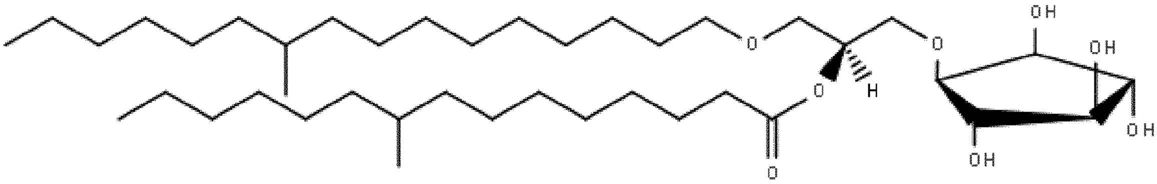

1-O-(1'S,2'S,3'R,4'R,5'S-tetrahydroxycyclopentyl)-2-(9-methylpentadecanoyl)-3-(10-methyl-hexadecanyl)-sn-glycerol

Beispiele für erfindungsgemäß aus lipoiden Phasen gewinnbare Glycoglycerolipide mit ionische Gruppen (wie z.B. Phosphat, Sulfat, Sulfonat) sind beispielsweise:

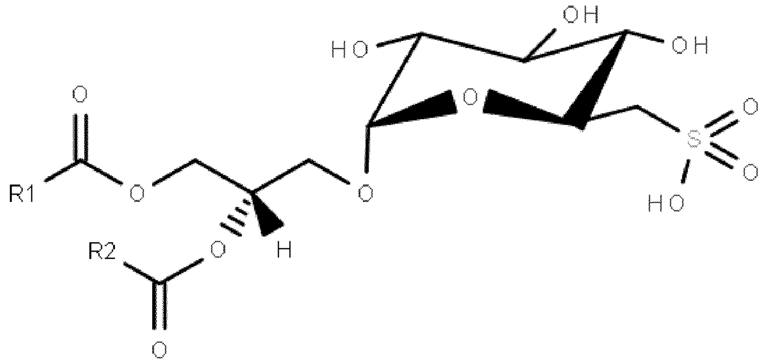

1,2-diacyl-3-(6-sulfo-α-D-quinovosyl)-sn-glycerol

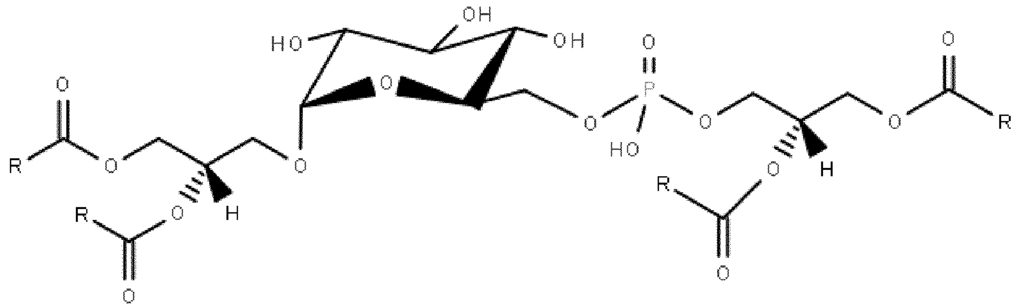

3-O-[6'-O-(1",2"-diacyl-3"-phospho-sn-glycerol)-a-D-glucopyranosyl]-1,2-diacyl-sn-glycerol

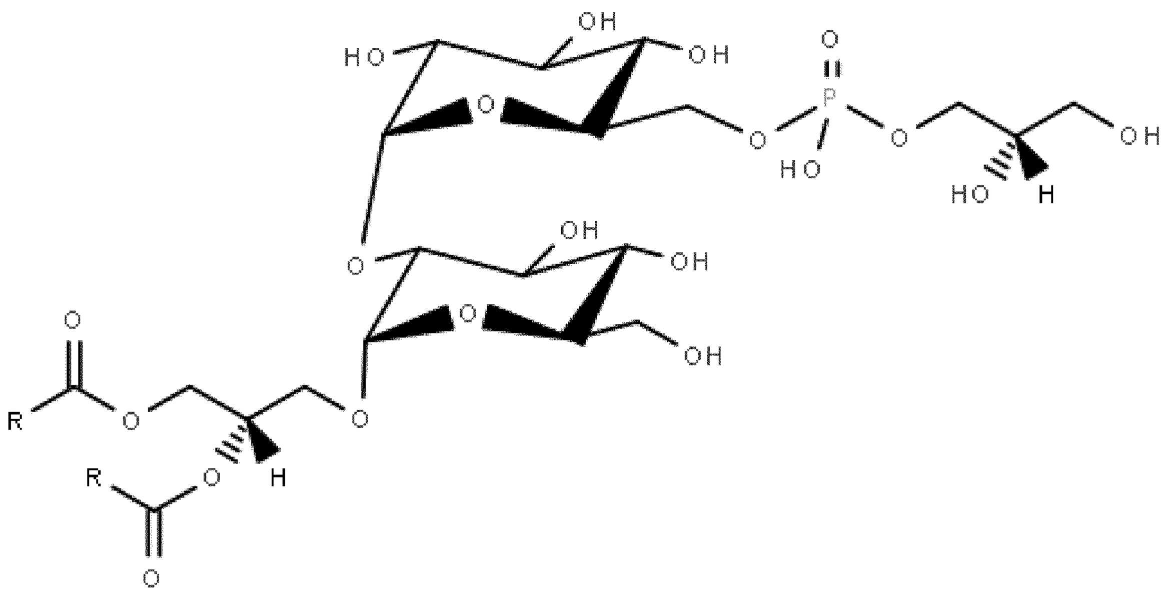

1,2-diacyl-3-[6"-(sn-glycero-1-phospho-)-α-D-kojibiosyl]-sn-glycerol

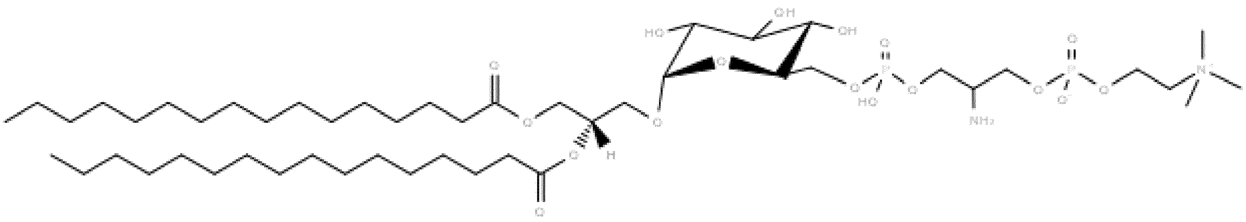

6'-O-(3"-phosphocholine-2"-amino-1"-phospho-1",3"-propanediol)-a-D-glucopyranosyl-(1'->3)-1,2-hexadecanoyl-glycerol

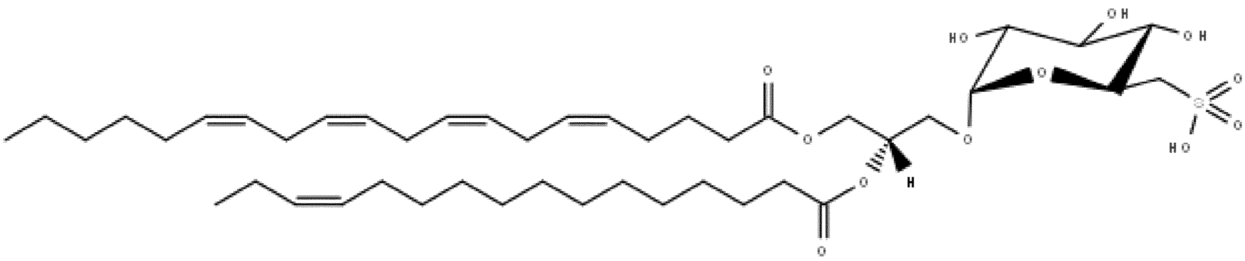

1-(5Z,8Z,11Z,14Z-eicosatetraenoyl)-2-(13Z-hexadecenoyl)-3-(6'-sulfo-α-D-quinovosyl)-sn-glycerol

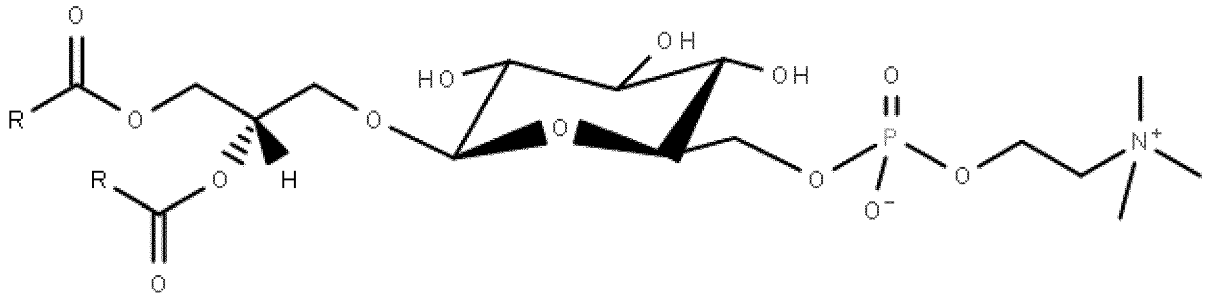

1,2-diacyl-3-(6'-O-phosphocholine-α-D-glucosyl)-sn-glycerol

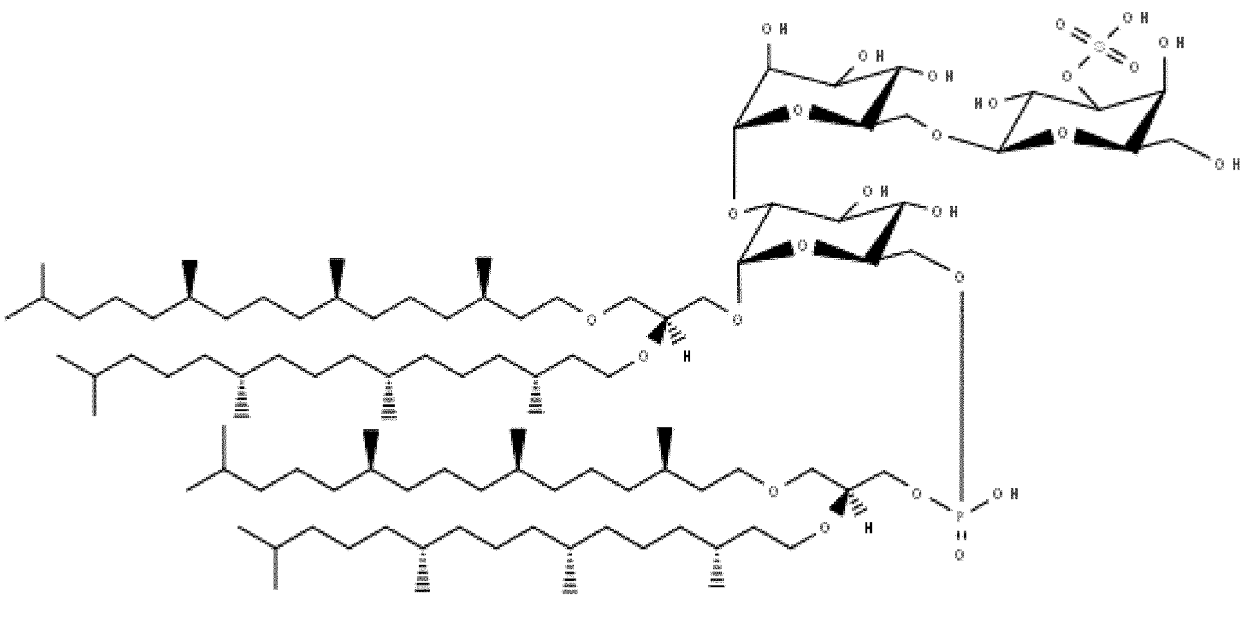

3-HSO3-Gal-α1-6-Man-α1-2-Glc-α1-1-[2,3-di-O-phytanyl-sn-glycerol]-6-[phospho-2, 3-di-O-phytanyl-sn-glycerol]

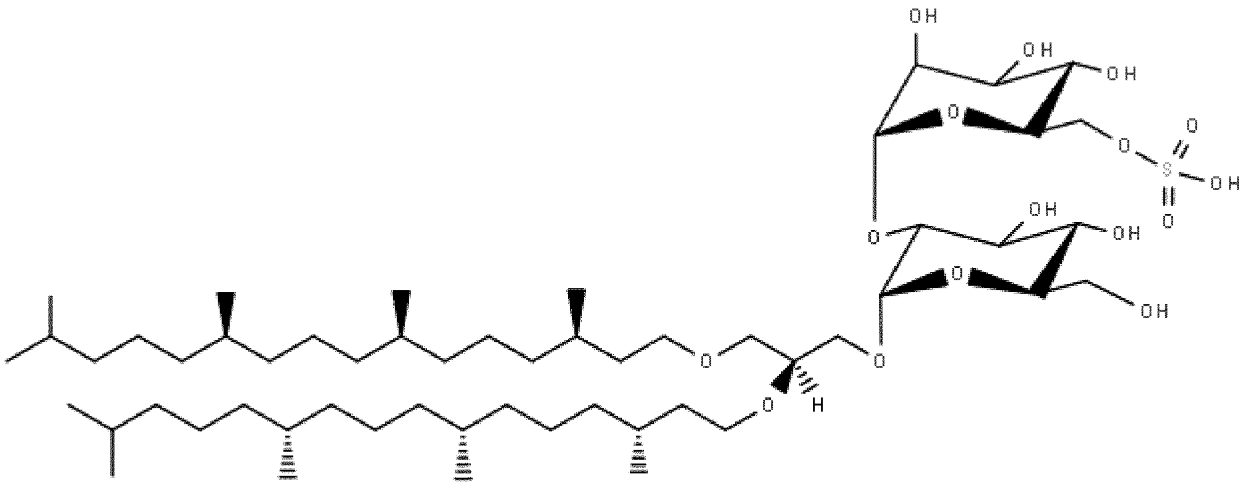

1-O-[6''-sulfo-α-D-Mannosyl-1''-2'-α-D-Glucosyl]-sn-2,3-di-O-phytanylglycerol

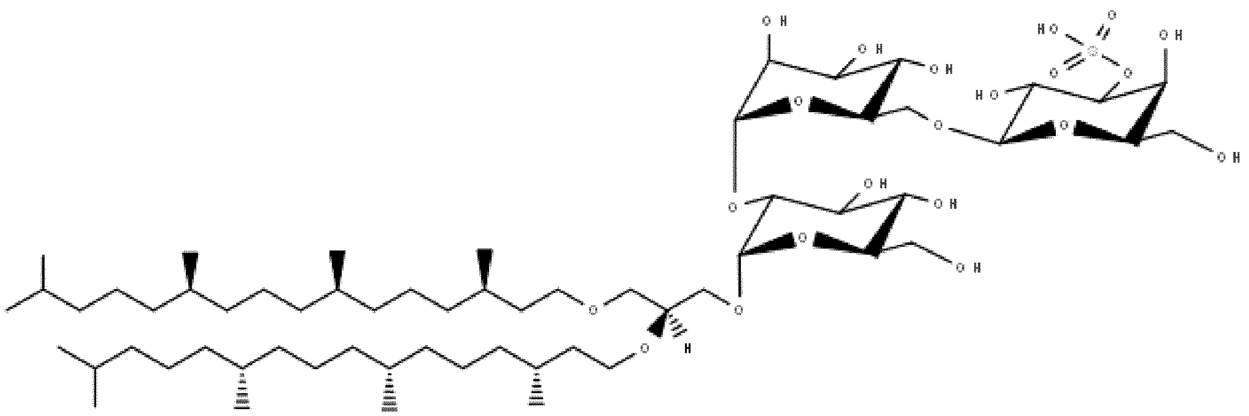

1-O-[3'''-sulfo-β-D-Galactosyl-1'''-6''-α-D-Mannosyl-1''-2'-α-D-Glucosyl]-sn-2, 3-di-O-phytanylglycerol

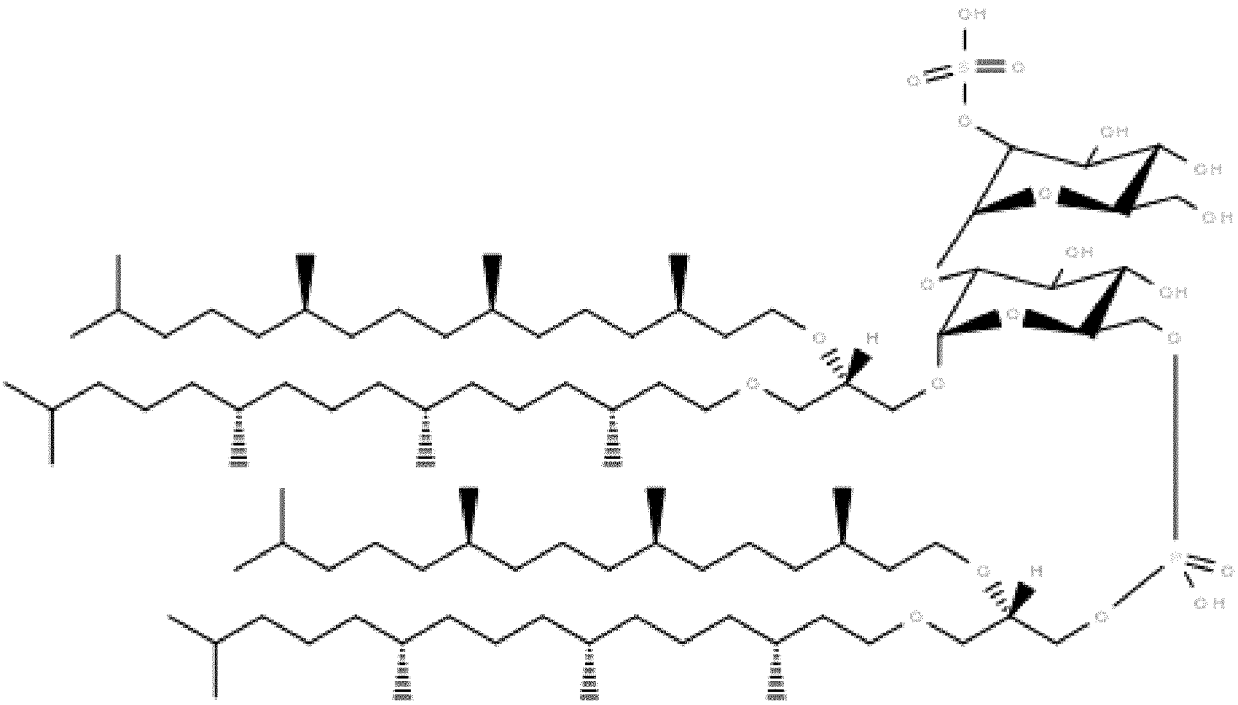

2'-HSO3-Mana1-2Glcα1-1-[2,3-di-O-phytanyl-sn-glycerol]-6-[phospho-2,3-di-O-phytanyl-sn-glycerol]

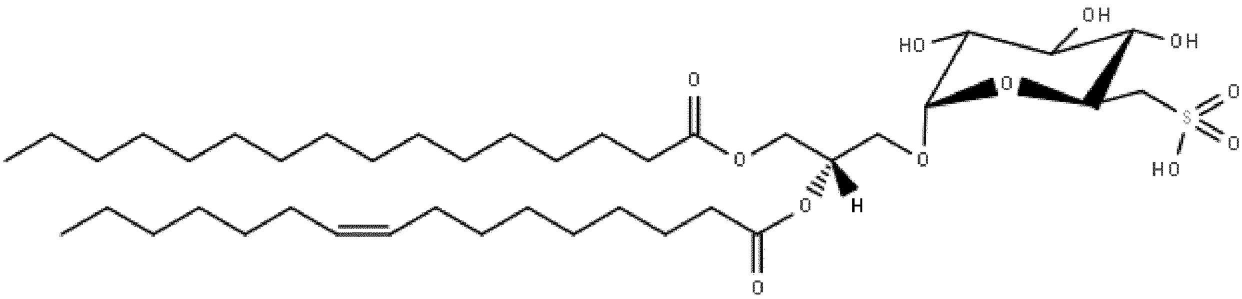

1-hexadecanoyl-2-(9Z-hexadecenoyl)-3-(6'-sulfo-α-D-quinovosyl)-sn-glycerol

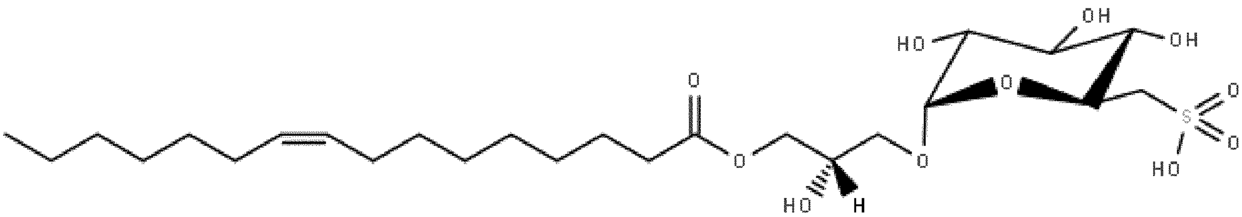

1-(9Z-hexadecenoyl)-3-(6'-sulfo-α-D-quinovosyl)-sn-glycerol

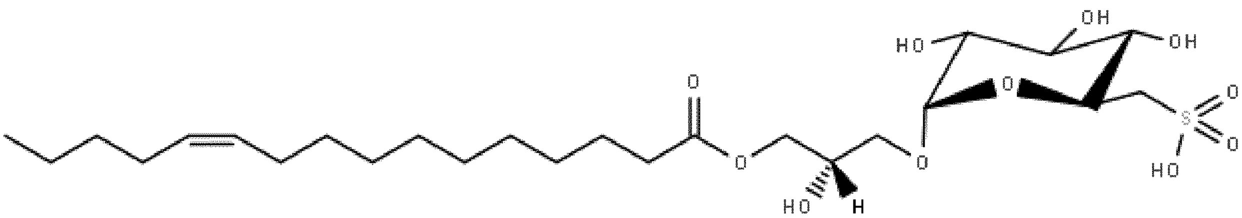

1-(11Z-hexadecenoyl)-3-(6'-sulfo-α-D-quinovosyl)-sn-glycerol

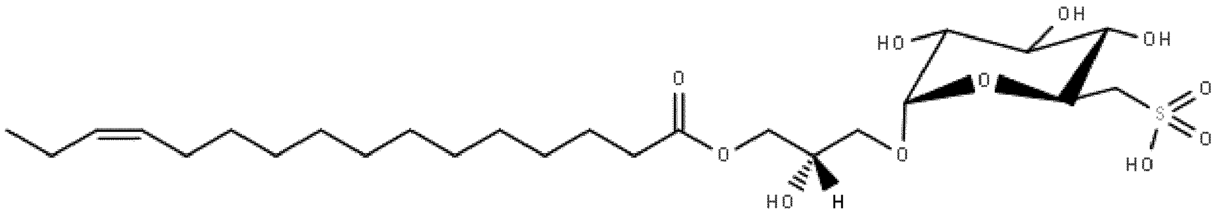

1-(13Z-hexadecenoyl)-3-(6'-sulfo-α-D-quinovosyl)-sn-glycerol

Die vorher genannten Verbindungen sind Beispiele von Glycoglycerolipiden, welche in der lipoiden Phase enthalten sind. Erfindungsgemäß werden vorzugsweise Glycoglycerolipide ohne ionische Gruppen aus der lipoiden Phase gewonnen. Der Begriff lipoide Phase bezeichnet daher das Ausgangsmaterial oder Edukt, welches gemäß der Schritte A1), B1), C1) und D1) oder gemäß der Schritte A1), A2), B1), C1) und D1) oder gemäß der Schritte A1), A2'), B1), C1) und D1) behandelt wird. Es ergibt sich eine wässrige Phase, welche auch als abgetrennte wässrige Phase bezeichnet wird, aus der die abgetrennten Glycoglycerolipide und sofern in der lipoiden Phasen vorhanden, die Glycosphingolipide, Glycolipide und/oder Glycophospholipide gemäß Schritt D2) gewonnen werden können. Diese Gewinnung einer hydrolysearmen Fraktion von Glycoglycerolipiden und Glycosphingolipiden erfolgt vorzugsweise durch Extraktion aus der nach den Schritten D1) oder D2) erhaltenen wässrigen Phase mit Lösungsmitteln wie z.B. Chloroform, Methylenchlorid und/oder Methanol. Zudem erhält man die lipoide glycoglycerolipidarme Phase, welche aufgrund der vorgenannten Schritte A1), B1), C1) und D1) optional kombiniert mit Schritt A2) oder A2') in den Gehalten an Kalium, Eisen, Calcium, Glycoglycerolipiden und sofern vorhanden im Edukt an Glycosphingolipiden, Glycolipide und/oder Glycophospholipide deutlich reduziert worden ist. Als lipoide glycoglycerolipidarme Phase bezeichnet man die lipoide Phase unmittelbar nach dem Schritt D1). Diese lipoide glycoglycerolipidarme Phase stellt bereits eine veredelte lipoide Phase dar und kann dann nach dem Schritt E1) weiter aufbereitet werden, um eine hochveredelte lipoide glycoglycerolipidarme Phase zu erhalten. Die nach Schritt E1) erhaltene hochveredelte lipoide glycoglycerolipidarme Phase wird als lipoide glycoglycerolipid- und carbonsäurearme Phase oder zur besseren Unterscheidung von der lipoiden glycoglycerolipidarmen Phase nach Schritt D1) als weiter aufbereitete lipoide glycoglycerolipidarme Phase bezeichnet.

Daher betrifft die vorliegende Offenbarung auch lipoide glycoglycerolipid- und/oder carbonsäurearme Phasen, vorzugsweise bestehend zu mindestens 90 Gew.-% aus Acylglyceriden, d.h. aus einem Gemisch von Triacylglyceriden, Diacylglyceriden und Monoacylglyceriden mit einem Gehalt an K < 5 ppm, P < 5 ppm, bevorzugt P < 4 ppm, weiter bevorzugt P < 3 ppm, weiter bevorzugt P < 2 ppm und insbesondere bevorzugt P < 1 ppm, Fe < 5 ppm, bevorzugt Fe < 4 ppm, weiter bevorzugt Fe < 3 ppm, weiter bevorzugt Fe < 2 ppm, weiter bevorzugt Fe < 1 ppm und insbesondere bevorzugt Fe < 0,1 ppm, Ca < 5 ppm und freien Fettsäuren < 0,30 Gew.-%, bevorzugt < 0,26 Gew.-%, weiter bevorzugt < 0,23 Gew.-%, weiter bevorzugt < 0,21 Gew.-%, weiter bevorzugt < 0,19 Gew.-%, weiter bevorzugt < 0,17 Gew.-%, weiter bevorzugt < 0,15 Gew.-% und insbesondere bevorzugt < 0,13 Gew.-%.

Die vorliegende Offenbarung betrifft des weiteren eine lipoide glycoglycerolipid- und/oder carbonsäurearme Phase, vorzugsweise bestehend zu mindestens 90 Gew.-% aus Acylglyceriden, d.h. aus einem Gemisch von Triacylglyceriden, Diacylglyceriden und Monoacylglyceriden mit einem Gehalt an P < 0,8 mg/kg, vorzugsweise P < 0,7 mg/kg, vorzugsweise P < 0,6 mg/kg, Fe < 0,015 mg/kg, vorzugsweise Fe < 0,013 mg/kg, vorzugsweise Fe < 0,011 mg/kg, vorzugsweise Fe < 0,009 mg/kg, Ca < 0,5 mg/kg, vorzugsweise Ca < 0,4 mg/kg, Mg < 0,12 mg/kg, vorzugsweise Mg < 0,11 mg/kg, vorzugsweise Mg < 0,10 mg/kg, Cr < 0,01 mg/kg, vorzugsweise Cr < 0,009 mg/kg, vorzugsweise Cr < 0,008 mg/kg, vorzugsweise Cr < 0,007 mg/kg, Zn < 0,01 mg/kg, vorzugsweise Zn < 0,009 mg/kg, vorzugsweise Zn < 0,008 mg/kg, vorzugsweise Zn < 0,007 mg/kg, Mn < 0,005 mg/kg, vorzugsweise Mn < 0,004 mg/kg, vorzugsweise Mn < 0,003 mg/kg und/oder FFA < 0,3 Gew.-%, vorzugsweise FFA < 0,28 Gew.-%, vorzugsweise FFA < 0,26 Gew.-%, vorzugsweise FFA < 0,24 Gew.-%, vorzugsweise FFA < 0,22 Gew.-%, vorzugsweise FFA < 0,20 Gew.-%.

Ferner betrifft die vorliegende Offenbarung lipoide glycoglycerolipidarme Phasen, bestehend zu mindestens 90 Gew.-% aus Acylglyceriden mit einem Gehalt an P < 1 ppm, Fe < 0,04 ppm, Ca < 0,4 ppm, Mg < 0,1 ppm, Pb < 0,02 ppm, Cu < 0,02 ppm, Cr < 0,02 ppm, Ni < 0,02 ppm, Cd < 0,02 ppm, Zn < 0,02 ppm und FFA < 0,3 Gew.-%.

Die offenbarten lipoiden glycoglycerolipidarmen Phasen lassen sich aus selbst qualitativ schlechten Edukten, d.h. lipoiden Phasen gewinnen. Unter qualitativ schlechten lipoiden Phasen werden solche verstanden, die bis zu 50 Gew.-% freie Fettsäuren enthalten und Mengen an K zwischen 50 und 500 ppm, P zwischen 100 und 1.500 ppm, Fe zwischen 50 und 500 ppm und Ca zwischen 50 und 500 ppm aufweisen.

Der Begriff "Fettsäuren" wird hierin synonym mit dem Begriff "freie Fettsäuren" verwendet. Der Zusatz "freie" soll verdeutlichen, dass es sich um nicht gebundene Fettsäuren handelt, weil in der lipoiden Phase der überwiegende Anteil der Bestandteile gebundene Fettsäuren enthält. Als Fettsäuren werden aliphatische Monocarbonsäuren mit mindestens 8 Kohlenstoffatomen bezeichnet.

Als "Carbonsäuren" werden Säuren mit mindestens einer Carboxylatgruppe bezeichnet. Somit umfassen Carbonsäure auch Fettsäuren.

Der Begriff **"lipoide Phase",** wie hierin verwendet, umfasst Stoffgemische biologischer Herkunft, die also aus Pflanzen, Algen, Tieren und/oder Mikroorganismen gewonnen werden können und die einen Wassergehalt von <10% und einen Gehalt an lipophilen Substanzen, umfassend Monoacylglyceride, Diacylglyceride und/oder Triacylglyceride von insgesamt >70 Gew.-% oder >75 Gew.-% oder >80 Gew.-% oder >85 Gew.-% oder >90 Gew.-% oder >95 Gew.-%, aufweisen. So kann es sich bei den lipoiden Phasen beispielsweise um Extrakte ölhaltiger Pflanzen und Mikroorganismen handeln wie Kerne von Raps, Soja, Leindotter, Jatropha, Palmen, aber auch von Algen und Mikroalgen sowie um tierische Fette und Öle.

Die lipoiden Phasen haben bevorzugt einen Wassergehalt von <10% und einen Anteil an Alkanen und/oder cyclischen Aromaten und/oder Mono-/Di-/Triglyceriden (Acylglyceride) von >75%. Dabei ist es unerheblich, ob es sich bei der lipoiden Phase um eine Suspension, Emulsion oder kolloidale Flüssigkeit handelt.

Sofern es sich bei den lipoiden Phasen um Extrakte bzw. Extraktionsphasen lipoider Stoffe aus einer zuvor erfolgten Abtrennung oder Extraktion handelt, kann die lipoide Phase auch zu einem Anteil von > 50% aus organischen Lösungsmitteln oder Kohlenwasserstoffverbindungen bestehen.

Der Begriff "lipoide Phase, welche Glycoglycerolipide und Acylglyceride enthält" sagt nur aus, dass die einsetzbaren lipoiden Phasen neben etlichen anderen Stoffen auch Glycoglycerolipide und Acylglyceride enthalten, jedoch keinesfalls nur aus Glycoglycerolipiden und Acylglyceriden bestehen. Gleiches gilt für den Begriff "lipoide Phase, welche Glycoglycerolipide und Glycosphingolipide und Acylglyceride enthält". Diese Bezeichnung sagt auch nur aus, dass die erfindungsgemäß einsetzbaren lipoiden Phasen Glycoglycerolipide und Glycosphingolipide und Acylglyceride enthalten, jedoch keinesfalls nur aus Glycoglycerolipiden und Glycosphingolipiden und Acylglyceriden bestehen. Einige beispielhafte Substanzen oder Stoffklassen, welche in den einsetzbaren lipoiden Phasen noch enthalten sein können, sind andere Glycolipide als Glycoglycerolipide und Glycosphingolipide, Glycophospholipide, Phospholipide, freie Fettsäuren, Fettsäureester, Sterylglycoside und viele andere Stoffe.

Den Hauptbestandteil der lipoiden Phase bilden evtl. abgesehen von organischen Lösungsmitteln die Acylglyceride. Die Acylglyceride werden durch die erfindungsgemäßen Verfahren jedoch nicht oder nur in sehr geringem Maße (weniger als 0,1% aller vorhandenen Acylglyceride, vorzugsweise weniger als 0,05% aller vorhandenen Acylglyceride, und insbesondere bevorzugt weniger als 0,01% aller vorhandenen Acylglyceride) in die wässrige Phase überführt und somit auch nicht oder nur in sehr geringem Maße zusammen mit den Glycoglycerolipiden und Glycosphingolipiden aus der lipoiden Phase abgetrennt.

Als natürlicher Bestandteil praktisch sämtlicher Zellen in pflanzlichen und tierischen Lebewesen, kommen sowohl Phospholipide, Glycolipide, Glycoglycerolipide als auch Glycosphingolipide unvermeidlich ebenfalls in aus diesen Lebewesen oder Pflanzen gewonnenen lipoiden Phasen (wie z.B. Pflanzenöle oder tierische Öle) vor. Zu welchem Anteil dies tatsächlich der Fall ist hängt nicht nur von dem Gewebe, aus dem extrahiert wurde, sondern auch von dem Extraktionsverfahren ab. In Tabelle 1 sind Zusammensetzungen lipoider Phasen zusammengefasst, die aus diversen Nutzpflanzen gewonnen wurden. Hier lässt sich bereits erkennen, dass in der Regel die Neutrallipide den Hauptanteil der lipoiden Phasen ausmachen, jedoch der Anteil an Phospholipiden und Glycolipiden / Glycoglycerolipiden / Glycosphingolipiden äußert variabel ist. So reicht der Anteil an Glycolipiden, Glycoglycerolipiden und Glycosphingolipiden von 0,2% in Kokosöl, über ca. 2% in Borretschöl und 6,3 - 7% in Reiskleienöl bis hin zu 19,4 % in Öl aus Avokadokernen.

Tab. 1: Gehalt an Lipiden ohne ionische Gruppen (NL) Acylglyceriden (AG), Phospholipiden (PL) und Glycolipiden zusammen mit Glycoglycerolipiden und Glycosphingolipiden (GL) in den Samen (S) bzw. den daraus gewonnenen Ölen ausgewählter Pflanzen. Der Gehalt an AG, PL sowie GL ist in Prozent des gesamten Öls angegeben. Bei Samen ist z.T. zusätzlich angegeben, wie hoch der prozentuale Ölanteil (total) im Vergleich zur Samenmasse ist.

| **Quelle** | **Öl** | **S** | **total** | **NL** | **PL** | **GL** |
|---|---|---|---|---|---|---|
| Soja: *Glycine soya* | X | | | 88 | 10 | **2** |
| Palme: *Elaieis guineensis* | X | | | 96 | 2,4 | **1,4** |
| Reiskleie:<br>*Oryza sativa* | | X | 21,9-<br>23,0 | 88,1-<br>89,2 | 4,5-<br>4,9 | **6,3-<br>7,0** |
| Mais<br>*Zea mays* | X | | | 96,8-<br>97,5 | 0,8-<br>0,95 | **1,5-<br>1,66** |
| Raps<br>*Brassica napus* | | X | | 95,8<br>95,5 | 3,2<br>3,6 | **0,9<br>0,9** |
| Raps - Variante "Golden": *Brassica* | | X | 34,8 | 98,8 | **3,0** | |
| Raps - Variante "Zero Eruca": *Brassica* | | X | 35,9 | 98,1 | **1,8** | |

(fortgesetzt)

| Quelle | Öl | S | total | NL | PL | GL |
|---|---|---|---|---|---|---|
| Sonnenblumenkerne: *Helianthus annuus* | | X | | | **< 4** | |
| Jatropha: *Jatropha curcus* | | X | 32 | 97,6 | 1,45 | **0,95** |
| Kokosnuss<br>Cocos *nucifera* | X | | | 93,6-<br>98,2 | 0,03-<br>0,4 | **0,2-<br>0,35** |
| Kakaobutter | X | | | 98,75 | 0,037 | **0,89** |
| Färberdistel: *Carthamus tinctorius* | X | | | 94 | 1,2 | **4,5** |
| Borretsch: *Borago officinalis* | | X | 34,0 | 95,7 | 2,3 | **2,0** |
| Krambe: *Crambe abyssinica* | | X | 32,2 | 98,5 | **1** | **,1** |
| Krambe: *Crambe abyssinica* | X | | 75 | 88,6 | 11 | - |
| Schwarzkümmel: *Nigella sativa* | X | | | 97,2 | 0,3 | **2,18** |
| Korianderöl: *Coriandrum sativum* | X | | | 96,0 | 0,85 | **2,39** |
| Nigersaat: *Guizotia abyssinca* | X | | | 97,0 | 0,28 | **1,90** |
| Langkapselige Jute: *Corchorus olitorius* | X | | | 93,2 | 1,9 | **3,7** |
| Hibiskus: *Hibiscus sabdariffa* | X | | | 94,1 | 2,1 | **2,6** |
| Avocado: *Persea americana* | | X | 10,8 | 60,2 | 20,4 | **19,4** |
| Weißer Sternapfel: *Chrysophyllum albidum* | | X | 7,7 | 54,6 | 23,4 | **22** |
| Bittermelone:<br>*Mormodica charantia* | X | | | 86,8-<br>91,1 | 3,22-<br>4,62 | **4,37-<br>7,43** |
| Sesam:<br>*Sesamum indicum* | X | | 42,5-<br>46,2 | 91,7-<br>93,3 | 0,08-<br>0,1 | **5,6-<br>5,8** |
| Mexikanischer Stachelmohn:<br>*Argemone mexicana* | X | | 35 | 92,1-<br>92,3 | 1,5-<br>1,7 | **5,5-<br>5,8** |
| Shiso:<br>*Perilla frutescens* | | X | 38,6-<br>47,8 | 91,2-<br>93,9 | 2,0-<br>3,0 | **3,5-<br>5,8** |
| Mango:<br>*Mangifera indica* | | X | 7,1-<br>10 | 58,5-<br>96,8 | 0,11-<br>0,8 | **0,6-<br>1,2** |
| Blaue Lupine: *Lupinus angustifolius* | | X | 8,6 | 76,3 | 14,9 | **3,5** |
| Paprika:<br>*Capsicum annum* | | X | 37,5<br>26,4 | 82<br>82 | 11,9<br>13,2 | **6,1<br>4,8** |
| Guinea-Pfeffer:<br>*Atremomum melequeta* | | X | | 67,72<br>50,0 | 13,68<br>10,12 | **3,75<br>2,38** |

Auch wenn die oben erwähnten Fettsäureglycoside, umfassend Trehalose-Lipide, Mannosylerythritol-Lipide, Cellobiose-Lipide, Rhamnolipide und Sophorolipide, nicht von Tieren oder Pflanzen sondern von Bakterien, Pilzen und Hefen synthetisiert werden und somit auch nicht als natürlicher Bestandteil pflanzlicher oder tierischer Öle vorkommen, so werden zu der Bildung dieser Emulgatoren Kohlenwasserstoffquellen benötigt. Ein wesentlicher Nachteil von Emulgatoren natürlicher Herkunft (Bio-Emulgatoren) gegenüber industriell synthetisierten Emulgatoren sind die bislang noch höheren Herstellungskosten. In der Biotechnologie werden daher zur Gewinnung der Bio-Emulgatoren im großen Maßstab Wachstumsmedien verwendet, die als kostengünstige Kohlenwasserstoffquelle pflanzliche Öle (z.B. Sonnenblumenöl, Rapsöl, Palmöl, Jatropha-Öl, Rizinusöl, etc.) aber auch bei deren Gewinnung anfallende Abfallprodukte (z.B. Kokosnusskuchen, Sojakuchen, Erdnusskuchen, Seifenstock oder "Soap-Stock", Rapsschrot, etc.) verwendet. Ebenfalls können als kostengünstige Kohlenwasserstoffquelle Abfallprodukte aus der Produktion tierischer Lebensmittel (z.B. Talg, Fischöle, Molke) verwendet werden. So entstehen also lipoide Phasen die neben pflanzlichen Ölen oder tierischen

Ölen auch durch Mikroorganismen gebildete Bio-Emulgatoren aus der Gruppe der Glycolipide enthalten.

Da ca. 60% der Herstellungskosten bei Bio-Emulgatoren durch die nach der Synthese durch Mikroorganismen anfallenden Kosten durch die Aufreinigung entstehen, existiert ein Bedarf an effizienten und kostengünstigen Verfahren zur Aufreinigung der zu den Glycolipiden gehörenden Trehalose-Lipide, Mannosylerythritol-Lipide, Cellobiose-Lipide, Rhamnolipide und Sophorolipide aus lipoiden Phasen.

Zu den lipoiden Phasen im Sinne der hierin verwendeten Definition zählen u.a. Acai-Öl, Acrocomia-Öl, Mandelöl, Babassuöl, Johannisbeersamenöl, Borretschsamenöl, Rapsöl, Cashew-Öl, Rizinusöl, Kokosöl, Korianderöl, Maisöl, Baumwollsamenöl, Kramben-Öl, Leinsamenöl, Traubenkernöl, Haselnussöl, andere Nussöle, Hanfsamenöl, Jatropha-Öl, Jojoba-Öl, Macadamianussöl, Mangokernöl, Wiesenschaumkraut-Öl, Senföl, Klauenöl, Olivenöl, Palmöl, Palmkernöl, Palmoleinöl, Erdnussöl, Pecan-Öl, Pinienkernöl, Pistazienöl, Mohnöl, Reiskeimöl, Distelöl, Kamelien-Öl, Sesamöl, Sheabutter-Öl, Sojaöl, Sonnenblumenöl, Tallöl, Tsubaki-Öl, Walnussöl, Sorten von "natürlichen" Ölen mit veränderten Fettsäurezusammensetzungen über genetisch veränderte Organismen (GVO) oder traditionellen Züchtungen, Neochloris oleoabundans Öl, Scenedesmus dimorphus Öl, Euglena gracilis Öl, Phaeodactylum tricornutum Öl, Pleurochrysis carterae Öl, Prymnesium parvum Öl, Tetraselmis chui Öl, Tetraselmis suecica Öl, Isochrysis galbana Öl, Nannochloropsis salina Öl, Botryococcus braunii Öl, Dunaliella tertiolecta Öl, Nannochloris Öl, Spirulina Öl, Chlorophyceae Öl, Bacilliarophyta Öl, eine Mischung aus den vorhergehenden Ölen sowie tierische Öle (besonders Seetieröle) und Biodiesel.

Die zu Beginn des erfindungsgemäßen Verfahrens zum Abtrennen von Glycoglycerolipiden und sofern in der lipoiden Phase vorhanden, von Glycosphingolipiden, Glycolipiden und/oder Glycophospholipiden bereitgestellte lipoide Phase kann auch als lipoide Rohphase bezeichnet werden und weist noch einen vergleichsweise hohen Gehalt an Begleitstoffen wie Metallionen, ionische Lipide, Fettsäuren und unter Umständen anderer Substanzen (z.B. Pflanzenschutzmittelrückstände, ätherische Öle) auf. Im Rahmen des/der erfindungsgemäßen wässrigen Extraktionsschritte(s) verändert sich natürlich die Zusammensetzung der lipoiden Rohphase. Folglich weisen die lipoiden Phasen, die nach jedem wässrigen Extraktionsschritt verbleiben, eine andere Zusammensetzung als die entsprechende Ausgangsphase auf, da Substanzen, die während der wässrigen Extraktion aus der lipoiden Phase in die wässrigen Phase übergehen zusammen mit dieser wässrigen Phase abgetrennt werden. Durch das erfindungsgemäße Verfahren werden neben den Glycoglycerolipiden eventuell zusammen mit Glycosphingolipiden, Glycolipiden und/oder Glycophospholipiden (sofern vorhanden) vor allem Metallionen, ionische Lipide und/oder freie Fettsäuren durch wässrige Extraktion aus der lipoiden Phase entfernt. Diese Entfernung muss sich nicht zwingend auf den Schritt D1) beziehen, sondern erfolgt vorzugsweise auch im Schritt A2) und A2') und vor allem auch im Schritt E1).

Die erfindungsgemäß gewonnene wässrige Phase enthaltend die Glycoglycerolipide oder die Glycoglycerolipide und Glycosphingolipide kann weiter separiert werden. Offenbart sind insbesondere und vorzugsweise erhaltene wässrige Phasen erhaltend Glycoglycerolipide und eventuell Glycosphingolipide solche, die einen Feststoffanteil von vorzugsweise >40 Gew.-% haben.

Es konnte gezeigt werden, dass eine unmittelbare Extrakten der lipophilen Feststoffbestandteile aus dem gewonnenen wässrigen Extraktionsmedium mittels organischer Lösungsmittel möglich ist. So kann der größte Teil der in der Wasserphase gelösten Glycoglycerolipide und Glycosphingolipide z. B. durch Chloroform unmittelbar abgetrennt werden. Das Abtrennergebnis kann durch die Hinzugabe eines geringen Anteils an Methanol weiter verbessert werden unter Gewinnung einer transparenten organischen und wässrigen Phase. Zu einem prinzipiell gleichen Ergebnis kommt man, wenn zunächst die Wasserphase unter milden Bedingungen entfernt wird und dann der Feststoff in $CHCl_3$, $CHCl_3$/MeOH oder Aceton gelöst wird. Die Verbindungen lassen sich mit etablierten Verfahren wie der Dünnschichtchromatographie in weitere Fraktionen auftrennen und gewinnen.

Der Begriff **"Fettsäuren",** wie hierin verwendet, bezieht sich auf freie Fettsäuren (auch FFAs angekürzt), also Fettsäuren, welche frei vorliegen und nicht glyceridisch (d.h. an Glycerin) oder glycosidisch (d.h. an Zuckerreste) gebunden sind.

Der Begriff "Fettsäuren" umfasst vorzugsweise die folgenden Verbindungen: Hexansäure, Octansäure, Decansäure, Dodecansäure, Tetradecansäure, Hexadecansäure, Heptadecansäure, Octadecansäure, Eicosansäure, Docosansäure, Tetracosansäure, cis-9-Tetradecensäure, cis-9-Hexadecensäure, cis-6-Octadecensäure, cis-9-Octadecensäure, cis-11-Octadecensäure, cis-9-Eicosensäure, cis-11-Eicosensäure, cis-13-Docosensäure, cis-15-Tetracosensäure, t9-Octadecensäure, t11-Octadecensäure, t3-Hexadecensäure, 9,12-Octadecadiensäure, 6,9,12-Octadecatriensäure, 8,11,14-Eicosatriensäure, 5,8,11,14-Eicosatetraensäure, 7,10,13,16-Docosatetraensäure, 4,7,10,13,16-Docosapentaensäure, 9,12,15-Octadecatriensäure, 6,9,12,15-Octadecatetraensäure, 8,11,14,17-Eicosatetraensäure, 5,8,11,14,17-Eicosapentaensäure, 7,10,13,16,19-Docosapentaensäure, 4,7,10,13,16,19-Docosahexaensäure, 5,8,11-Eicosatriensäure, 9c11t13t-Eleostearinsäure, 8t10t12c-Calendulasäure, 9c11t13c-Catalpinsäure, 4,7,9,11,13,16,19-Docosaheptadecansäure, Taxoleinsäure, Pinolensäure, Sciadonsäure, 6-Octadecinsäure, t11-Octadecen-9-insäure, 9-Octadecinsäure, 6-Octadecen-9-insäure, t10-Heptadecen-8-insäure, 9-Octadecen-12-insäure, t7,t11-Octadecadien-9-insäure, t8,t10-Octadecadien-12-insäure, 5,8,11,14-Eicosatetrainsäure, Retinsäure, Isopalmitinsäure, Pristansäure, Phytansäure, 11,12-Methylen-Octadecansäure, 9,10-Methylen-Hexadecansäure, Coronarinsäure, (R,S)-Liponsäure, (S)-Liponsäure, (R)-Liponsäure, 6,8(methylsulfanyl)-Oktansäure, 4,6-bis(methylsulfanyl)-Hexansäure, 2,4-bis(methylsulfanyl)-Butansäure, 1,2-Dithiolan-Carboxylsäure, (R,S)-6,8-Dithian-Oktansäure, (S)-6,8-Dithian-Oktansäure, Taririnsäure, Santalbinsäure, Stearolsäure, 6,9-Octadeceninsäure, Pyrulinsäure, Crepeninsäure, Heisterinsäure, t8,t10-Octadecadien-12-insäure, ETYA, Cerebronsäure, Hydroxynervonsäure, Ricinoleinsäure, Lesquerolinsäure, Brassylinsäure, und Thapsinsäure.

Der Begriff **"Acylglyceride",** wie hierin verwendet, beschreibt Verbindungen, bei denen zumindest eine Hydroxy-Gruppe eines Glycerin-Reste mit einer Fettsäure verestert ist. Zu den Acylglyceriden zählen die **Monoacylglyceride,** bei denen eine Hydroxy-Gruppe des Glycerins mit einer Fettsäure verestert ist, die **Diacylglyceride,** bei denen zwei Hydroxy-Gruppen des Glycerins mit je einer Fettsäure verestert sind, sowie die **Triacylglyceride,** bei denen alle drei Hydroxy-Gruppen des Glycerins mit je einer Fettsäure verestert sind.

Unter dem Begriff **"Glycolipid",** wie hierin verwendet, sind Verbindungen zusammengefasst, in denen ein oder mehrere Monosaccharid-Rest(e) über eine glycosidische Bindung mit einem hydrophoben Acylrest verbunden ist.

**"Glycoglycerolipide"** sind Verbindungen, worin ein Saccharidrest an eine primäre Hydroxygruppe von Glycerin gebunden ist und die beiden anderen Hydroxygruppen des Glycerins mit lipophilen Acylresten und insbesondere Fettsäureresten verestert sind.

**"Glycosphingolipide"** sind Verbindungen, worin ein Saccharidrest glycosidisch an vorzugsweise ein Sphingolipid gebunden ist.

**"Glycophosphatidylinositole"** sind Verbindungen, bei denen Saccharide glycosidisch an die Inositol-Gruppe von Phosphatidylinositolen gebunden sind.

**"Phospholipide",** wie hierin verwendet, sind amphiphile Lipide, die eine Phosphatgruppe enthalten und entweder zu den Phosphoglyceriden oder zu den Phosphosphingolipiden gehören. **"Phosphoglyceride"** (auch als Glycerophospholipide oder Phosphoglycerolipide bezeichnet) bestehen aus einem Diacylglycerid, dessen verbleibende endständige Hydroxy-Gruppe an einen Phosphatrest gebunden ist, welcher entweder nicht weiter modifiziert ist (Phosphatidsäure) oder mit einem Alkohol verestert ist. Die häufigsten Vertreter der letzteren Gruppe sind Phosphatidylcholine (auch als Lecithine als bezeichnet), Phosphatidylethanolamine und Phosphatidylserine. Die **"Phosphosphingolipide",** umfassen Lipide mit einem Sphingosin-Grundgerüst, an dessen C2-Aminogruppe über eine Amidbindung eine Fettsäure gebunden ist und dessen C1-Hydroxygruppe mit einer Phosphatgruppe über eine Phosphoesterbindung verknüpft ist, wobei (wie bei den Phospholipiden) diese Phosphatgruppe wiederum mit einem Alkohol verestert sein kann. Die Begriffe "Phospholipide" und "Phosphatide" können gleichbedeutend verwendet werden.

**neutrale Glycoglycerolipide**

Zu den neutralen Glycoglycerolipiden zählen Glycosyldiacylglycerine, Glycosylacylalkylglycerine, Glycosyldialkylglycerine, sowie Glycoglycerolipide, deren Saccharidrest an Position 6, 2 und/oder 3 acyliert ist. Zusätzlich gibt es an der Position 1 oder 2 des Glycerinrestes deacetylierte Glycoglycerolipide, so genannte Glycosylmonoacylglycerine. In Pflanzen ist Galactose der vorherrschende Zuckerrest in Glycoglycerolipiden, wobei prominente Beispiele Monogalactosyldiacylglycerine (MGDG, 1,2-Di-O-acyl-3-O-β-D-galactopyranosyl-sn-glycerine) und Digalactosyldiacylglycerine (DGDG, 1,2-Di-O-acyl-3-O-(6'-O-a-D-galactopyranosyl-β-D-galactopyranosyl)-sn-glycerine)) sowie Trigalactosyldiacylglycerine und Tetragalactosyldiacylglycerine sind. In den meisten Angiospermen ist sowohl bei MGDG als auch bei DGDG Linolensäure (18:3n-3) der fast ausschließlich vorkommende Fettsäurerest an den Positionen 1 und 2 des Glycerin-Restes. In einigen Angiospermen (Solanaceae, Brassicaceae und Chenopodiaceae) sowie niederen Pflanzen kommt jedoch in der Position 2 des Glycerin-Restes häufig eine spezielle Triensäure (16:3 n-3) vor. MGDG und dessen lyso-Formen sind für das Backverhalten von Weizenmehlprodukten von großer Bedeutung und damit industriell von hohem Interesse. In Haferkörnern konnten so genannte DGDG-Monoestolide nachgewiesen werden, die als Fettsäuren am Glycerin eine Linolsäure und eine an Position 15 hydroxylierte Linolsäure enthalten, wobei bei letzterer die Hydroxy-Gruppe an Position 15 mit einer weiteren Linolsäure verestert ist. Analog hierzu gibt es DGDG-Diestolide, DGDG-Triestolide und sogar DGDG-Tetraestolide, bei denen die erste an Position 15 hydroxylierte Linolsäure am Glycerin-Rest mit ein bis drei weiteren an Position 15 hydroxylierten Linolsäuren verestert ist bevor die abschließenden Veresterung mit Linolsäure erfolgt. Zudem existieren bei Pflanzen Glycoglycerolipide mit einem Galactose-Rest, der an Position 6 acyliert ist. Obgleich, wie bereits erwähnt Galactose der vorherrschende Zuckerrest in pflanzlichen Glycoglycerolipiden ist, gibt es auch Glucose-basierte pflanzliche Glycoglycerolipide, wie z.B. das 1,2-Di-O-acyl-3-O-ß-D-glucopyranosyl-sn-glycerin aus Reiskleie. Auch bei Tieren konnten Monogalactosyldiacylglycerine, Digalactosyldiacylglycerine, Monogalactosylacylalkylglycerine, Digalactosylacylalkylglycerine sowie Glucosylacylalkylglycerine nachgewiesen werden.

| **Bakterien** | |
|---|---|
| 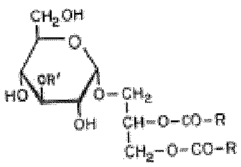 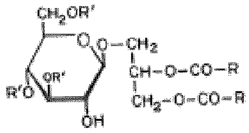 (1) R' = H (1') R' = acyl (2) R' = H (2') R' = acyl | 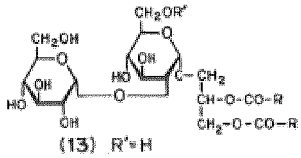 (13) R' = H (13') R' = acyl 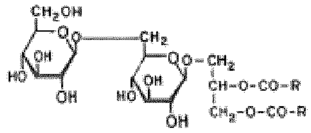 (14) |
| Monoglucosyldiacylglycerine | Diglucosyldiacylglycerine |
| 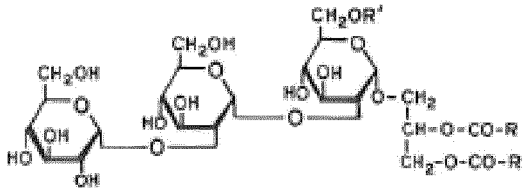 (22) R' = H (22') R' = acyl 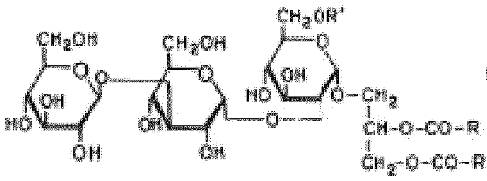 (23) 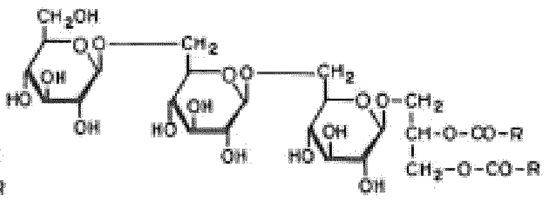 (24) | |
| Triglucosyldiacylglycerine | |
| 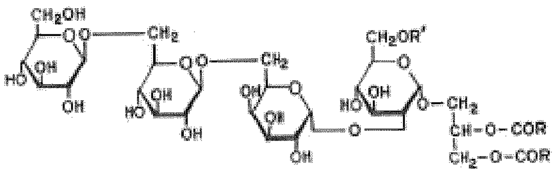 (28) R' = H (28') R' = acyl | 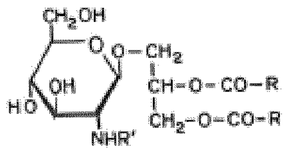 (10) R' = H (10') R' = acyl |
| Tetraglucosyldiacylglycerine | Diacylglycerine + Glucosamin |
| 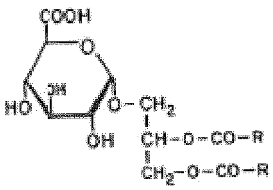 | |
| Diacylglycerine + Glucuronsäure | |

| **Pflanzen** | |
|---|---|
| 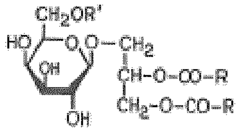 (1) R' = H (1') R' = acyl | B 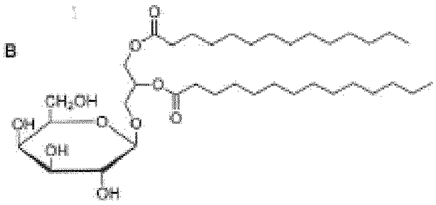 |
| Monogalactosyldiacylglycerine (MGDG) | MGDG |
| D 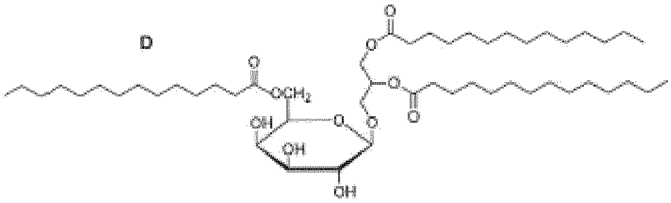 | C 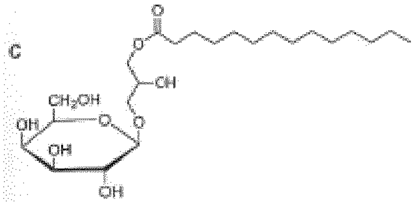 |
| 6'-Acyl-MGDG | MGDG (deacetyliert an sn-2) |
| 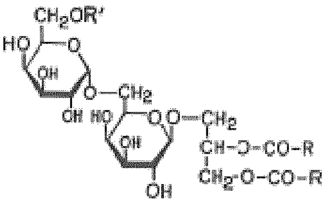 (2) R'=H ; (2') R'=acyl | 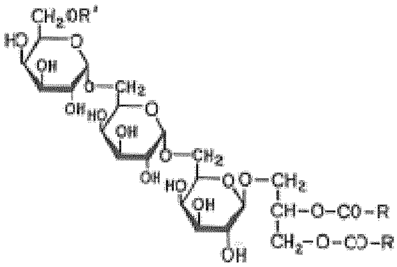 (3) R'=H ; (3') R'= Galpα |
| Digalactosyldiacylglycerine (DGDG) | Tri- & Tetragalactosyldiacylglycerine |
| 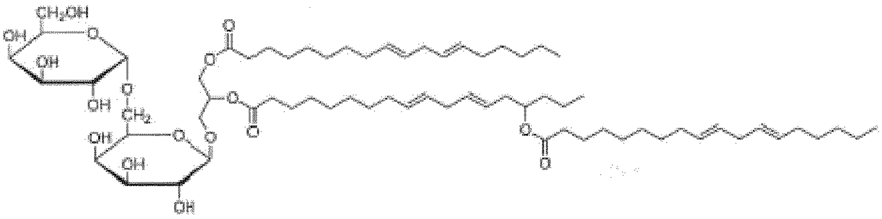 | |
| DGDG-Monoestolid | |

| **Tier** | |
|---|---|
| 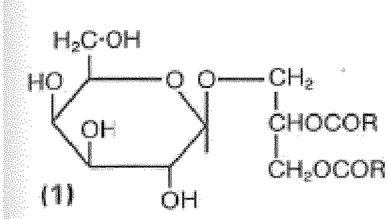 (1) | 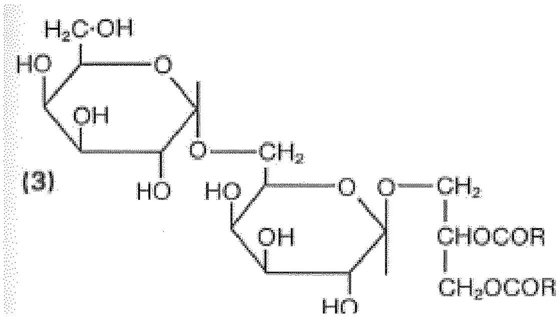 (3) |
| Monogalactosyldiacylglycerine | Digalactosyldiacylglycerine |

(fortgesetzt)

| Tier | |
|---|---|
| 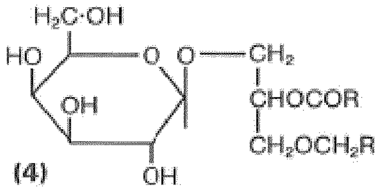 (4) | 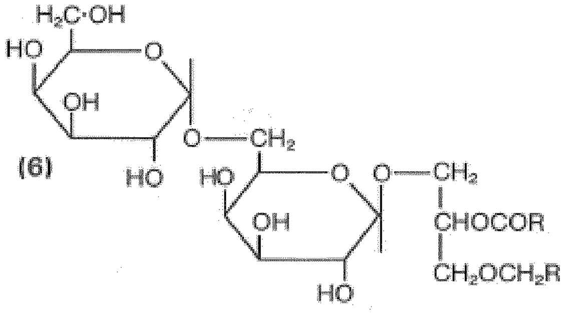 (6) |
| Monogalactosylacylakylglycerine | Digalactosylacylakylglycerine |

**saure Glycoglycerolipide**

Neben den neutralen Glycoglycerolipiden gibt es auch saure Glycoglycerolipide, deren Azidität dadurch zustande kommt, dass deren Saccharid-Rest mit Schwefelsäure oder Sulfonsäure verestert ist. Man spricht auch von Sulfoglycoglycerolipiden. Beispiele hierfür sind

| | |
|---|---|
| 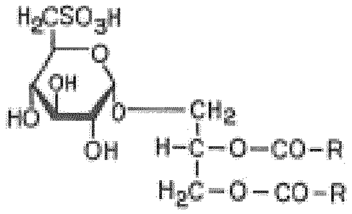 | 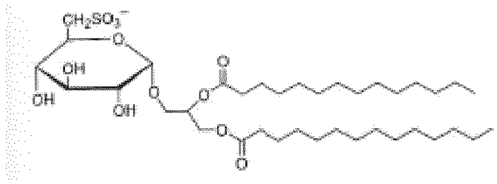 |
| Sulfoquinovosyldiacylglycerin | Sulfoquinovosyldiacylglycerin |
| 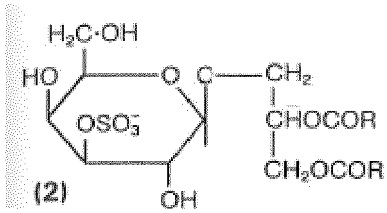 (2) | 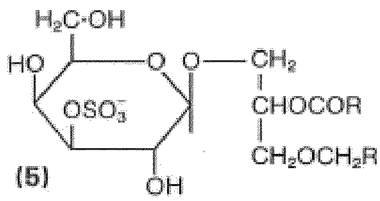 (5) |
| 1,2-di-O-acyl-3-O-(3'-deoxy-3'-sulfo-α-D-galactopyranosyl)-sn-glycerine | 1-O-acyl-2-O-alkyl-3-O-(3'-deoxy-3'-sulfo-α-D-galactopyranosyl)-sn-glycerine |

**Glycosphingolipide**

Glycosphingolipide sind Glycolipide, die mindestens einen Mono-, Oligo- bzw. Polysaccharid-Rest enthalten, der glycosidisch an vorzugsweise ein Sphingoid-Rückgrat gebunden ist.

Das Sphingoid-Rückgrat umfasst hierbei bevorzugt die folgenden Aminoalkohole: Sphingosin (d18:1, auch 4-Sphingenin), Dihydrosphingosin (d18:0, auch Sphinganin), C20-Dihydrosphingosin (d20:0, auch Eicosasphinganin), Phytosphingosin (t18:0, auch 4-Hydroxysphinganin), C20-Phytosphingosin (t20:0, auch 4-Hydroxyeicosasphinganin), Dehydrophytosphingosin (t18:1, auch 4-Hydroxy-8-sphingenin), Sphingadienin (d18:2, auch 4,8-Sphingadienin) sowie deren Strukturanaloga.

Ist die Amino-Gruppe des Sphingoid-Rückgrates mit einer Fettsäure verbunden, so spricht man von "Ceramiden".

**neutrale Glycosphingolipide**

I) Mono-, Oligo-, und Polyglycosylceramide:

Analog zu den oben aufgeführten Definitionen, sind Glycosylceramide Glycolipide, die mindestens einen Mono-, Oligo- bzw. Polysaccharid-Rest enthalten, der glycosidisch an ein Ceramid gebunden ist. Die Monoglycosylceramide werden auch als "Cerebroside" bezeichnet.

Die häufigsten Monoglycosylceramide bei Wirbeltieren sind die Galactocerebroside, bei denen ein Galactose-Rest glycosidisch an ein Sphingoid-Rückgrat aus Sphingosin oder Dihydrosphingosin gebunden ist, welches an seiner Amino-Gruppe mit einer nichthydroxylierten oder an Position 2 hydroxylierten Fettsäure mit einer Kettenlänge von 20 bis 24 Kohlenstoffatomen verknüpft ist. Ebenso konnten bei Wirbeltieren (vor allem in Blut und Milz) und bei Wirbellosen Glucocerebroside nachgewiesen werden, die als Saccharid-Rest Glucose an Stelle von Galactose enthalten. In Pflanzen kommen im Vergleich zu den ebenfalls vorhandenen Galactocerebrosiden hauptsächlich Cerebroside vor, die Glucose als Saccharid-Rest besitzen, hierbei handelt es sich bevorzugt um Glucocerebroside, deren Sphingoid-Rückgrat aus Phytosphingosin besteht und mit einer an Position 2 hydroxylierten Fettsäure (gesättigt oder einfach ungesättigt) mit einer Kettenlänge von 16 bis 24 Kohlenstoffatomen verknüpft ist. Glucocerebroside mit 4,8-Sphingadienin als Sphingoid-Rückgrat wurden in lipoiden Phasen, die aus Sojabohnen oder Mandeln extrahiert wurden, nachgewiesen. In Wolfsmilchgewächsen wurden Glucocerebroside mit Dehydrophytosphingosin als Sphingoid-Rückgrat und hydroxylierten Fettsäuren variabler Länge nachgewiesen.

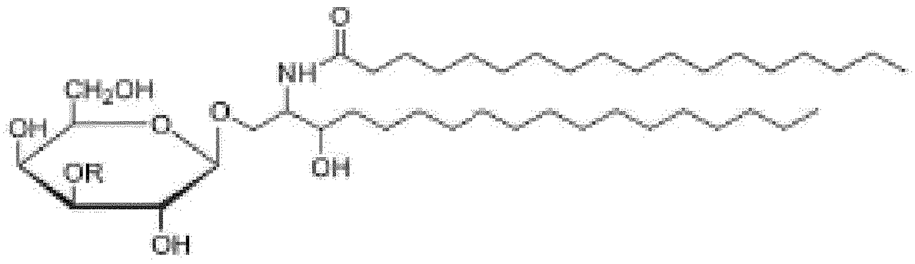

Galactocerebrosid (R=H) mit Ceramid aus Dihydrosphingosin-Rückgrat und Stearinsäure

Neben den Monoglycosylceramiden, existiert auch eine Vielzahl an höher glycosylierten Glycosylceramiden. Zu den neutralen Oligoglycosylceramiden gehören die Digalactosylceramide, die Lactosylceramide, die Triglycosylceramide (früher als Globotriaosylceramide bezeichnet) sowie die Tetraglycosylceramide, die in tierischen aber auch in pflanzlichen Geweben vorkommen. Zu den Tetraglycosylceramiden gehört das menschliche N-Acetylgalactosaminyl-galactosyl-galactosyl-glucosylceramid.

Es folgt Tabelle **2,** in der eine Auswahl an tierischen neutralen Glycosphingolipiden (Mono-, Oligo-, und Polyglycosylceramide) dargestellt ist.

Tabelle 2: Liste neutraler Glycosphingolipide in Säugetieren (Quelle: Glycoscience III: Chemistry and Chemical Biology. Fraser-Reid, B.; Tatsuta, K.; Thiem, J. (Ed.), Springer Verlag, 2001, ISBN 3-540-67765-8)

| Structure | Trivial name | Symbol[1] | Abbreviation |
|---|---|---|---|
| Glcβ1→1Cer | Glucosylceramide | GlcCer | - |
| Galβ1→4Glcβ1→1Cer | Lactosylceramide | LacCer | - |
| GalNAcβ1→4Galβ1→4Glcβ1→1Cer | Ganglictriaosylceramide | $GgOse_3Cer$ | Gg3 |
| Galβ1→3GalNAcβ1→4Galβ1→4Glc β1→1Cer | Gangliotetraosylceramide | $GgOse_4Cer$ | Gg4 |
| GalNAcβ1→4Galβ1→3GalNAcβ1→ 4Galβ1→4Glcβ1→1Cer | Gangliopentaosylceramide | $GgOse_5Cer$ | Gg5 |
| Galα1→4Galβ1→4Glcβ1→1Cer | Globotriaoslyceramide | $GbOse_3Cer$ | Gb3 |
| GalNAcβ1→3Galα1→4Galβ1→4Glc β1→1Cer | Globotetraosylceramide | $GbOse_4Cer$ | Gb4 |
| Galβ1→3GalNAcβ1→3Galα1→4Gal β1→4Glcβ1→1Cer | Globopentaosylceramide | $GbOse_5Cer$ | Gb5 |
| GalNAcα1→3GalNAcβ1→3Galα1→ 4Galβ1→4Glcβ1→1Cer | Forssman GSL | - | - |
| GlcNAcβ1→3Galβ1→4Glcβ1→1Cer | Lactotriaosylceramide | $LcOse_3Cer$ | Lc3 |
| Galβ1→3GlcNAcβ1→3Galβ1→4Glc β1→1Cer | Lactotetraosylceramide | $LcOse_4Cer$ | Lc4 |

(fortgesetzt)

| Structure | Trivial name | Symbol[1] | Abbreviation |
|---|---|---|---|
| Galβ1→4GlcNAcβ1→3Galβ1→4Glc β1→1Cer | Neolactotetraosylceramide | $nLcOse_4Cer$ | nLc4 |
| Galβ1→4GlcNAcβ1→3Galβ1→4Glc NAcβ1→3Galβ1→4Glcβ1→1Cer | Neolactohexaosylceramide | $nLcOse_6Cer$ | nLc6 |
| Galβ1→4GlcNAcβ1→3Galβ1→4Glc NAcβ1→3Galβ1→4GlcNAcβ1→ 3Galβ1→4Glcβ1→1Cer | Neolactooctaosylceramide | $nLcOse_8Cer$ | nLc8 |

| | **Tier** |
|---|---|
| C 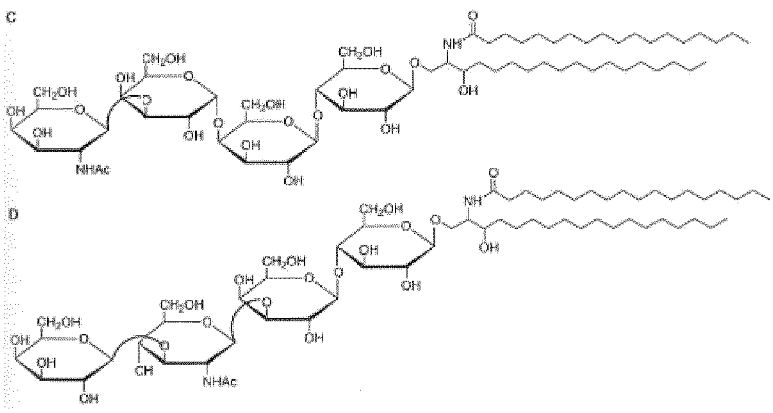 D | Globotetraosylceramid mit Ceramid aus Dihydrosphingosin-Rückgrat und Stearinsäure |
| | Lactotetraosylceramid mit Ceramid aus Dihydrosphingosin-Rückgrat und Stearinsäure |

| **Pflanze** |
|---|
| 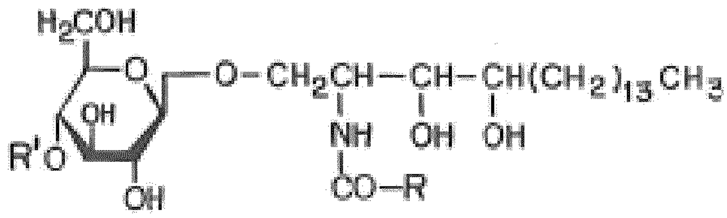 (6) RCO = acyl ; R' = H<br>(6') RCO = acyl ; R' = Glcpβ<br>(6") RCO = acyl ; R' = Manpβ<br>(6"') RCO = acyl ; R' = Manpβ1-4Manpβ<br>Glucosylceramid mit Ceramid aus XYZ |
| 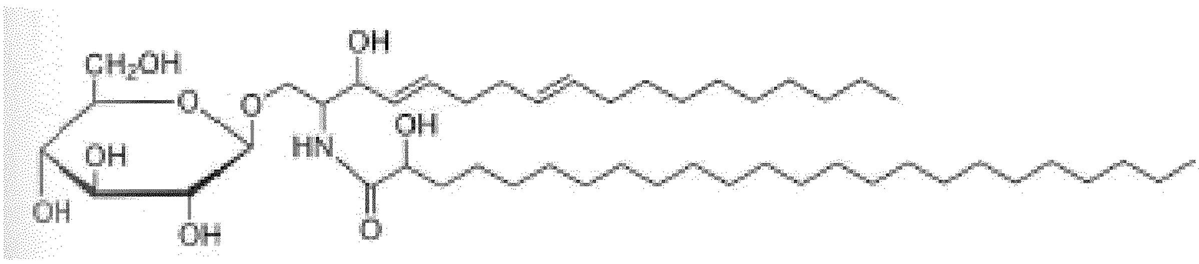 |

(fortgesetzt)

| **Pflanze** |
| --- |
| Glucocerebrosid mit Ceramid aus 4,8-Sphingadienin und 2-Hydroxy-Tetracosansäure |

II) Mono-, Oligo-, und Polyglycosylsphingoide

Eine weitere Gruppe der neutralen Glycosphingolipide sind die Glycosylsphingoide, bei denen zumindest ein Saccharid-Rest glycosidisch an ein Sphingoid-Rückgrat gebunden ist, dies jedoch an seiner Aminogruppe nicht mit einer Fettsäure verknüpft ist. Man spricht daher auch von deacetylierten Glycosylceramiden. Hierzu gehören zum Beispiel die im Gehirn von Vertebraten vorkommenden O-Sphingosylgalactoside (früher als Psychosine bezeichnet).

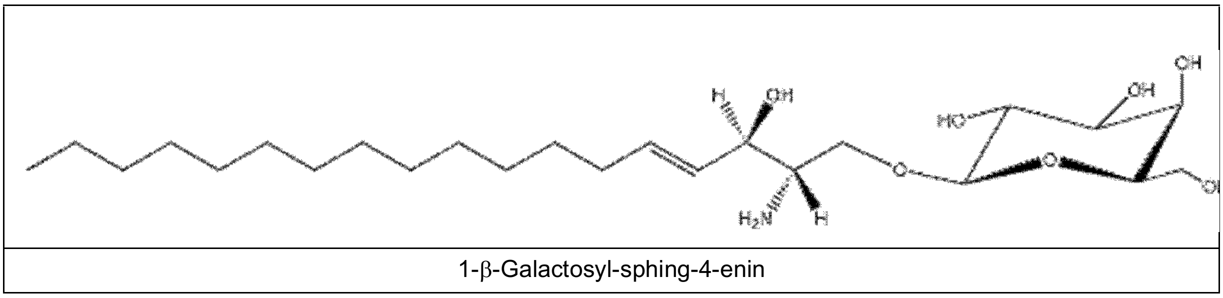

1-β-Galactosyl-sphing-4-enin

III) saure Glycosphingolipide: Glycosphingolipide, die als saure Gruppe einen Sulfat-Rest, einen Phosphat-Rest oder eine Carboxyl-Rest besitzen. Zu ihnen gehören folgende Untergruppen:

IV) Sialoglycosphingolipide:

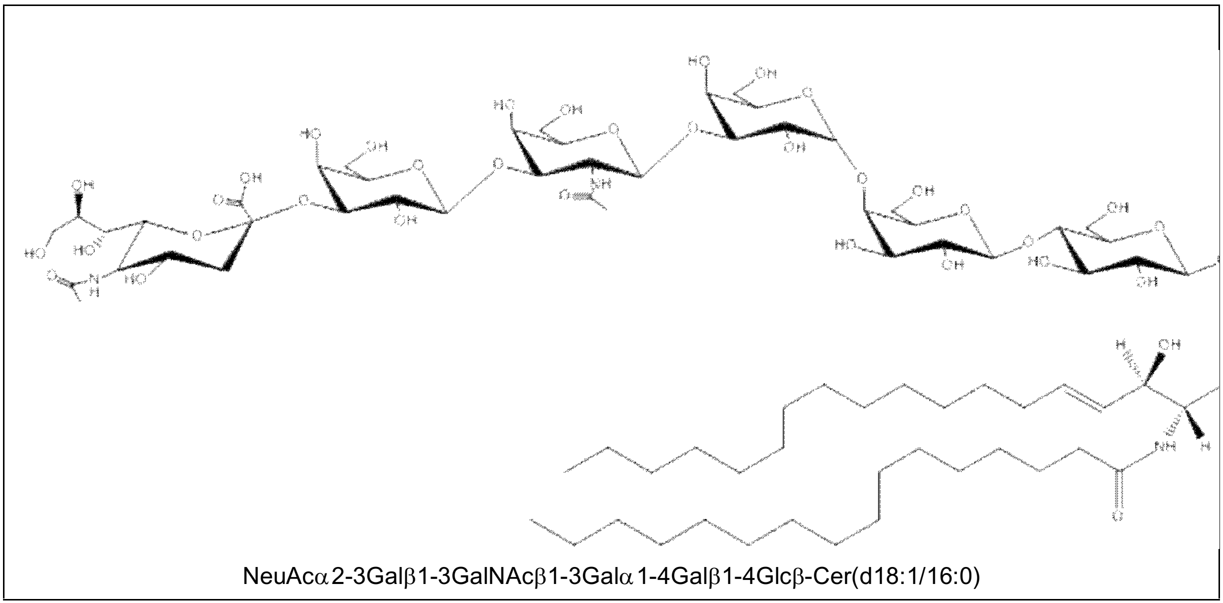

NeuAcα2-3Galβ1-3GalNAcβ1-3Galα1-4Galβ1-4Glcβ-Cer(d18:1/16:0)

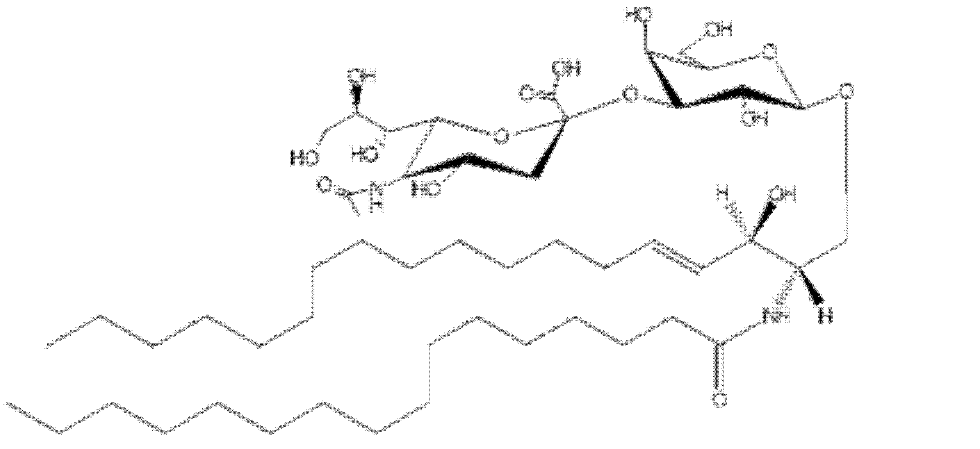

NeuAcα2-3Galβ-Cer(d18:1/16:0)

Glycuronoglycosphingolipide: Glycuronoglycosphingolipide sind Sphingolipide, die einen oder mehrere Uronsäure-Reste enthalten.

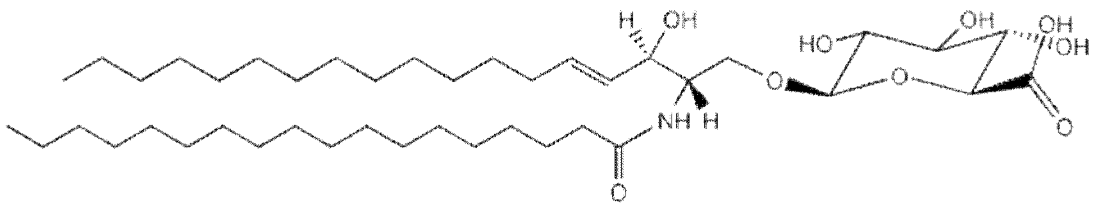

GlcAβ-Cer(d18:1/18:0)

V) Sulfoglycosphingolipide: Sphingolipide, die eine oder mehrere Sulfatester an Saccharid-Resten enthalten. Hierbei handelt es sich in der Regel um Galactocerebroside, wobei die Galactose an Position 3 mit einer Sulfatgruppe verestert ist. An diesen sulfatierten Galactose-Rest können weitere Saccharid-Reste geknüpft sein, bei denen es sich bevorzugt um Glucose-Reste handelt. Ein prominenter Vertreter ist das in der Myelinscheide von Säugetierneuronen vorkommende 3-O-Sulfogalactosylcerebrosid.

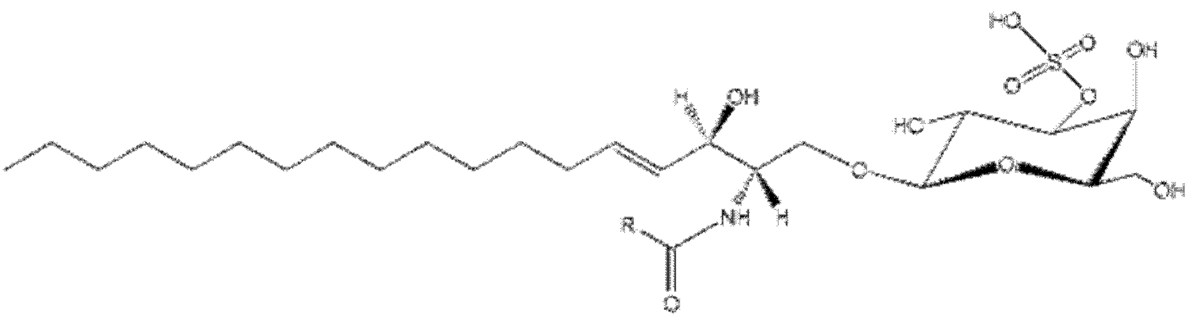

(3’-Sulfo)Galβ-N-(acyl)-sphing-4-enin

(fortgesetzt)

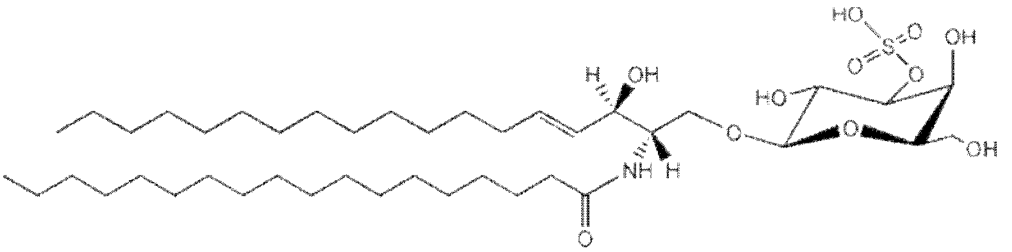

(3'-Sulfo)Galβ-Cer(d18:1/18:0)

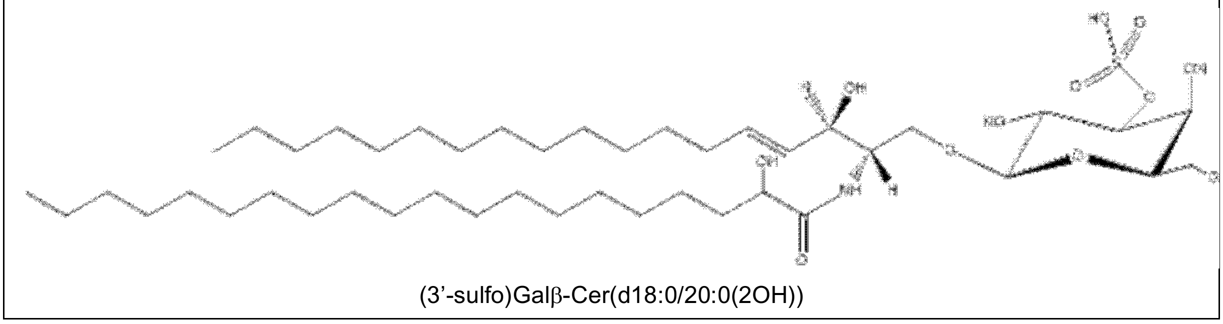

(3'-sulfo)Galβ-Cer(d18:0/20:0(2OH))

VI) Phosphoglycosphingolipide: Phosphoglycosphingolipide sind Sphingolipide, die eine oder mehrere Phosphomonoester- oder Phosphodiestergruppen enthalten. In der Regel ist ein Ceramid über seine Hydroxy-Gruppe an Position 1 mit einem Inositolphosphat verknüpft. Man spricht daher auch teilweise von "Inositol-Phosphorylceramiden". Bei Pflanzen kann das Inositolphosphoceramid-Rückgrat weitere N-Acetylglucosamin-, Glucosamin-, Fucose-, Glucuron-, Arabinose-, Mannose- oder Galactose-Reste tragen, die entweder an das Kohlenstoffatom in Position 2 und oder Position 6 des Inositols gebunden sein können.

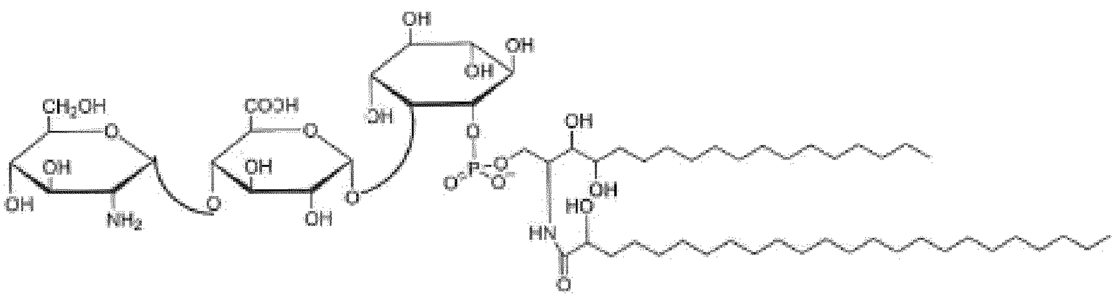

Cer - P - Ins - Glucuronic acid - Acetylglucosamine

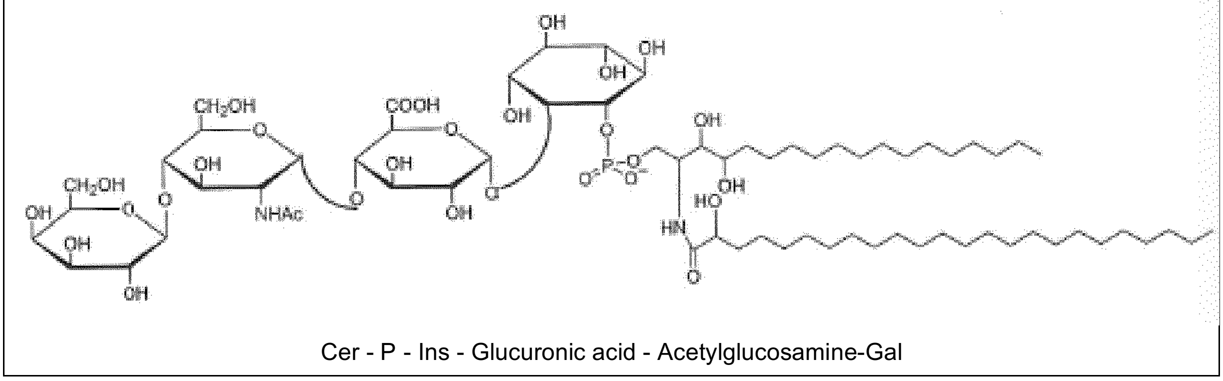

Cer - P - Ins - Glucuronic acid - Acetylglucosamine-Gal

(fortgesetzt)

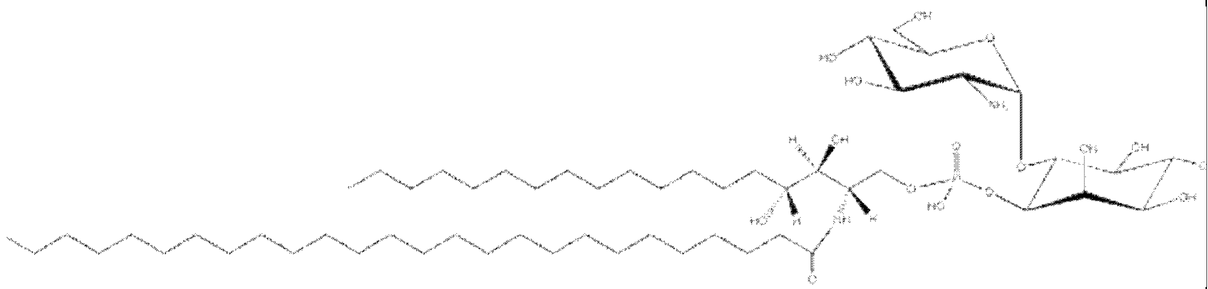

GlcNα1-6Ins-1-P-Cer(t18:0/26:0)

VII) Phosphonoglycosphingolipide sind Sphingolipide, die vornehmlich in Wirbellosen vorkommen und die sich durch zumindest eine Phosphonsäureester-Bindung auszeichnen.

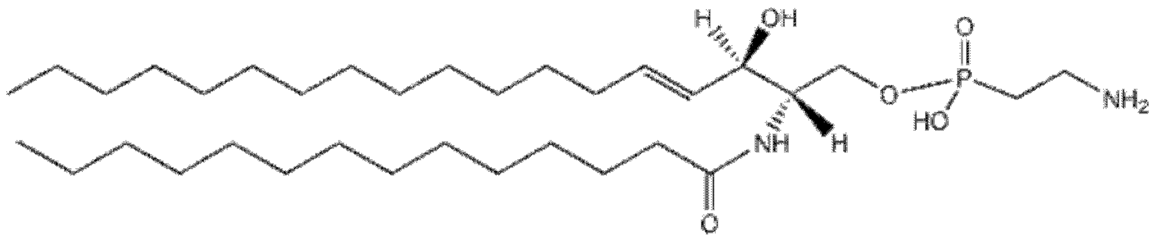

N-(Tetradecanoyl)-sphing-4-enine-1-(2-aminoethylphosphonat)

Glycophosphatidylinositole: Glycophosphatidylinositole sind Glycolipide, bei denen Saccharide glycosidisch an die Inositol-Gruppe von Phosphatidylinositolen gebunden sind. Sie umfassen hierbei sowohl die entsprechenden lyso-Formen der Glycophosphatidylinositole als auch Glycophosphatidylinositole mit substituierten Glycerin-bzw. Inositol-Resten, z.B. durch O-acyl-, O-alkyl- oder O-alk-1-en-1-yl-Substitutionen.

Rhamnolipide: Rhamnolipide bestehen aus ein oder zwei Rhamnose-Einheiten, die an eine Hydroxy-Gruppe einer hydroxylierten Fettsäure gebunden sind, welche wiederum mit einer weiteren hydroxylierten Fettsäure verestert ist, wobei die Fettsäuren eine Kettenlänge von bevorzugt 8 bis 12 Kohlenstoffatomen, weiter bevorzugt jedoch von 10 Kohlenstoffatomen besitzen. Rhamnolipide werden bevorzugt von Bakterien der Gattung *Pseudomonas* gebildet, die auf einer Kohlenwasserstoffquelle wachsen, und besitzen antifungale Eigenschaften.

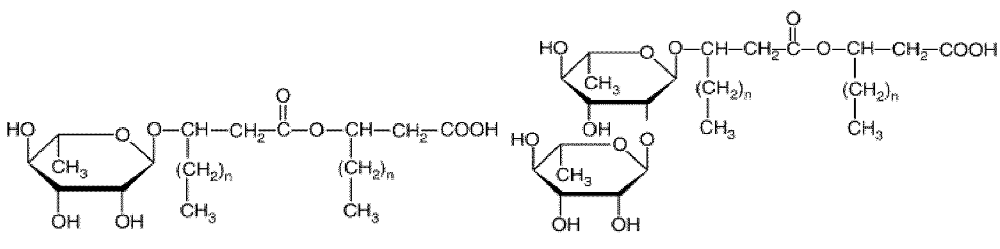

Monorhamnolipids Dirhamnolipid

Sophorolipide: Sophorolipide bestehen aus dem Disaccharid Sophorose (auch 2-0-Glucopyranosyl-D-glucopyranose), die an die Hydroxy-Gruppe einer hydroxylierten Fettsäure gebunden ist, wobei sich die Hydroxy-Gruppe entweder an dem endständigen Kohlenstoffatom (Position n) oder der unmittelbar vorhergehenden Position (Position n-1) handelt. Die Sophorose kann zudem an einer oder beiden Hydroxy-Gruppen an Position 6 acetyliert sein. Eine Untergruppe der Sophorolipide sind die laktonischen Sophorolipide, bei denen die Carboxyl-Gruppe der hydroxylierten Fettsäure mit der Hydroxy-Gruppe in 4'-Position der Glucose-Untereinheit der Sophorose verestert ist. Sophorolipide werden bevorzugt von Hefen der Gattung *Candida,* besonders bevorzugt jedoch von *Candida bombicola* und *Candida apicola,* gebildet und ausgeschieden.

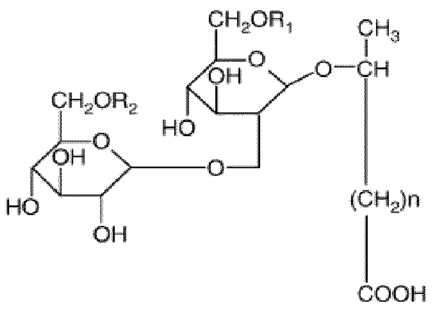

Acidic sophorolipid

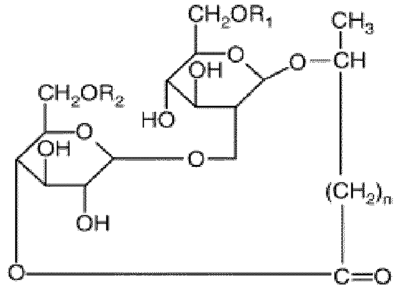

Lactonic sophorolipid

Es sei an dieser Stelle angemerkt, dass auch in Pflanzen einige Fettsäureglycoside nachgewiesen werden konnten, bei denen ein Saccharid-Rest mit einer hydroxylierten Fettsäure über eine glycosidische Bindung zwischen zwei Hydroxy-Gruppen verknüpft ist. Ein Beispiel hierfür ist z.B. das Tuberonsäure-Glycosid aus der Kartoffelpflanze, bei dem die Fettsäure Tuberonsäure (12-Hydroxy-Jasmonsäure) über die Hydroxy-Gruppe an Position 12 glycosidisch mit der Hydroxy-Gruppe an Position 1 von Glucose verbunden ist.

Neben den oben aufgeführten Fettsäureglycosiden, bei den ein Saccharid-Rest an eine Fettsäure über eine glycosidische Bindung zwischen zwei Hydroxy-Gruppen gebunden ist, existieren auch Fettsäureglycoside, die durch eine Esterbindung zwischen einem Saccharid-Rest und einer Fettsäure entstehen. Die folgende Strukturformel zeigt ein Fettsäureglycosid aus Cyanobakterien.

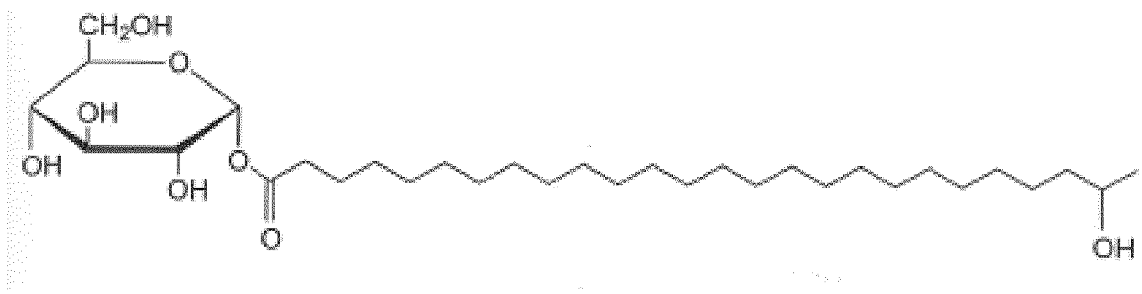

1-α-Glucosyl-25-hydroxy-hexacosansäure

Zum Beispiel existieren in Nachtschattengewächsen Fettsäureglycoside, bei denen ein Glucose-Rest oder ein Saccharose-Rest an zwei bis fünf Hydroxy-Gruppen acyliert ist, wobei die Kettenlänge der Acylgruppen bevorzugt 2 bis 12 Kohlenstoffatome beträgt. Beispiele umfassen 2,3-Diacylglucose, 1,2,3-Triaclyglucose, 2,3,4-Triacylglucose, 2,3,4-Triacylsaccharose, 2,3,6-Triacylsaccharose, 2,3,1'-Triacylsaccharose, 1,2,3,4-Tetraacylglucose, 2,3,4,6-Tetraacylglucose, 2,3,4,6-Tetraacylsaccharose, 2,3,4,1'-Tetraacylsaccharose, 2,3,4,3'-Tetraacylsaccharose, 1,2,3,4,6-Pentaacylglucose und 2,3,4,6,3'-Pentaacylsaccharose.

Trehalose-Lipide: Trehalose-Lipide bestehen aus dem Disaccharid Trehalose (1-α-Glucopyranosyl-1-α-Glucopyranosid oder Glc(α1→1)Glc), welches über Esterbindungen mit zumindest einer an Position 2 verzweigten und an Position 3 hydroxylierten Fettsäure verknüpft ist. Bevorzugt handelt es sich bei diesen Fettsäuren um Mykolsäuren oder deren Derivate Corynomykolsäure oder Nocardomykolsäure. Ist nur die Hydroxy-Gruppe an Position 6 eines der beiden Glucose-Einheiten der Trehalose verestert, spricht man von Monomykolaten. Sind jedoch beide Glucose-Einheiten der Trehalose an ihrer Hydroxy-Gruppe in Position 6 verestert, so spricht man von Dimykolaten. Ein der bekanntesten Vertreter der Trehalose-Lipide ist der so genannte Cord-Faktor (ein Trehalose-6-6'-dimykolat) aus *Mycobacterium tuberculosis,* der für die Virulenz und die Wirkstoff-Resistenz des Bakteriums von Bedeutung zu sein scheint. Darüber hinaus sind polyacylierte Formen von Trehalose-Lipiden bekannt, bei denen mehr als zwei Fettsäurereste mit der Trehalose verknüpft sind (z.B Triacyltrehalosen und Pentaacyltrehalosen). Bei den Trehalose-Lipiden handelt es sich folglich um komplexe Glycolipide, die lange und zudem auch komplex verzweigte Fettsäurereste enthalten können.
Trehalose-Lipide kommen bevorzugt in Bakterien der Gattungen Mycobacterium, Rhodococcus und Corynebacterium sowie in Pilzen, Algen und auch in Insekten vor.

| | |
|---|---|
| 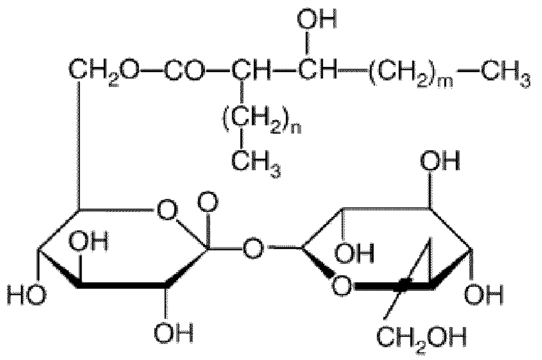 | 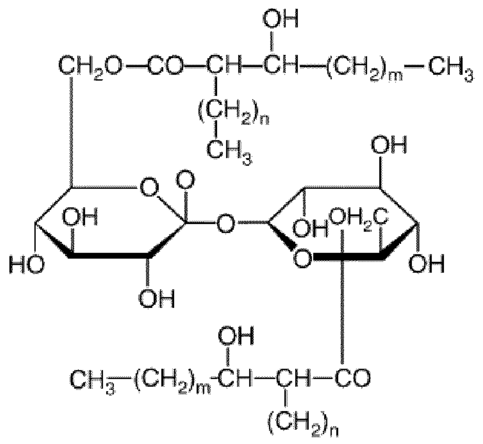 |
| m+n = 27 TO 31<br>Trehalose monomycolates | m+n = 27 TO 31<br>Trehalose dimycolates |

Lipopolysaccharide: Die Lipopolysaccharide sind höchst komplexe bakterielle Glycolipide, die aus Lipid A und einem daran gebundenen Polysaccharid-Komplex bestehen, welcher wiederum in eine Kernregion und ein damit verbundenes O-spezifisches Polysaccharid unterteilt werden kann. Lipid A ist ein Fettsäureglycosid aus einen Disaccharid aus zwei N-Acetylglucosaminphosphat-Einheiten, wobei dieses Dissacharid mit mehreren Fettsäureresten verestert ist. Hierbei sind die häufigsten Fettsäuren Capron-, Laurin-, Myristin-, Palmitin- und Stearinsäure. Über die Hydroxy-Gruppe in Position 6 des zweiten N-Acetylglucosaminphosphats ist das Lipid A mit der Kernregion des anschließenden Polysaccharid-Komplexes verbunden.

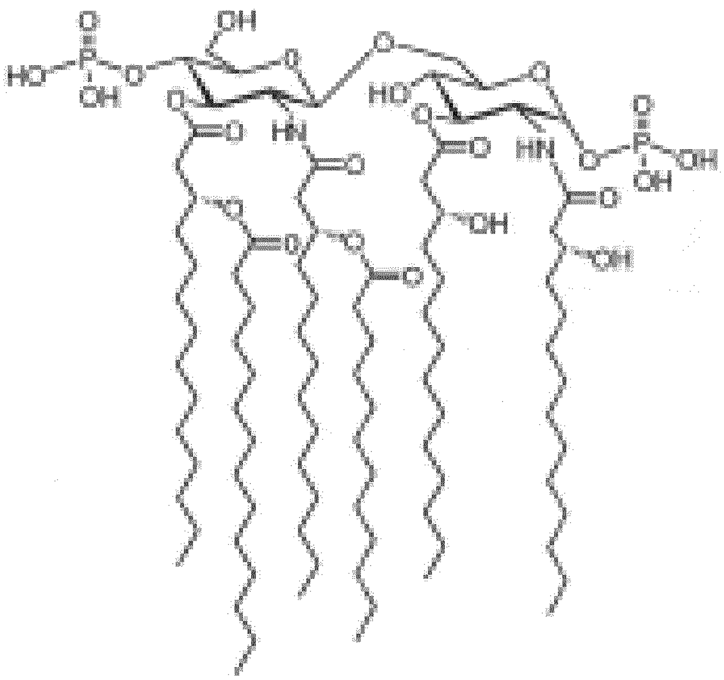

Lipid A von E.coli

**Sterylglykoside:** Bei den Sterylglykosiden handelt es sich um Sterole, die über eine Hydroxygruppe glykosidisch mit zumindest einem Saccharid-Rest verknüpft sind. Sterylglykoside kommen in Pflanzen, Tieren, Pilzen und auch in einigen Bakterien vor. In Tieren existiert beispielsweise das Cholesterol-Glucoronid, bei dem ein Cholesterol-Rest mit einem Glucuronsäure-Rest verknüpft ist. Bei Pflanzen handelt es sich bei dem Sterol-Rest bevorzugt um Campesterol, Stigmasterol, Sitosterol, Brassicasterol oder Dihydrositosterol und bei dem Saccharid-Rest bevorzugt um Glucose, Galactose, Mannose, Glucuronsäure, Xylose, Rhamnose oder Arabinose. Der Saccharid-Rest ist bei pflanzlichen Sterylglykosiden über die Hydroxygruppe am C3 des A-Ringes des Sterols mit dem Sterol verbunden. An diesen ersten Saccharid-Rest können weitere Saccharid-Reste über eine ß-1,4-glykosidische Bindung oder eine ß-1,6-glykosidische Bindung geknüpft sein. Es existieren zu dem acylierte Sterylglykoside (ASGs), bei denen ein Saccharid-Rest an seiner Hydroxygruppe an Position 6 mit einer Fettsäure verestert ist. In vielen Pflanzen konnten acylierte Sterylglykoside in praktisch allen Pflanzenteilen in bis zu 0,125 Gew.-% nachgewiesen werden. Besonders hoch ist der Anteil an nichtacylierten und acylierten Sterylglykosiden in Palm- und Sojaöl. Bei der Herstellung von Biodiesel wird ein hoher Anteil an Sterylglykosiden im Zusammenhang mit einer schlechteren Filtrierbarkeit diskutiert.

| Pflanze | |
|---|---|
| 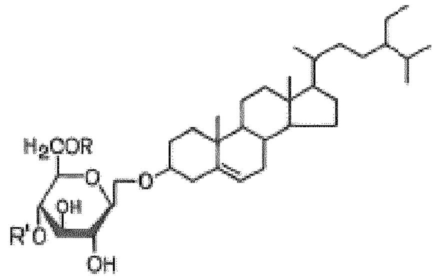 (5) R = H ; R' = H<br>(5') R = $-COC_{15}H_{31}$ ; R' = H<br>(5") R = H ; R' = Glcpβ<br>(5"') R = H ; R' = Glcpβ1–4Glcpβ | 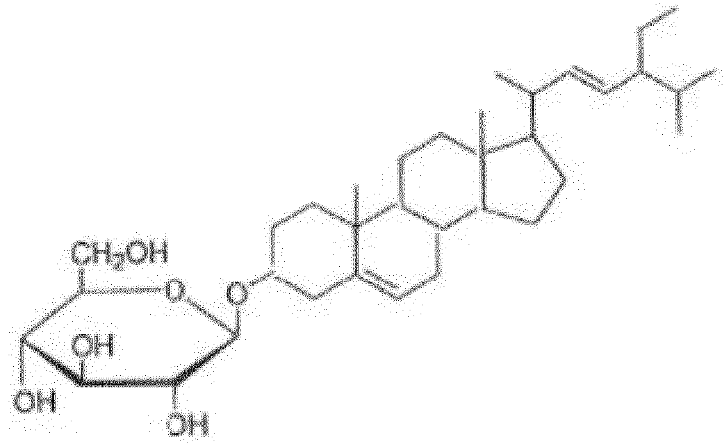 |
| ß-Sitosterol-glucoside | Stigmasterin-glucoside |

**Beschreibung der Figuren**

**Fig. 1:** Figur 1 zeigt ein typisches Bild einer nach Schritt B1) in einem Probengefäß mittels Zentrifugation von der wässrigen Phase (unten) separierte glycoglycerolipidarme lipoide Phase (oben).

**Fig. 2:** zeigt die HLB-Lipophilitätsskala, wobei im Bereich von 10 bis 0 die Lipophilie ansteigt und im Bereich von 10 bis 20 die Hydrophilie ansteigt und um 10 die Substanzen gleichermaßen lipophil wie hydrophil sind, d.h. sie sind equi-amphiphil. Beispielhaft ist für verschiedene TWEEN und SPAN Emulgatoren der Wert gemäß HLB-Lipophilitätsskala angegeben.

**Fig. 3:** Figur 3 zeigt eine erfindungsgemäße Vorrichtung zur Ausführung der hierin beschriebenen Verfahren. **1** bedeutet ein Aufnahmegefäß für die Aufnahme der wässrigen Phase, enthaltend die erwähnten Salze, **2** steht für eine Leistung, **3** für einen Behälter, **4** steht für eine Überlaufrückführung, **5** ist eine Ablaufleitung, **6** ist ein Ventil, **7** ein Mischer, **8** eine Zuleitung, **9** Ablaufleitung, **10** eine Zentrifuge, **11** und **12** sind zwei Abläufe aus der Zentrifuge, **13** eine Pumpe, **14** eine weitere Pumpe und **15** ein Verteiler.

**Beispiele**

**Methoden**

Die Effektivität der erfindungsgemäßen Technik zur Abtrennung einer glycoglycerolipidreichen Fraktion aus lipoiden Phasen lässt sich durch verschiedene Methoden aus dem Stand der Technik überprüfen.

Viskosimetrie

Es konnte gezeigt werden, dass die Abtrennung der Glycolipidfraktion die Viskosität der aufgereinigten lipoiden Phase deutlich verringert. Daher wird als erfindungsgemäßer Effekt eine Reduktion der Viskosität der aufgereinigten lipoiden Phase um mind. 10% mehr bevorzugt um mind. 20% und am meisten bevorzugt um mind. 30% angesehen.

Erdalkalimetal- und Metallsalzbindungskapazität

Erdalkalimetallionen sowie Metallionen verteilen sich praktisch nicht in einer apolaren Lipidphase. Zuckerreste von Glycoglycerolipiden und Glycosphingolipiden sind in der Lage, solche Ionen über Wasserstoffbrückenbindungen zu fixieren, wodurch viele lipoide Phasen wie z. B. Pflanzenöle mit diesen Ionen belastet werden. Das Bindungsvermögen einer glycoglycerolipidhaltigen lipoiden Phase gegenüber derartigen Ionen kann daher zur Abschätzung des Gehalts an Zuckerverbindungen herangezogen werden. Mit dem erfindungsgemäßen Verfahren wird bevorzugt eine Abreicherung der Bindungskapazität für Erdalkalimetallionen sowie Metallionen um 80%, mehr bevorzugt um >90% und am meisten bevorzugt um >95% erreicht.

Zur Prüfung der physiko-chemischen Eigenschaften der abgetrennten glycoglycerolipidhaltigen Fraktion können etablierte Verfahren verwerdet werden wie z.B., HLB-Chromatographie, Tensiometrie sowie Bestimmung der kritschen micellaren Konzentration (CMC).

Für den qualitativen Nachweis von Glycoglycerolipiden und Glycosphingolipiden können Verfahren wie die Atom-Emissionsspektroskopie und Dünnschichtchromatographie eingesetzt werden. Durch letztere kann eine Auftrennung in unterschiedliche Verbindungsklassen mit anschließender Differenzierung der vorliegenden Zuckerreste erfolgen. Eine enge und scharfe Begrenzng der sich darstellenden Banden deutet auf eine hohe Einheitlichkeit der hierin befindlichen Verbindungen hin, während eine Verbreiterung und unscharfe Begrenzng der Banden auf eine Heterogenität der Verbindungen und insbesondere der Zuckerreste hindeutet und somit für den Nachweis einer erfolgten Hydrolyse geeignet ist.

Abkürzungen:

| | |
|---|---|
| FFA | freie Fettsäuen |
| ppm | parts per milion |
| na | nicht anwendbar/ nicht geprüft |
| n. u. | nicht untersucht |
| rpm | Umdrehungen pro Minute |

**Beispiel 1:**

Gewinnung einer Glykolipidfraktion nach Entschleimung eines Pressöls

Zur Prüfung, ob nach erfolgter Entschleimung eines Pressöls bzw. Abtrennnung von noch in der lipoiden Phase verbliebenen Fettsäuren mittels ener wässrigen Lösung, die Guanidinverbindungen enthält, wurde ein gefiltertes Rohöl einer Schneckenpressung von gelagerten Jatrophanüssen, das bei einer Temperatur von 60°C mit den Ausgangskennzahlen: Gesamtphosphorgehalt 248 ppm, FFA 1,8 %, Calzium 70 ppm, gewonnen wurde, wird nach folgendem Schema raffiniert:

a) 3%ige Zugabe einer 0,5-1,0 molaren NaOH-Lösung
b) 3%ige Zugabe einer 75%igen Phosphorsäurelösung
a1) raffiniertes Öl nach Schritt a) 0,3%ige Zugabe einer 0,6 molaren Argininlösung
b1) raffiniertes Öl nach Schritt b) 0,3%ige Zugabe einer 0,6 molaren Argininlösung

Die mit den zuvor beschriebenen Schritten raffinierten Öle wurden dann jeweils mit den folgenden Schritten weiterbehandelt:

c) 5%ige Zugabe einer 10%igen Natrium-Metasilikat-Lösung
d) 4%ige Zugabe einer 15%igen Kalium-Carbonat-Lösung

Für die Versuche wurden jeweils 200ml aus jedem Raffinationsschritt untersucht. Dabei erfolgte die Einbringung der wässrigen Lösungen durch eine Homogenisierung mit einem Ultrathurrax bei 24000 Umdrehungen pro Minute für 3 Minuten unter manueller kreisender Bewegung des Vorratsgefäßes bei Raumtemperatur. Die resultierenden Emulsionen wurden in einer Becherglaszentrifuge mit 3800 rpm für 5 Minuten zentrifugiert. Die anschließende Phasenabtrennung erfolgte durch Dekantieren oder Abziehen der oberen Phasen.

In den Ölphasen wurde der Gehalt an Phosphor, freien Fettsäuren und Calzium bestimmt und in den wässrigen Phasen der Raffinationsschritte c) und d) die Trockensubstanzmengen. Hierzu erfolgte ein Wasserentzug durch eine Vakuumtrocknung. Ferner wurde das Wasserbindungvermögen der zuvor raffinierten Öle (a1 und b1) sowie der nach dem erfindungsgemäßen Verfahren reduzierten lipoiden Phasen (a1) + c), a1) + d), b1) + c), b1) + d)) ermittelt, indem in die erhaltene Ölphase deionisiertes Wasser (1ml in 50ml) mit einem Ultrathurrax (20.000 Umdrehungen pro Minute für 2 Minuten) eingebracht wurde und anschließend mit 4000 rpm in einer Zentrifuge abgetrennt wurde. Die Bestimmung des Wassergehaltes des Öls erfolgte nach dem Karl-Fischer-Verfahren. Die getrockneten Feststoffgemische wurden in Chloroform gelöst, anschließend Zentrifugation mit 5000 rpm für 5 Minuten. Abtrennen der Lösungsmittelphase, die anschließend vollständig mittels Vakuumverdampfer entfernt wird. Es werden je 20 mg der so erhaltenen Feststoffmenge in 1 L deionisiertem Wasser aufgelöst. Von diesen Lösungen wurden Proben entnommen und die Oberflächenspannung mit einem Tensiometer (K 100, Krüss, Deutschland) bestimmt.

Ergebnisse:

Die Entschleimungsverfahren führten zu einer weitgehenden Entfernung von hydratisierbaren Phospholipiden, freien Fettsäuren und einer deutlichen Reduktion von Erdalkalimetallionen. Durch den erfindungsgemäßen Intensiveintrag einer wässrigen Lösung der erfindungsgemäß verwendeten Salzverbindungen konnten relevante Mengen an lipophilen Begleitstoffen in das wässrige Medium überführt und entfernt werden. Die gewonnenen Trockensubstanzmengen waren dabei erheblich größer als die rechnerische Summe von auch abgetrennten Restmengen an Phospholipiden, Fettsäuren und Erdalkalimetallen. Aus der erhaltenen Trockenmasse können Glycerolipide in ein organisches Lösungsmitel überführt und aus diesem zurückgewonnen werden. Die Glycerolipide weisen in Wasser außerordentlich gute tensidische Eigenschaften auf.

**Beispiel 2:**

Die ölige Fraktion nach Sedimentation der Pressflüssigkeit der Accrocromia Palmenfrucht mit den Kenndaten: Phosphorgehalt 128ppm, FFA 2,6%, Calzium 48,8 mg pro kg lipoide Phase wurden hinsichtich abtrennbarer Glykolipidfraktionen untersucht.

Hierzu wurden je 200ml der lipoiden Phase nach folgenden Verfahren vorbehandelt:

a) Einrühren von 5% ionenarmen Wasser mit einem Propellerrührer mit 2500 rpm unter Erwärmen auf 50°C für 90 Minuten

b) 3%ige Zugabe einer Zitronensäurelösung, die mittels eines Ultrathurrax über 5 Minuten mit 24Tsd rpm homogenisiert wird. Dabei Erwärmung der Emulsion auf ca. 50°C.

Die mit den zuvor beschriebenen Schritten raffinierten lipoiden Phasen wurden nach Zentrifugation jeweils mit den folgenden Schritten weiterbehandelt:

c) 8%ige Zugabe einer 15%igen Kupferacetat-Lösung
d) 4%ige Zugabe einer 20%igen Natrium-Hydrogencarbonat-Lösung

Die wässrigen Phasen von c) und d) werden mittels eines Ultrathurrax über 5 Minuten mit bei 24000 Umdrehungen pro Minute homogenisiert. Dabei Erwärmung der Emulsion auf ca. 50°C.

Die resultierenden Emulsionen wurden in einer Becherglaszentrifuge mit 4.000 rpm für 5 Minuten zentrifugiert. Die anschließende Phasenabtrennung erfolgte durch Dekantieren oder Abziehen der oberen lipoiden Phasen. Die lipoiden Phasen der Raffinationsschritte c) und d) werden dem gleichen Raffinationsverfahren erneut zugeführt und werden als c1) und d1) bezeichnet. Anschließend werden die hieraus resultierenden lipoiden Phasen c1) mit dem Verfahren d) und d1) mit dem Verfahren c) in gleicher Weise behandelt.

In den lipoiden Phasen wurde der Gehalt an Phosphor, freien Fettsäuren und Calzium bestimmt und in den wässrigen Phasen der Raffinationsschritte c) und d) die Trockensubstanzmengen ferner erfolgte für letztgenannte Proben eine Bestimmung des HLB-Wertes. Die Analyse erfolgt mit ener Asahipak GF-310 HQ Mehrfach-Lösungsmittel GPC-Säule. Hierduch können ionische und nicht-ionische Tenside differenziert und nach ihrem HLB-Wert eingeordnet werden.

Dünnschichtchromatographie erfolgte mit Silica Gel G Platten. Die Trennung erfolgt mit einem Gemisch aus Chloroform/Aceton/Wasser (30/60/2). Die Entwicklung erfolgte mit einem Naphthyl ethylenediamine-Reagenz, wodurch Zuckerreste der Glycerolipide farblich dargestellt werden können.

Ergebnisse:

Die lipoide Phase einer Pressung von Palmkernschalenmaterial weist einen hohen Gehalt an Nicht-Triglyceridbegleitstoffen aus, der zum größten Teil durch Glycerolglyceride und Sterole bedingt ist und nur zu einem geringen Anteil durch Phospholipide. Sie bedingen auch eine Trübung und hohe Viskosität der öligen Phase. Durch eine wässrige oder saure Entschleimung wurden die Residualewerte für Phosphor, freie Fettsäuren und Erdalkalimetallionen bereits wesentlich abgesenkt, die lipoiden Phasen blieben allerdings noch hoch viskos. Durch den erfindungsgemäßen Intensiveintrag der wässrigen Lösungen der erfindungsgemäß verwendeten Verbindungen kam es zu einer erheblichen Emulsionsbildung mit einem weitern Viskositätsanstieg. Unter der Scherhomogenisierung verflüssigte sich diese Emulsion wieder und ließ sich durch Zentrifugation in eine leicht trübe lipoide Phase sowie eine weißliche halbfeste Masse auftrennen. Die in der ersten Abtrennstufe erreichte Abtrennmenge war weitgehend unabhängig von dem zuvor durchgeführten Entschleimungsverfahren und dem in den erfindungsgemäßen wässrigen Phasen gelösten Salz. Es zeigte sich, dass auch noch bei einer 2. Abtrennung relevante Mengen an Ölbegleitstoffen abgetrennt werden konnten. Ein erneuter erfindungsgemäßer Intensiveintrag mit den wässrigen Lösungen der Salze erbrachte praktisch keine zusätzliche Abtrennung von Begleitstoffen mehr. Die insgesammt abgetrennten Feststoffmengen liegen weit über der rechnerischen Summe der ebenfalls mit abgetrennten Phospholipide, Fettsäuren und Metallionen. Durch eine Dünnschichtchromatographie konnte zum einen die Mitabtrennung relevanter Mengen an Triacylglycerolen ausgeschlossen werden. Zum anderen konnten Digalactosyl- und Monogalactosyldiglyceride sowie Sterylglycoside nachgewiesen werden. Bei der Tensidanalytik zeigte sich ein diskreter Nachweis für das Vorhandensein von ionischen Tensiden mit einem HLB-Wert von 13 sowie ein deutlicher Nachweis von nichtionischen Tensiden mit einem mittleren HLB-Wert von 8 bzw. 9 für die abgetrennte Phase von c) und d).

**Beispiel 3**

Leinsamenpresskuchen wurde in eine wässrige Lösung mit einem 5%-igen Isopropanol-Zusatz gegeben und mit einem Stabmixer homogenisiert. Anschließend wurde die breiige Masse über 30 Minuten bei 50°C gerührt. Dann folgt die Hinzugabe einer 3-fachen Menge an Petrolether. Nach erneutem Homogenisieren erfolgt eine zentrifugale Phasentrennung. Die abgetrennte organische Phase (OP1) wird auf die Hälfte ihres Ausgangsvolumens mittels eines Vakuumverdampfers reduziert. Es folgen die Zugabe von 5 Vol-% eines Methanol-Wassergemisches (95/5) zu OP1, Mischen der Lösung und anschließend zentrifugale Phasenseparation. Die leicht trübe Alkohol-Wasserphase wird abpipetiert. Die verbleibende organische Phase (OP2) wird aufgeteilt und die resultierenden Fraktionen mit den folgenden Volumenanteilen und Konzentrationen der erfindungsgemäßen Substanzen versehen:

A) Kaliumcarbonat, B) Natriumdihydrogencarbonat, C) Gemisch aus Natrium- und Kalium-Metasilikat, D) Calziumactetat, E) Aluminiumacetat-Tartrat, F) Natriumborat, mit den Konzentrationen 3%, 10% und 15% und einem Volumenzusatz von 2%, 4% bzw. 8%. Zu je 100 ml der organischen Phasen (OP2) werden die erfindungsgemäßen Lösungen hinzugegeben und mit einem Ultrathurrax bei 24000 Umdrehungen pro Minute für 30 Sekunden homogenisiert. Nach einer Standzeit von 3 Minuten erfolgt die Phasenseparation bei 4000 Umdrehungen pro Minute über eine Dauer von 10 Minuten. Anschließend wird der Überstand (organischen Phase, OP3) abgetrennt. Die zugehörige wässrige Phase (WP3) wird jeweils mit 50 ml n-Heptan intensiv gemischt, anschließend erfolgt eine Phasentrennung wie zuvor beschrieben. Die resultierende wässrige Phase (WP4) wird mit einem Vakuumverdampfer getrocknet und die Trockensubstanzmenge abgewogen. Von den Trockensubstanzen aus den Ansätzen mit einer 10%igen Volumenzugabe wurde für alle untersuchten Substanzen eine Analyse auf den Gehalt an Phosphor (Atomabsorptionsanalyse) und Stickstoff (Kjeldahlsche-Methode) vorgenommen. Von jedem Ansatz werden von der Trockensubstanz Dünnschichtchromatographien gemäß Beispiel 2 angefertigt.

Ergebnisse

Das Mischen der organischen Phasen (OP2) mit den erfindungsgemäßen wässrigen Lösungen A)-F) führte zu einer erheblichen Emulsionsbildung. Durch Zentrifugation konnte eine Phasenseparation bewirkt werden unter Erhalt einer cremefarbenen halbfesten (bei 2 Vol-%) bis dickflüssigen (bei 15 Vol-%) wässrigen Phase (WP3) und einer leicht bis mäßig trüben organischen Phase (OP3). Nach Extraktion von Di- und Triacylglyceriden mittels n-Heptan aus WP3 und Vakuumtrocknung verbleibt eine bräunliche klebrige hochvisköse Masse. In dieser befinden sich nur geringe Mengen an stickstoffhaltigen Verbindungen (z.B. Sphingolipide oder Proteine) oder phosphathaltigen Verbindungen (z.B. Phospholipide). Die dünnschichtchromatographische Analyse zeigte bei allen Proben Banden, die Digalactosyl- und Monogalactosyldiglyceriden sowie Sterylglycosiden entsprachen. Somit konnte gezeigt werden, dass eine selektive Abtrennung von Glycoglycerolipiden und Glycosphingolipiden aus einer lipoiden Phase einer Phytoextraktion mit dem erfindungsgemäßen Verfahren möglich ist.

**Beispiel 4**

Reiskleie (auch *Rice bran*) aus einem Standardprozess der Reisverarbeitung mit einem Ölgehalt von 18% und einer Feuchtigkeit von 35% wurde nach der Gewinnung bis zur Lipidextraktion bei -8°C gelagert. Dieses Ausgangsmaterial wurde mit 10% Wasser gemischt und bei 30°C für 2 Stunden gerührt. Das Gemisch wurde mit n-Hexan bei 50°C 2-mal extrahiert. Die wässrige Phase (WP1) wurde mittels eines Vakuumverdampfers zu einer hochviskösen Masse eingeengt. Jeweils 100 g hiervon wurden mit 300 ml eines Gemisches aus Chloroform und Aceton (80:20) gemischt und die organische Phase (OP2) mittels zentrifugaler Phasenseparation abgetrennt. Je 150 ml der organischen Phase (OP2) wurden je nach Versuchsdurchführung mit jeweils 20 ml A) Natriumcarbonat, B) Natriumorthosilikat, C) Kupferacetat ($Cu(OAc)_2$), D) Kaliumtartrat oder E) Kaliumborat, die jeweils als 15%ige Lösungen vorlagen, versetzt und mit einem Ultrathurrax für 20 s homogenisiert. Nach einer Standzeit von 60 Sekunden erfolgte die Phasenseparation in einer Zentrifuge bei 5000 Umdrehungen pro Minute für 10 Minuten. Die jeweilige organische Phase (OP3) wurde abgetrennt und die jeweilige wässrige Phase (WP3), die eine hochvisköse bis halbfeste Konsistenz aufwies, homogenisiert. Von der homogenisierten wässrigen Phase (WP3) wurde je 1 ml als Probe abgetrennt, der Rest wurde mittels Vakuumverdampfer eingetrocknet und die zurückbleibende Substanzmenge anschließend gewogen. Die abgetrennte Probe wurde mit in Methanol gelöstem Natriummethoxylat hydrolysiert und anschließend mittels Silicagel-Chromatographie fraktioniert. Die Glycosylceramid-Fraktion wurde in Pyridin gelöst und MTPA-Cl (a-Methoxy-$\alpha$-trifluormethylphenylessigsäurechlorid) wurde bei 0°C hinzugegeben. Die Lösung wurde über 24 Stunden bei Raumtemperatur gerührt und konzentriert. Nach erneuter Aufreinigung mittels Silicagel-Säulenchromatographie mit Hexan/Ethylacetat (1:1) als Elutionsmittel wurde nach Abblasen des Elutionsmittels ein weißer Feststoff erhalten. Mittels ESI-TOF-MS (Elektrospray-Flugzeit-Massenspektrometrie) erfolgte der Nachweis der Zuckerester (m/z 1195.52 $[M+H]^+$). Die organischen Phasen (OP3) wurden vollständig eingedampft und die erhaltenen Feststoffe mit dem gleichen Verfahren wie zuvor beschrieben hydrolysiert und aufbereitet.

Ergebnisse:

Mit der wässrigen Extraktion von OP2 konnten Substanzmengen von A) 8,4g, B) 11,7g, C) 10,2g, D) 9,9g, E) 10,1g abgetrennt werden. In den abgetrennten Substanzen, die in der aus OP2 extrahierten WP3 enthalten waren, können nach Hydrolyse Zuckerverbindungen nachgewiesen werden, so dass von dem Vorliegen verschiedener zuckerhaltiger Lipidverbindungen (Glycoglycerolipide, Glycosphingolipide) ausgegangen werden kann. In den organischen Phasen (OP3) konnten praktisch keine Zuckerverbindungen nach der Hydrolyse nachgewiesen werden, so dass die Abtrennung zuckerhaltiger Verbindungen durch die wässrige Extraktion mit dem erfindungsgemäßen Verfahren weitgehend vollständig erfolgt.

**Beispiel 5:**

Untersuchung zur Extraktion und Gewinnung von Glycoglycerolipide und Glycosphingolipide aus lipoiden Pflanzenextrakten und zu deren Verwendung als Backhilfsstoff.

Kalt gepresstes Fruchtfleisch von Kernen der Acrocomia Plame mit einem Ölanteil von ca. 70% wird mit einem Gemisch aus Acteon, Dichlormethan und Hexan (Verhältnis 1:1:5) 1:1 verdünnt und gut mechanisch gemischt. Hiernach erfolgen zwei Extraktionsschritte mit einer wässrigen 0,4 molaren Arginin-Lösung bei einem Volumenzusatz von je 4%. Es folgt die Phasenseparation mittels Zentrifugation. Die lipoiden Phasen werden zunächst zweimal mit einer 10%igen Natriumcarbonat-Lösung mit einem Volumenzusatz von 4% vesetzt und gemischt, wobei der erste Eintrag mit einem Rührwerk bei 500 Umdrehungen pro Minute über 15 Minuten erfolgt und der zweite Eintrag einer wässrigen Lösung mit einer identischen Konzentration und Volumenanteil mit einem Ultrathurrax bei 18000 Umdrehungen pro Minute über 5 Minuten erfolgt. Die Phasenseparation erfolgt jeweils durch Zentrifugation mit 5000 Umdrehungen pro Minute über 15 Minuten. Die wässrigen Phasen der beiden subsequenten Abtrennungen bestehen jeweils aus einer viskösen weißen Emulsion. Die Emulsionen werden vereint und dann gefriergetrocknet. Anschließend wird die gefiergetrocknete Masse in einem Gemisch aus Chloroform und Methanol (5:1) gelöst, gefolgt von einer präparativen Säulenchromatographie (Kieslgelmatrix 60). Es folgt die Elution mit Aceton/Chloroform (5:1). Das Eluat wird mittels Vakuumverdampfer eingetrocknet. Es wird eine Trockenmasse von 56g bei einer Ausgangsmenge von 800g erhalten.
Mit der so gewonnenen Fraktion enthaltend Glycoglycerolipide und Glycosphingolipide werden Backversuche im Kleinformat nach einer Standardvorschrift vorgenommen: Anteigen von 10 g Mehl, 7% Frischhefe, 2% NaCL, 1 % Saccharose, 0,002% Ascorbinsäure, Wasser, in dem jeweils 30mg der Masse enthaltend Glycoglycerolipide und Glycosphingolipide gelöst wurden, mit 1200 Umdrehungen pro Minute bei 20°C für 1 Minute. Dann 40 Minuten Garen bei 30°C. Danach Backen bei 185°C für 10 Minuten. Anschließend Bestimmung des Volumens des Backgutes.

Zum Vergleich erfolgten Backversuche unter identischen Bedingungen ohne Zusatz der der Masse enthaltend Glycoglycerolipide und Glycosphingolipide und dafür mit 0,3% von Reinlecithinpulver (Jean-Puetz, Deutschland).

Ergebnisse:

Der Presssaft des Palmkernfruchtfleischs von Palmen der Gattung Acrocomia stellt eine lipoide Phase dar, in der sich ein hoher Anteil an Glycoglycerolipiden und Glycosphingolipiden, Wachsen, freien Fettsäuren und Faserstoffen befindet. Durch einen Wassereintrag kommt es zu einer stabilen Emulsion, die sich durch physikalische Maßnahmen nicht brechen lässt. Es hat sich gezeigt, dass die Abtrennung der freien Fettsäuren bei gleichzeitiger Lösungsvermittlung anderer lipoider Stoffe in organischen Lösungsmitteln mittels einer wässrigen Argininlösung möglich ist und dass sich anschließend eine Fraktion enthaltend Glycoglycerolipide und Glycosphingolipide durch die erfindungsgemäße wässrige Extraktion abtrennen lässt. Dabei kam es beim ersten Eintrag der Lösung mit einem Intensivmischer zu einer starken Emulsionsbildung, die zu einem schlechten Trennergebnis führte. Erfolgte der erfindungsgemäße Eintrag der wässrigen Lösung durch einen Rührvorgang mit einem Propellermischer, wodurch ein Lufteintrag vermieden wurde, so kam es zwar auch zu einer deutlichen Aggregatbildung, eine Phasenseparation war allerdings möglich. Nach der bereits erfolgten Abreicherung der lipoiden Phase kam es bei der Wiederholung des erfindungsgemäßen Eintrages der Salzlösung unter Verwendung eines Intensivmischers zwar erneut zu einer Emulsion, die sich allerdings diesmal durch eine zentrifugale Phasentrennung in eine klare Öl-Phase und eine feststoffhaltige Wasserphase trennen ließ.. Aus den vereinigten Fraktionen der abgetrennten Wasserphasen konnten nach präparativer Aufreinigung ein Gemisch aus Glycoglycerolipide und Glycosphingolipide erhalten werden, das sich sehr leicht in Wasser lösen ließ. In den Backversuchen konnte eine deutliche Steigerung des Volumens des Backgutes gegenüber Backergebnissen ohne Zusatz der Masse enthaltend Glycoglycerolipide und Glycosphingolipide (+300%) und gegenüber einem Lecithinzusatz (+120%) beobachtet werden.

**Beispiel 6:** Untersuchungen des Einflusses von Prozessparametern auf die Extraktionseffizienz und Hydrolysestabilität von Fraktionen enthaltend Glycoglycerolipide und Glycosphingolipide

Leindotteröl, gewonnen mittels einer Schneckenpresse bei 50°C, wurde mit 3% deionisiertem Wasser für 1 Stunde bei 45% intensiv gerührt. Anschließend erfolgte die Abtrennung der Wasserphase mittels eines Separators. Jeweils 200ml des wasserentschleimten Öls (Öl1) wurden 4% einer wässrigen Lösung von Kaliumhydrogencarbonat, Natrium-Metasilikat-Anhydrat und Calziumacetat (jeweils 15%ig) zugesetzt und mit einem Ultrathurrax für 5 Minuten bei 25000 Umdrehungen pro Minute homogenisiert. Unmittelbar anschließend erfolgte eine Zentrifugation bei 5000 Umdrehungen pro Minute für 10 Minuten. Abtrennung der Ölphase (Öl2). Ölreste in der zugehörigen wässrigen Phase (WP1) werden durch Aufschichten von Hexan und anschließender erneuter Zentrifugation sowie Abziehen der organischen Phase entfernt. Die so erhaltene wässrige Phase (WP2) hat eine hochvisköse Konsistenz. Die WP2 wird aufgeschüttelt und in 2 gleiche Volumenanteile fraktioniert. Jeweils eine Volumenfraktion der WP2 wird einer Vakuumtrocknung unterzogen, anschließend wird das Trockengewicht bestimmt. An dem entschleimten Öl (Öl1) sowie an der daraus nach

wässriger Extraktion erhaltenen Ölphase (Öl2) wurden dünnschichtchromatographische Untersuchungen durchgeführt. Das entschleimte Öl (Öl1) zeigte deutliche und scharf begrenzte Banden, die Monogalactosldiglyceriden, Digalactosyldiglyceriden sowie Glycosphingolipiden entsprachen. In den Ölphasen (Öl2), die mit den o. g. wässrigen Lösungen behandelt wurden, waren praktisch keine Banden mehr sichtbar. Die zweite Volumenfraktion der WP2 wurden unmittelbar nach der Gewinnung mit einem Lösungsmittelgemisch (Chloroform/Methanol/Essigsäure, 90/8/2) intensiv ausgeschüttelt und die organischen Phasen (OP1) abgetrennt. Die Lösungsmittel der erhaltenen organischen Phasen wurden mittels Vakuumtrocknung entfernt und die Trockensubstanz, welche aus der organischen Phase (OP1) verbleibt, in dem Laufmittel für die anschließende Dünnschichtchromatographie gelöst. Die Chromatographieergebnisse wurden bezüglich des Nachweises von Banden für die o. g. Glycoglycerolipide und Glycosphingolipide vorgenommen sowie die Breite der Banden analysiert. Die jeweils 100ml der Öl-Phase 2 wurde mit dem Ultrathurrax zusammen mit der auch zuvor verwendeten Salzlösung für 5 Minuten wie oben beschrieben homogenisiert. Anschließend erfolgen wie zuvor beschrieben eine Abtrennung der Wasserphase sowie die Bestimmung der Trockensubstanzmenge der Wasserphasen.

Es wurden unterschiedliche Versuchsabwandlungen an je 100ml Öl mit erfindungsgemäßen wässrigen Lösungen der o. g. Salze durchgeführt. Dabei wurden die Prozesstemperaturen (35°, 55° und 75°C) und die Intensität der Mischung nach Zugabe der erfindungsgemäßen wässrigen Lösungen der o. g. Salze variiert. Ferner wurden Mischer mit unterschiedlicher Intensität des Mischeintrages verwendet: A) Ultrathurrax 25000 Umdrehungen pro Minute, B) Propellermischer 2500 Umdrehungen pro Minute, C) Ultraschall. Die Mischvorgänge erfolgten über 5 Minuten unter kontinuierlicher Temperaturkontrolle.

In einer weiteren Versuchsserie wurden die Untersuchungen mit den Mischern B) und C) für eine Behandlungsdauer von 10 und 20 Minuten wiederholt.

<u>Ergebnisse:</u>

Mit dem in Voruntersuchungen etablierten Intensiveintrag der wässrigen Lösungen mit den offenbarten Substanzen konnte in den entsprechend erfolgten Referenzuntersuchungen gezeigt werden, dass hierdurch eine fast vollständige Abtrennung von Glycoglycerolipiden und Glycosphingolipiden ermöglicht wird. Darüber hinaus zeigte sich bei der Dünnschichtchromatographie der aus den wässrigen Phasen (WP2) gewonnenen Fraktionen, dass die hierin enthaltenen Banden der Glycoglycerolipide und Glycosphingolipide scharf begrenzt waren, also dass keine relevante Hydrolyse stattgefunden hat.

Die abgetrennten Trockensubstanzmengen, die nach einem Mischvorgang mit einem Propellermischer erhalten wurden, waren deutlich geringer als die, die durch eine Intensivmischung erhalten wurden. Übereinstimmend fanden sich im so behandelten Öl auch noch deutlich erkennbare Mengen an Glycoglycerolipiden und Glycosphingolipiden. Durch die erneute Behandlung, bei der der Eintrag der wässrigen Lösungen mit einem Intensivmischer erfolgte, konnten Trockensubstanzmengen abgetrennt werden, die in der Summe mit der zuvor erfolgten Abtrennung ungefähr auf die Trockensubstanzmenge kam, die mit dem Intensivmischer abgetrennt werden konnte.

Die Banden der Glycoglycerolipide und Glycosphingolipide in der chromatographischen Auftrennung der aus der wässrigen Abtrennung mittels der oben genannten Salzlösungen erhaltenen Substanzen waren bei allen Abtrennungen vorhanden und zeigten bei einer Behandlungsdauer von 5 Minuten keine Verbreiterung, so dass keine relevante Hydrolyse vorlag. Durch eine Verlängerung des Mischprozesses mit dem Propellermischer konnte die Menge an extrahierbarem Feststoff bis auf die Menge, die bei einer Intensivmischung abtrennbar war, gesteigert werden. Während es bei einer Mischtemperatur von 35°C nur bei einer Behandlungsdauer von mehr als 10 Minuten zu einem geringen Anzeichen für eine Hydrolyse der Glycoglycerolipide und Glycosphingolipide kam, waren hydrolysebedingte Verbreiterungen der Banden der Glycoglycerolipide und Glycosphingolipide bei Verwendung höherer Behandlungstemperaturen bereits nach 5 Minuten zu sehen und nahmen deutlich zu, wenn bei diesen Temperaturen länger gemischt wurde.

**Beispiel 7**

Trauben-Pressrückstände wurden in einem Fermentierbehälter unter kontinuierlichen Perkolationsbedingungen und Zusatz von Carbonsäuren mikrobiell zersetzt. Nach 7 Tagen wurde eine Probe von 1 Liter entnommen und mit 1 Liter Biodiesel (C8 bis C18-Methylester) intensiv gemischt. Das Gemisch wurde zentrifugiert und die stark trübe organische Phase (OP1) abgetrennt. Je 100 ml der organischen Phase (OP1) wurden mit 5ml Magnesiumhydrogencarbonat oder Kaliumacetat (jeweils 10%ige wässrige Lösung) bei 30°C mit einem Propellermischer bei 1000 Umdrehungen pro Minute für 7 Minuten gemischt. Danach erfolgte eine Zentrifugation mit 4000 Umdrehungen pro Minute für 10 Minuten. Die resultierende organische Phase (OP2) ist anschließend nur noch leicht trüb, die wässrige Phase (WP2) besteht aus einer cremefarbenen halbfesten Masse. Nach sorgfältigem Dekantieren der organischen Phase (OP2) wird die wässrige Phase (WP2) in 100ml Chloroform gegeben und gemischt (OP3). Dann Zugabe von 2ml 0,1 molarem HCl (in deionisiertem Wasser) zu WP2 und intensives Mischen. Dann Zentrifugation und abpipettieren der wässrigen Phase (WP3). Hiernach Zugabe von 2ml eine Methanol.-Wasser-Gemisch (80:20) Mischen und Zentrifugieren des Gemisches.

Nach abpipettieren der leicht trüben Methanolphase wird das Lösungsmittel der erhaltenen organischen Phase (OP4) mittels Vakuumverdampfer entfernt und die Trockensubstanz gewogen. Es werden 5µg hiervon separiert und mit einem Gemisch aus Chloroform und Methanol (90:10) gelöst. Anschließend Auftragung auf einer Dünnschichtchromatographieplatte (Macherey Nagel, Germany) und Entwicklung unter Verwendung eines Gemischs aus Chloroform:Methanol: Wasser (70:28:2) als Laufmittel.

Entwicklung und Färbung der Platten mit Anisaldehyd-Reagenz (Sigma, Deutschland) bei 200°C. Als Referenz wurden Konzentrate aus pflanzlichen Mono- und Diglycoglycerolipide sowie XX separat aufgetragen. Die aufgetrennten zuckerhaltigen Verbindungen können nach der erfolgten Farbreaktion eingeordnet werden: Glycoglycerolipide - grün/blaugrün, Glycosphingolipide - blau, Glycerophospholipide - grau oder violett, hydrolysierte Glycoglycerophospholipide - intensiv rot oder violett.

Ergebnisse:

Aus einem lipoiden Stoffgemisch, das in eine organische Phase überführt wurde, konnten mittels des erfindungsgemäßen wässrigen Extraktionsverfahrens relevante Mengen der hierin gelösten Lipide in einer wässrigen Phase abgetrennt werden (mit Magnesiumcarbonat 8g und mit Kaliumactetat 7g). Mittels Dünnschichtchromatographie konnte das Vorhandensein von Ceramiden, Sphingolipiden und Glycosphingolipide sowie Glycoglycerolipide identifiziert werden, wobei für letztere sowohl Monoglycosylglycero- und Diglycosylglycerolipide nachgewiesen werden konnten. Es zeigten sich nur andeutungsweise Banden, die Glycophospholipiden entsprechen können.

Die CMC (kritische Mizellbildungskonzentration) betrug für beide Extrakte 55mg/l. Die ermittelte Oberflächenspannung betrug 28,1 mN/m.

**Beispiel 8: Einzelschritte**

Für die im Folgenden beschriebenen Schritte wurden je 200 kg lipoide Phase (kaltgepresstes Camelinaöl) verwendet.

**Beispiel 8A: Schritt A2') Behandlung mittels Zitronensäurelösung:**

Die lipoide Phase im Vorlagetank wird auf 60°C erwärmt und anschließend mit 0,1 Gew.-% Zitronensäure (33%ig, auf Raumtemperatur) versetzt und für 30 Sekunden intensiv gerührt und danach 10 min bei cirka 100 bis 150 UpM gerührt. Anschließend wird 0,3 Gew.-% Wasser hinzugegeben.

Das Gemisch aus lipoider Phase und verdünnter Zitronensäure wird anschließend in den Trenn-Separator gepumpt und so bei einer Leistung von 200l/h die wässrige Phase von der öligen Phase getrennt. Die ölige Phase wird zur weiteren Prozessierung in einen weiteren Vorlagetank (Vorlagetank 2) überführt.

**Beispiel 8B: Schritt A2) Behandlung mittels Wasser:**

Die lipoide Phase im Vorlagetank wird auf 65°C erwärmt und anschließend mit 3 Gew.-% Wasser (auf Raumtemperatur) versetzt und für 30 Sekunden intensiv gerührt und danach 10 min bei cirka 100 bis 150 UpM gerührt.

Das Gemisch aus lipoider Phase und Wasser wird anschließend in den Trenn-Separator gepumpt und so bei einer Leistung von 200l/h die wässrige Phase von der öligen Phase getrennt. Die ölige Phase wird zur weiteren Prozessierung in einen weiteren Vorlagetank (Vorlagetank 2) überführt.

**Beispiel 8C: Schritt B1) Behandlung mittels Natriumhydrogencarbonat- / Natrium-acetat-Lösung:**

Die lipoide Phase wird auf eine Prozesstemperatur von 45°C bis 50°C gebracht und ein ausreichendes Volumen an 8%iger Natriumhydrogencarbonat-Lösung / Natriumacetat-Lösung hinzugegeben. Anschließend wird eine Fraktion (A)) mittels eines Ystral-Mischers für 30 Sekunden intensiv gerührt und danach 10 min normal gerührt. Eine zweite Fraktion wird mit dem erfindungsgemäßen Intensivmischer (B)) für 2 Minuten homogenisiert.

Das Gemisch aus A) wird anschließend in einen standardmäßigen Trenn-Separator gepumpt und so bei einer Leistung von 200l/h die wässrige Phase von der öligen Phase getrennt. Die ölige Phase wird zur weiteren Prozessierung in einen Vorlagetank überführt.

Das Gemisch aus B) wird anschließend in den Trenn-Separator gemäß Figur 3 gepumpt und so bei einer Leistung von 200l/h die wässrige Phase von der öligen Phase getrennt. Die ölige Phase wird zur weiteren Prozessierung in den Vorlagetank (Vorlagetank 1) überführt.

**Beispiel 8D: Schritt E1) Behandlung mittels Arginin-Lösung**

Die lipoide Phase wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt. Anschließend wird für 10 min schonend gerührt.

Das Gemisch wird anschließend in den Trenn-Separator gepumpt und so bei einer Leistung von 200l/h die wässrige Phase von der öligen Phase getrennt. Die ölige Phase wird gesammelt.

Ergebnisse:

Die öligen Phasen wiesen nach den Verfahrensschritten A2 eine deutliche und nach A2') eine leichte Trübung auf und hatte in der chemischen Analyse Werte für: FFA 0,31%, $H_2O$-Gehalt 0,55 %, Eisen-Gehalt 0,23 ppm, Phosphor-Gehalt 34 ppm nach A2) und für FFA 0,42%, $H_2O$-Gehalt 0,30 %, Eisen 0,15 ppm, Phosphor 19,6 ppm nach A2').
Durch die Intensivmischung mit einem Standard-Mischer im Rahmen des Verfahrensschritts B1) kam es zu einer deulichen Emulsionsbildung, die mit einer Steigerung der Viskosität verbunden war. Eine Einleitung in einen Standard-Trennseparator konnte nur durch eine Erhöhung der Temperatur im Vorlagebehälter auf 60°C ermöglicht werden. Die mit dem Standard-Trennseparator erhaltene wässrige Phase hat eine leicht gelbliche Farbe und eine cremeartige Beschaffenheit.
Die Bestimmung der Trockensubstanzmengen der wässrigen Phasen ergaben Werte von von 2,3kg für die Abtrennung nach Verfahrensschritt A2) und 2,1 kg für die Abtrennung nach Verfahrensschritt A2'). Dabei zeigte sich dass ca. 20% des wasserfreien Rückstands Triacylglyceriden entsprach. Die öligen Phasen waren deutlich trüb.

Mit dem offenbarten Intensivmischer gemäß Figur 3 der im Rahmen des Verfahrensschritts B1) eingesetzt wurde, kam es zu praktisch keiner Emulsionsbildung der lipoiden Phasen. Die Einleitung der so lufteintragsfrei gemischten lipoiden Phase in den Separator war auch unter Niedrigtemperaturbedingungen problemlos möglich. Die durch den Separator erhaltenen wässrigen Phasen hatten eine weißliche Farbe und eine milchige Beschaffenheit.
Die Bestimmung der Trockensubstanzmengen der wässrigen Phasen ergaben Werte von von 2,1 kg für die Abtrennung nach Verfahrensschritt A2) und 2,0 kg für die Abtrennung nach Verfahrensschritt A2'). Die öligen Phasen waren diskret trüb.
In der chemischen Analyse werden folgende Werte bestimmt:

B1) mit Standard-Mischer/Separator: FFA 0,21%, $H_2O$-Gehalt 0,65%, Eisen-Gehalt 0,15 ppm, Phosphor-Gehalt 20 ppm nach A2) und FFA 0,22%, $H_2O$-Gehalt 0,52%, Eisen 0,1 ppm, Phosphor 15 ppm nach A2');
B1) mit Mischer/Separator gemäß Figur 3: FFA 0,20%, $H_2O$-Gehalt 0,35 %, Eisen-Gehalt 0,14 ppm, Phosphor-Gehalt 16 ppm nach A2) und FFA 0,18%, $H_2O$-Gehalt 0,28%, Eisen 0,1 ppm, Phosphor 12 ppm nach A2').

Mit der lipoiden Phase, die aus dem Verfahren B1) mit dem Mischer/Separatorsystem gemäß Figur 3 erhalten wurde, erfolgte der Verfahrensschritt E1). Die Einmischung der wässrigen Lösung war ohne relevante Emulsionsbildung möglich. Die so behandelten lipoiden Phasen konnten problemlos mit einem konventionellen Separator zu klaren Ölphasen und trüben Wasserphasen getrennt werden.

In der chemischen Analyse werden folgende Werte bestimmt:

Lipoide Phase aus Verfahrensschritt A2): FFA 0,13%, $H_2O$-Gehalt 0,25%, Eisen-Gehalt 0,1 ppm, Phosphor-Gehalt 5 ppm;
Lipoide Phase aus Verfahrensschritt A2'): FFA 0,12%, $H_2O$-Gehalt 0,20%, Eisen 0,1 ppm, Phosphor 3 ppm.

Alle hierin gemachten Prozentangaben (%) beziehen sich auf Gewichtsprozente (Gew.-%) sofern nichts anderes bei der jeweiligen Angabe ausgeführt ist.

**Beispiel 9: Zweistufige Verfahren**

**Beispiel 9A: Schritte B1) und E1)**

130 kg Rapsöl (FFA-Gehalt 1,40%, $H_2O$-Gehalt 0,17%, Eisen-Gehalt 0,44 ppm, Phosphor-Gehalt 65,0 ppm) werden in den Vorlagetank (Vorlagetank 1) eingefüllt.

Anschließend wird das Rohöl im Vorlagetank 1 auf eine Prozesstemperatur von 45°C gebracht und mit 3,9 kg an 10%iger Natriummetasilicat-Lösung versetzt. Anschließend wird mittels Ystral-Mischer für 30 Sekunden intensiv gerührt und danach 10 min normal gerührt.

Das resultierende Gemisch wird anschließend in den Trenn-Separator gepumpt und so bei einer Leistung von

200l/h die wässrige Phase A von der öligen Phase A getrennt. Die wässrige Phase A wird gesammelt und bis zur weiteren Verwendung gelagert. Die ölige Phase A wird zur weiteren Prozessierung wieder zurück in den Vorlagetank 1 überführt. 125 ml der öligen Phase A wurden zur chemischen Analyse verwendet (FFA-Gehalt 0,10%, $H_2O$-Gehalt 0,15 %).

Die ölige Phase A wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt und es bei der Zugabe nicht zu einem Eintrag von Luft kommt. Anschließend wird für 10 min schonend gerührt. Das resultierende Gemisch wird anschließend in den Trenn-Separator gepumpt und so bei einer Leistung von 200l/h die wässrige Phase B von der öligen Phase B getrennt. Die wässrige Phase B und die ölige Phase B werden getrennt gesammelt. 125 ml der öligen Phase B wurden zur chemischen Analyse verwendet (FFA-Gehalt 0,1%, $H_2O$-Gehalt 0,2 %).

**Beispiel 9B: Schritte B1) und E1)**

200 bis 350 kg Rapsöl (FFA-Gehalt 0,42%, $H_2O$-Gehalt 0,03 %, Eisen-Gehalt 0,42 ppm, Phosphor-Gehalt 66,6 ppm) werden in den Vorlagetank (Vorlagetank 1) eingefüllt.

Das Rohöl wird auf eine Prozesstemperatur von 45°C bis 50°C gebracht und ein ausreichendes Volumen an 8%iger Natriumhydrogencarbonat-Lösung hinzugegeben. Anschließend wird mittels Ystral-Mischer für 30 Sekunden intensiv gerührt und danach 10 min normal gerührt. Das resultierende Gemisch wird anschließend in einen Trenn-Separator gepumpt und so bei einer Leistung von 200l/h die wässrige Phase A von der öligen Phase A getrennt. Die wässrige Phase A wird gesammelt und bis zur weiteren Verwendung gelagert. Die ölige Phase A wird zur weiteren Prozessierung wieder zurück in den Vorlagetank 1 überführt. 125 ml der öligen Phase A wurden zur chemischen Analyse verwendet (FFA-Gehalt 0,31 %, $H_2O$-Gehalt 0,30 %, Eisen-Gehalt 0,15 ppm, Phosphor-Gehalt 19,6 ppm).

Die ölige Phase A wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt und es bei der Zugabe nicht zu einem Eintrag von Luft kommt. Anschließend wird für 10 min schonend gerührt. Das resultierende Gemisch wird anschließend in den Trenn-Separator gepumpt und so bei einer Leistung von 200l/h die wässrige Phase B von der öligen Phase B getrennt. Die wässrige Phase B und die ölige Phase C werden getrennt gesammelt. 125 ml der öligen Phase B wurden zur chemischen Analyse verwendet (FFA-Gehalt 0,13 %, $H_2O$-Gehalt 0,41 %, Eisen-Gehalt 0,09 ppm, Phosphor-Gehalt 12,8 ppm).

**Beispiel 9C: Schritte B1) und E1)**

200 bis 350 kg Rapsöl (FFA-Gehalt 0,42%, $H_2O$-Gehalt 0,01 %, Eisen-Gehalt 0,42 ppm, Phosphor-Gehalt 67,9 ppm) werden in den Vorlagetank (Vorlagetank 1) eingefüllt.

Das Rohöl wird auf eine Prozesstemperatur von 45°C bis 50°C gebracht und ein ausreichendes Volumen an 8%iger Natriumacetat-Lösung hinzugegeben, Anschließend wird mittels Ystral-Mischer für 30 Sekunden intensiv gerührt und danach 10 min normal gerührt. Das resultierende Gemisch wird anschließend in den Trenn-Separator gepumpt und so bei einer Leistung von 200l/h die wässrige Phase A von der öligen Phase A getrennt. Die wässrige Phase A wird gesammelt und bis zur weiteren Verwendung gelagert. Die ölige Phase A wird zur weiteren Prozessierung wieder zurück in den Vorlagetank 1 überführt. 125 ml der öligen Phase A wurden zur chemischen Analyse verwendet (FFA-Gehalt 0,42%, $H_2O$-Gehalt 0,55 %, Eisen-Gehalt 0,23 ppm, Phosphor-Gehalt 34 ppm).

Die ölige Phase A wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt und es bei der Zugabe nicht zu einem Eintrag von Luft kommt.

Anschließend wird für 10 min schonend gerührt. Das resultierende Gemisch wird anschließend in den Trenn-Separator gepumpt und so bei einer Leistung von 200l/h die wässrige Phase B von der öligen Phase B getrennt. Die wässrige Phase B und die ölige Phase B werden getrennt gesammelt. 125 ml der öligen Phase B wurden zur chemischen Analyse verwendet (FFA-Gehalt 0,16%, $H_2O$-Gehalt 0,45 %, Eisen-Gehalt 0,1 ppm, Phosphor-Gehalt 11,8 ppm).

**Beispiel 9D: Schritte B1) und E1)**

200 bis 350 kg Rapsöl (FFA-Gehalt 0,43%, $H_2O$-Gehalt 0,12 %, Eisen-Gehalt 1,15 ppm, Phosphor-Gehalt 57,4 ppm) werden in den Vorlagetank (Vorlagetank 1) eingefüllt.

Das Rohöl wird auf eine Prozesstemperatur von 45°C bis 50°C gebracht und ein ausreichendes Volumen an 8%iger Natriumcarbonat-Lösung hinzugegeben, Anschließend wird mittels Ystral-Mischer für 30 Sekunden intensiv gerührt und danach 10 min normal gerührt. Das resultierende Gemisch wird anschließend in den Trenn-Separator gepumpt und so bei einer Leistung von 200l/h die wässrige Phase A von der öligen Phase A getrennt. Die wässrige Phase A wird gesammelt und bis zur weiteren Verwendung gelagert. Die ölige Phase A wird zur weiteren Prozessierung wieder zurück in den Vorlagetank 1 überführt. 125 ml der öligen Phase A wurden zur chemischen Analyse verwendet

(FFA-Gehalt 0,26%, $H_2O$-Gehalt 0,25 %, Eisen-Gehalt 0,16 ppm, Phosphor-Gehalt 18,75 ppm).

Die ölige Phase A wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt und es bei der Zugabe nicht zu einem Eintrag von Luft kommt. Anschließend wird für 10 min schonend gerührt. Das resultierende Gemisch wird anschließend in den Trenn-Separator gepumpt und so bei einer Leistung von 200l/h die wässrige Phase B von der öligen Phase B getrennt. Die wässrige Phase B und die ölige Phase B werden getrennt gesammelt. 125 ml der öligen Phase B wurden zur chemischen Analyse verwendet (FFA-Gehalt 0,11%, $H_2O$-Gehalt 0,32 %, Eisen-Gehalt 0,11 ppm, Phosphor-Gehalt 9,0 ppm).

Die folgende Tabelle **3** beschreibt Art und Aussehen der reduzierten lipoiden Phase als auch der wässrigen Phase nach der Trennung mittels Zentrifugation nach Schritt B1) und vor Schritt E1) bei einer Versuchsreihe mit Rapsöl (FFA-Gehalt 0,43%, $H_2O$-Gehalt 0,12 %, Eisen-Gehalt 1,15 ppm, Phosphor-Gehalt 57,4 ppm) gemäß obiger Vorschriften:

| Reagenz | Konz. | Zugabemenge | Schleudertest 1min./20°C | | | |
|---|---|---|---|---|---|---|
| | | | Ölphase | | | Vol.% schwere Phase |
| | | | Wasser-% | FFA-% | Aussehen | |
| MS | 10%ig | 0,5% | 0,28 | 0,65 | trüb | 6 (braun) |
| | | 1,0%* | 0,12 | 0,21 | blank | 10 (braun) |
| pH=13 | | 3,0%* | 0,07 | 0,07 | l.trüb | 10 (nougat-braun) |
| | | 5,0% | 0,18 | 0,04 | trüb | 9 (nougat-braun) |
| | | | | | | |
| NC | 10%ig | 0,5% | 0,24 | 0,56 | trüb | 7(braun) |
| | | 1,0%* | 0,14 | 0,36 | blank | 6 (braun) |
| pH=11 | | 3,0%* | 0,16 | 0,07 | l.trüb | 8 (hell-braun) |
| | | 5,0% | 0,18 | 0,05 | trüb | 9 (hell-braun) |
| | | | | | | |
| NAc | 10%ig | 0,5% | 0,39 | 0,96 | trüb | 0,3 (braun) |
| | | 1,0% | 0,12 | 0,60 | blank | 13 (braun) |
| pH=8,1 | | 3,0%* | 0,15 | 0,59 | blank | 6,5 (hell braun) |
| | | 5,0% | 0,15 | 0,59 | f.blank | 7,5 (hell braun) |
| | | | | | | |
| NHC | 8%ig | 0,5% | 0,09 | 0,65 | fast blank | 15/davon 0,6 Wasser (braun) |
| | | 1,0% | 0,09 | 0,62 | fast blank | 15/davon 0,6 Wasser (braun) |
| pH=8,1 | | 3,0%* | 0,11 | 0,58 | blank | 8,5/davon 0,6Wasser (braun) |
| | | 5,0% | 0,11 | 0,62 | blank | 7,5/davon 2,5Wasser (braun) |
| MS: Na-Metasilicat f. fast<br>NC: Natriumcarbonat l. leicht<br>NAc: Natriumacetat FFA: freie Fettsäuren<br>NHC: Natriumhydrogencarbonat | | | | | | |

**Beispiel 10: Dreistufige Verfahren**

**Beispiel 10A: Schritte A2) und B1) und E1)**

130 kg Rapsöl (FFA-Gehalt 1,40%, $H_2O$-Gehalt 0,17%, Eisen-Gehalt 0,44 ppm, Phosphor-Gehalt 65,0 ppm) werden in den Vorlagetank (Vorlagetank 1) eingefüllt.

Anschließend wird die lipoide Phase im Vorlagetank 1 auf 50°C bis 55°C erwärmt und anschließend mit 6 kg Wasser versetzt und für 30 Sekunden intensiv gerührt und danach 10 min bei cirka 100 bis 150 UpM gerührt. Das

Gemisch aus lipoider Phase und Wasser wird anschließend in den Trenn-Separator gepumpt und so bei einer Leistung von 200l/h die wässrige Phase A von der öligen Phase A getrennt. Die wässrige Phase A wird gesammelt und bis zur weiteren Verwendung gelagert. Die ölige Phase A wird zur weiteren Prozessierung in einen weiteren Vorlagetank (Vorlagetank 2) überführt. 125 ml der öligen Phase A wurden zur chemischen Analyse verwendet (FFA-Gehalt 1,05%, $H_2O$-Gehalt 0,18 %).

49 kg der so gewonnenen öligen Phase A werden auf eine Prozesstemperatur von 40°C bis 45°C gebracht und mit 1,5 kg an 10%iger Natriummetasilicat-Lösung hinzugegeben. Anschließend wird mittels Ystral-Mischer für 30 Sekunden lufteintragsfrei intensiv gerührt und danach 10 min. normal lufteintragsfrei gerührt.

Das resultierende Gemisch wird anschließend in den Trenn-Separator gepumpt und so bei einer Leistung von 200l/h die wässrige Phase B von der öligen Phase B getrennt. Die wässrige Phase B wird gesammelt und zur Extraktion der abgetrennten Glycoglycerolipide verwendet. Durch Extraktion mittels Chloroform wurden die Glycoglycerolipide aus der wäßrigen Phase B gewonnen. 125 ml der öligen Phase B wurden zur chemischen Analyse verwendet (FFA-Gehalt 0,13%, $H_2O$-Gehalt 0,2 %).

Die ölige Phase B wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt und es bei der Zugabe nicht zu einem Eintrag von Luft kommt. Anschließend wird für 10 min schonend gerührt. Das resultierende Gemisch wird anschließend in den Trenn-Separator gepumpt und so bei einer Leistung von 200l/h die wässrige Phase C von der öligen Phase C getrennt. Die wässrige Phase C und die ölige Phase C werden getrennt gesammelt. 125 ml der öligen Phase C wurden zur chemischen Analyse verwendet. Der Gehalt an freien Fettsäuren konnte auf 0,14 Gew.-% reduziert werden. Zudem wurde die Menge an Kalium, Phosphor, Eisen sowie Calcium auf weniger als 5 ppm gesenkt (K < 5 ppm, P < 5 ppm, Fe < 5 ppm, Ca < 5 ppm).

**Beispiel 10B: Schritte A2') und B1) und E1)**

Ca. 200 kg Rapsöl (FFA-Gehalt 0,5%, $H_2O$-Gehalt 0,04 %, Eisen-Gehalt 0,63 ppm, Phosphor-Gehalt 74,8 ppm) werden in den Vorlagetank (Vorlagetank 1) eingefüllt.

Anschließend wird die lipoide Phase im Vorlagetank 1 auf 40°C bis 60°C erwärmt und anschließend mit 0,1 Gew.-% Zitronensäure (33%ig, auf Raumtemperatur) versetzt und für 30 Sekunden intensiv gerührt und danach 10 min bei cirka 100 bis 150 UpM gerührt. Danach wird 0,3 Gew.-% Wasser hinzugegeben.

Das Gemisch aus lipoider Phase und verdünnter Zitronensäure wird anschließend in den Trenn-Separator gepumpt und dann bei einer Leistung von 200l/h die wässrige Phase A von der öligen Phase A getrennt. Die wässrige Phase A wird gesammelt und bis zur weiteren Verwendung gelagert. Die ölige Phase A wird zur weiteren Prozessierung in einen weiteren Vorlagetank (Vorlagetank 2) überführt. 125 ml der öligen Phase A wurden zur chemischen Analyse verwendet (FFA-Gehalt 0,48%, $H_2O$-Gehalt 0,33 %, Eisen-Gehalt 0,13 ppm, Phosphor-Gehalt 15,9 ppm).

Die so gewonnene ölige Phase A wird auf eine Prozesstemperatur von 40°C bis 45°C gebracht und ein ausreichendes Volumen an 8%iger Natriumhydrogencarbonat-Lösung hinzugegeben, so dass ein theoretische Neutralisationsgrad der freien Fettsäuren von 90% erreicht wird. Anschließend wird mittels Ystral-Mischer für 30 Sekunden intensiv aber ohne Eintrag von Luft, d.h. ohne Gaseintrag gerührt und danach 10 min normal aber weiterhin ohne Eintrag von Luft, d.h. ohne Gaseintrag gerührt. Das resultierende Gemisch wird anschließend in den Trenn-Separator gepumpt und so bei einer Leistung von 200l/h die wässrige Phase B von der öligen Phase B getrennt.

Die wässrige Phase B wird gesammelt. In dieser wurden Sterylglycosiden mittels DC nachgewiesen. Die ölige Phase B wird zur weiteren Prozessierung wieder zurück in den Vorlagetank 1 überführt. 125 ml der öligen Phase B wurden zur chemischen Analyse verwendet (FFA-Gehalt 0,39 %, $H_2O$-Gehalt 0,41 %, Eisen-Gehalt 0,06 ppm, Phosphor-Gehalt 4,08 ppm).

Die ölige Phase B wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt und es bei der Zugabe nicht zu einem Eintrag von Luft kommt. Anschließend wird für 10 min schonend gerührt. Das resultierende Gemisch wird anschließend in den Trenn-Separator gepumpt und so bei einer Leistung von 200l/h die wässrige Phase C von der öligen Phase C getrennt. Die wässrige Phase C und die ölige Phase C werden getrennt gesammelt. 125 ml der öligen Phase C wurden zur chemischen Analyse verwendet. Der Gehalt an freien Fettsäuren konnte auf 0,15 Gew.-% reduziert werden. Zudem wurde die Menge an Kalium und Calcium auf unter 0,5 ppm gesenkt (K < 1 ppm; Ca < 1 ppm) und die Menge an Phosphor bis auf ,08 ppm und die Mengen an Eisen auf 0,02 ppm gesenkt.

**Beispiel 10C: Schritte A2') und B1) und E1)**

Ca. 250 kg Rapsöl (FFA-Gehalt 0,42%, $H_2O$-Gehalt 0,08%, Eisen-Gehalt 0,43 ppm, Phosphor-Gehalt 70 ppm) werden in den Vorlagetank (Vorlagetank 1) eingefüllt.

Anschließend wird die lipoide Phase im Vorlagetank 1 auf 50°C bis 55°C erwärmt und anschließend mit 0,1

Gew.-% Zitronensäure (33%ig, auf Raumtemperatur) versetzt und für 30 Sekunden intensiv gerührt und danach 10 min bei cirka 100 bis 150 UpM gerührt. Danach wird 0,3 Gew.-% Wasser hinzugegeben.

Das Gemisch aus lipoider Phase und verdünnter Zitronensäure wird anschließend in den Trenn-Separator gepumpt und dann bei einer Leistung von 200l/h die wässrige Phase A von der öligen Phase A getrennt. Die wässrige Phase A wird gesammelt und bis zur weiteren Verwendung gelagert. Die ölige Phase A wird zur weiteren Prozessierung in einen weiteren Vorlagetank (Vorlagetank 2) überführt. 125 ml der öligen Phase A wurden zur chemischen Analyse verwendet (FFA-Gehalt 0,4%, $H_2O$-Gehalt 0,30%, Eisen-Gehalt 0,13 ppm, Phosphor-Gehalt 17 ppm).

Die so gewonnene ölige Phase A wird auf eine Prozesstemperatur von 45°C bis 50°C gebracht und ein ausreichendes Volumen an 8%iger Natriumacetat-Lösung hinzugegeben, so dass ein theoretischer Neutralisationsgrad der freien Fettsäuren von 90% erreicht wird. Anschließend wird mittels Ystral-Mischer für 30 Sekunden intensiv und vorzugsweise gaseintragsfrei gerührt und danach 10 min normal und vorzugsweise gaseintragsfrei gerührt. Das resultierende Gemisch wird anschließend in den Trenn-Separator gepumpt und so bei einer Leistung von 200l/h die wässrige Phase B von der öligen Phase B getrennt.
Die wässrige Phase B wurden Sterylglycoside mittels DC nachgewiesen. Die ölige Phase B wird zur weiteren Prozessierung wieder zurück in den Vorlagetank 1 überführt. 125 ml der öligen Phase B wurden zur chemischen Analyse verwendet (FFA-Gehalt 0,37 %, $H_2O$-Gehalt 0,40, Eisen-Gehalt 0,07 ppm, Phosphor-Gehalt 6 ppm).

Die ölige Phase B wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt und es bei der Zugabe nicht zu einem Eintrag von Luft kommt. Anschließend wird für 10 min schonend und ohne Eintrag von Lauf gerührt. Das resultierende Gemisch wird anschließend in den Trenn-Separator gepumpt und so bei einer Leistung von 200l/h die wässrige Phase C von der öligen Phase C getrennt. Die wässrige Phase C und die ölige Phase C werden getrennt gesammelt. 125 ml der öligen Phase C wurden zur chemischen Analyse verwendet. Der Gehalt an freien Fettsäuren konnte auf 0,12 Gew.-% reduziert werden. Zudem wurde die Menge an Kalium und Calcium auf unter 5 ppm gesenkt ($K < 5$ ppm; $Ca < 5$ ppm) und die Menge an Phosphor bis auf 1,1 ppm und die Mengen an Eisen auf 0,05 ppm gesenkt.

**Beispiel 10D: Schritte A2') und B1) und E1)**

Ca. 300 kg Rapsöl (FFA-Gehalt 0,47%, $H_2O$-Gehalt 0,04 %, Eisen-Gehalt 0,53 ppm, Phosphor-Gehalt 85,1 ppm) werden in den Vorlagetank (Vorlagetank 1) eingefüllt.

Anschließend wird die lipoide Phase im Vorlagetank 1 auf ca. 70°C erwärmt und anschließend mit 0,1 Gew.-% Zitronensäure (33%ig, auf Raumtemperatur) versetzt und für 30 Sekunden intensiv gerührt und danach 10 min bei cirka 100 bis 150 UpM gerührt. Danach wird 0,3 Gew.-% Wasser hinzugegeben.

Das Gemisch aus lipoider Phase und verdünnter Zitronensäure wird anschließend in den Trenn-Separator gepumpt und dann bei einer Leistung von 200l/h die wässrige Phase A von der öligen Phase A getrennt. Die wässrige Phase A wird gesammelt und bis zur weiteren Verwendung gelagert. Die ölige Phase A wird zur weiteren Prozessierung in einen weiteren Vorlagetank (Vorlagetank 2) überführt. 125 ml der öligen Phase A wurden zur chemischen Analyse verwendet (FFA-Gehalt 0,46%, $H_2O$-Gehalt 0,53 %, Eisen-Gehalt 0,13 ppm, Phosphor-Gehalt 16,2 ppm).

Die so gewonnene ölige Phase A wird auf eine Prozesstemperatur von 40 - 45°C gebracht und ein ausreichendes Volumen an 8%iger Natriumcarbonat-Lösung hinzugegeben, so dass ein theoretischer Neutralisationsgrad der freien Fettsäuren von 90% erreicht wird. Anschließend wird mittels Ystral-Mischer für 30 Sekunden intensiv und vorzugsweise gaseintragsfrei gerührt und danach 10 min vorzugsweise gaseintragsfrei normal gerührt. Das resultierende Gemisch wird anschließend in den Trenn-Separator gepumpt und so bei einer Leistung von 200l/h die wässrige Phase B von der öligen Phase B getrennt. Die wässrige Phase B wird gesammelt und zur Extraktion der abgetrennten Glycoglycerolipide verwendet. Durch Extraktion mittels Chloroform wurden die Glycoglycerolipide aus der wäßrigen Phase B gewonnen. Die ölige Phase B wird zur weiteren Prozessierung wieder zurück in den Vorlagetank 1 überführt. 125 ml der öligen Phase B wurden zur chemischen Analyse verwendet (FFA-Gehalt 0,24%, $H_2O$-Gehalt 0,48 %, Eisen-Gehalt 0,03 ppm, Phosphor-Gehalt 2,25 ppm).

Die ölige Phase B wird auf eine Prozesstemperatur von 40°C bis 45°C gebracht und mit einem Volumen an 0,6 M Arginin-Lösung versetzt, so dass 1,5 mol Arginin je Mol freier Fettsäuren vorliegt und es bei der Zugabe nicht zu einem Eintrag von Luft kommt. Anschließend wird für 10 min schonend und lufteintragsfrei gerührt. Das resultierende Gemisch wird anschließend lufteintragsfrei in den Trenn-Separator gepumpt und so bei einer Leistung von 200l/h die wässrige Phase C von der öligen Phase C getrennt. Die wässrige Phase C und die ölige Phase C werden getrennt gesammelt. 125 ml der öligen Phase C wurden zur chemischen Analyse verwendet. Der Gehalt an freien Fettsäuren konnte auf 0,10 Gew.-% reduziert werden. Zudem wurde die Menge an Kalium und Calcium auf unter 0,4 ppm gesenkt ($K < 0,4$ ppm; $Ca < 0,5$ ppm) und die Menge an Phosphor bis auf 0,8 ppm und die Mengen an Eisen auf 0,02 ppm gesenkt.

**Beispiel 10E:**

Gemäß Beispiel 10D) wurde Rapsöl als lipoide Phase untersucht. Die Angaben sind in mg pro kg lipoide Phase außer bei FFA. Bei FFA sind die Angaben in Gew.-%. "Roh" bezeichnet die Ausgangswerte der lipoiden Phase. A2') bedeuten die Werte nach dem Schritt A2'). B1) bedeuten die Werte nach dem Schritt B1). E1) bedeuten die Werte nach dem Schritt E1).

| | Roh | A2') | B1) | E1) |
|---|---|---|---|---|
| FFA [%] | 0,58 | 0,55 | 0,28 | 0,18 |
| P [mg/kg] | 96,18 | 10,04 | 1,20 | 0,712 |
| Fe [mg/kg] | 0,64 | 0,15 | 0,032 | 0,012 |
| Ca [mg/kg] | 48,71 | 2,74 | 0,506 | 0,473 |
| Mg [mg/kg] | 8,62 | 0,56 | 0,131 | 0,109 |
| Cr [mg/kg] | 0,016 | 0,009 | 0,009 | 0,007 |
| Zn [mg/kg] | 0,167 | 0,027 | 0,015 | 0,007 |
| Mn [mg/kg] | 0,136 | 0,015 | 0,004 | 0,001 |

| Probenname | H2O | FFA % | P mg/kg | Fe mg/kg | Ca mg/kg | Mg mg/kg |
|---|---|---|---|---|---|---|
| Roh - NC | 0,05 | 0,54 | 78,32 | 0,53 | 33,04 | 5,70 |
| NC Degumming = A2' | 0,53 | 0,48 | 16,57 | 0,15 | 1,78 | 0,28 |
| NC Lipoide = B1) | 0,49 | 0,25 | 2,21 | 0,15 | 0,32 | 0,07 |
| NC Native Neutra =E1) | 0,59 | 0,23 | 0,90 | 0,04 | 0,34 | 0,09 |

**Beispiel 10F:**

Gemäß Beispiel 10C) wurde Rapsöl als lipoide Phase untersucht. Die Angaben sind in mg pro kg lipoide Phase außer bei FFA. Bei FFA sind die Angaben in Gew.-%. "Roh" bezeichnet die Ausgangswerte der lipoiden Phase. A2') bedeuten die Werte nach dem Schritt A2'). B1) bedeuten die Werte nach dem Schritt B1). E1) bedeuten die Werte nach dem Schritt E1).

| | Roh | A2') | B1) | E1) |
|---|---|---|---|---|
| Mg [mg/kg] | 8,62 | 0,511 | 0,125 | 0,093 |
| Cr [mg/kg] | 0,016 | 0,007 | 0,009 | 0,006 |
| Mn [mg/kg] | 0,136 | 0,016 | 0,004 | 0,002 |

| Probenname | H2O | FFA % | P mg/kg | Fe mg/kg | Ca mg/kg | Mg mg/kg |
|---|---|---|---|---|---|---|
| Roh-NA-Mittel | 0,05 | 0,43 | 52,52 | 0,60 | 31,33 | 5,43 |
| NA-Degumming =A2' | 0,26 | 0,43 | 12,49 | 0,17 | 1,85 | 0,40 |
| NA-Lipoide = B1 | 0,24 | 0,44 | 5,79 | 0,09 | 0,89 | 0,25 |
| NA-Native Neutra =E1 | 0,37 | 0,13 | 0,80 | 0,00 | 0,21 | 0,07 |

## Patentansprüche

1. Verfahren zur hydrolysearmen Abtrennung von Glycoglycerolipiden aus einer lipoiden Phase, welche Glycoglycerolipide und Acylglyceride enthält, umfassend die Schritte:

A1) Bereitstellung einer lipoiden Phase enthaltend Glycoglycerolipide und Acylglyceride,
B1) Versetzen der lipoiden Phase mit einer wässrigen Phase, enthaltend Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$), Acetat ($CH_3COO^-$), Borat ($BO_3^{3-}$) und/oder Tartrat ($C_4H_4O_6^{2-}$) bildet,
C1) Durchmischung der lipoiden Phase und der wässrigen Phase,
D1) und Abtrennung der wässrigen glycoglycerolipidreichen Phase und Erhalt einer glycoglycerolipidarmen lipoiden Phase.

**2.** Verfahren gemäß Anspruch 1, umfassend nach Schritt D1) den folgenden Schritt D2):

D2) Gewinnung der Glycoglycerolipide aus der abgetrennten wässrigen glycoglycerolipidreichen Phase.

**3.** Verfahren gemäß Anspruch 1 oder 2, umfassend nach Schritt D1) oder sofern vorhanden nach Schritt D2) den folgenden Schritt E1):

E1) Versetzen der lipoiden glycoglycerolipidarmen Phase mit einer wässrigen Phase, enthaltend mindestens eine Verbindung, welche mindestens eine Amidinogruppe und/oder mindestens eine Guanidinogruppe aufweist, nachfolgendes Durchmischen der lipoiden glycoglycerolipidarmen Phase und der wässrigen Phase und Abtrennung der wässrigen Phase.

**4.** Verfahren gemäß einem der Ansprüche 1 - 3, wobei es sich bei den Glycoglycerolipiden um lipophile Glycoglycerolipide mit einem Lipophilitätsindex GL von 1,0 ≤ GL ≤ 6,0 handelt, wobei sich der Lipophilitätsindex GL gemäß folgender Formel berechnet:

$$GL = \frac{\text{Summe der Kohlenstoffatome der Acylreste}}{\text{Summe der Hydroxy- und Aminogruppen}}$$

**5.** Verfahren gemäß einem der Ansprüche 1 - 4, wobei es sich bei den Glycoglycerolipiden um Glycosyldiacylglycerine, Glycosylacylalkylglycerine und Glycosyldialkylglycerine handelt.

**6.** Verfahren gemäß einem der Ansprüche 1 - 5 zum Gewinnung von hydrolysearmen Glycoglycerolipiden und Glycosphingolipiden aus einer lipoiden Phase, welche Glycoglycerolipide und Glycosphingolipide und Acylglyceride enthält, umfassend die Schritte:

A1) Bereitstellung einer lipoiden Phase, enthaltend Glycoglycerolipide, Glycosphingolipide und Acylglyceride,
B1) Versetzen der lipoiden Phase mit einer wässrigen Phase, enthaltend Anionen mindestens eines Salzes, welches in Wasser bei 20°C eine Löslichkeit von mindestens 30 g/L besitzt und bei der Dissoziation in Wasser Carbonat ($CO_3^{2-}$), Hydrogencarbonat ($HCO_3^-$), Metasilicat ($SiO_3^{2-}$), Orthosilicat ($SiO_4^{4-}$), Disilicat ($Si_2O_5^{2-}$), Trisilicat ($Si_3O_7^{2-}$), Acetat ($CH_3COO^-$), Borat ($BO_3^{3-}$) und/oder Tartrat ($C_4H_4O_6^{2-}$) bildet,
C1) Durchmischung der lipoiden Phase und der wässrigen Phase,
D1) und Abtrennung der wässrigen glycoglycerolipidreichen und/oder glycosphingolipidreichen Phase und Erhalt einer lipoiden glycoglycerolipidarmen und/oder glycosphingolipidarmen Phase.

**7.** Verfahren gemäß Anspruch 6, wobei es sich bei den Glycosphingolipiden um lipophile Glycosphingolipide mit einem Lipophilitätsindex SL von 1,0 ≤ SL ≤ 7,0 handelt, wobei sich der Lipophilitätsindex SL gemäß folgender Formel berechnet:

$$SL = \frac{\text{Summe der Kohlenstoffatome des Ceramidrestes}}{\text{Summe der Hydroxy- und Amino- und Amidgruppen}}$$

**8.** Verfahren gemäß einem der Ansprüche 1 - 7, wobei die Glycoglycerolipide keine Carboxylat-, Sulfat-, Sulfonat- oder Phosphatgruppe(n) enthalten.

9. Verfahren gemäß einem der Ansprüche 1 - 8, wobei die lipoide Phase in Schritt A1) ferner Sterylglykoside enthält, welche in der wässrigen Phase in Schritt D1) abgetrennt werden.

10. Verfahren gemäß einem der Ansprüche 1 - 9, wobei in Schritt B1) die lipoide Phase mit einer wässrigen Phase versetzt wird, welche Kationen eines Salzes enthält, welches in Wasser bei 20°C eine Löslichkeit von mindestens 30 g/L besitzt und bei der Dissoziation in Wasser $Mg^{2+}$, $Ca^{2+}$, $Ti^{2+}$, $Ti^{4+}$, $Co^{2+}$, $Co^{3+}$, $Ni^{2+}$, $Cu^{2+}$, $Zn^{2+}$, $Sn^{2+}$ oder $Sn^{4+}$ Ionen bildet.

11. Verfahren gemäß einem der Ansprüche 1 - 10, wobei sofern in der lipoiden Phase Phospholipoide und/oder Fettsäuren enthalten sind, sich phospholipidfreie und/oder fettsäurenfreie glycoglycerolipidreiche Phasen gewinnen lassen, sofern nach Schritt A1) und vor Schritt B1) der folgende Schritt A2) oder A2') durchgeführt wird:

A2) Versetzen der lipoiden Phase mit Wasser als wässrige Phase, nachfolgendes Durchmischen der lipoiden Phase und der wässrigen Phase und Abtrennung der wässrigen Phase
oder
A2') Versetzen der lipoiden Phase mit einer wässrigen Carbonsäure-Lösung oder einer wässrigen Lösung einer anorganischen Säure mit einem pH-Wert zwischen 3,0 bis 5,0 als wässrige Phase, nachfolgendes Durchmischen der lipoiden Phase und der wässrigen Phase und Abtrennung der wässrigen Phase.

12. Verfahren gemäß einem der Ansprüche 1 - 11, wobei lipoide Phase und wässrige Phase in Schritt C1) und/oder A2) oder A2') intensiv gemischt werden.

**Claims**

1. A method for the hydrolysis-poor separation of glycoglycerolipids from a lipoid phase, which contains glycoglycerolipids and acylglycerides, comprising the steps:

A1) providing a lipoid phase containing glycoglycerolipids and acylglycerides,
B1) adding to the lipoid phase an aqueous phase containing anions of at least one salt, which has a solubility of at least 30 g/l in water at 20°C and which, upon dissociation in water, forms carbonate ($CO_3^{2-}$), hydrogen carbonate ($HCO_3^-$), metasilicate ($SiO_3^{2-}$), orthosilicate ($SiO_4^{4-}$), disilicate ($Si_2O_5^{2-}$), trisilicate ($Si_3O_7^{2-}$), acetate ($CH_3COO^-$), borate ($BO_3^{3-}$) and/or tartrate ($C_4H_4O_6^{2-}$)
C1) mixing the lipoid phase and the aqueous phase, and
D1) separating the glycoglycerolipid-rich aqueous phase and obtaining a glycoglycerolipid-poor lipoid phase.

2. The method according to claim 1, comprising the following step D2) after step D1):

D2) recovering the glycoglycerolipids from the separated aqueous glycoglycerolipid-rich phase.

3. The method according to claim 1 or 2, comprising the following step E1) after step D1) or, if present after step D2):

E1) adding to the glycoglycerolipid-poor lipoid phase an aqueous phase containing at least one compound which has at least one amidino group and/or at least one guanidino group, followed by mixing the lipoid glycoglycerolipid-poor phase and the aqueous phase and separating the aqueous phase.

4. The method according to any one of claims 1 - 3, wherein the glycoglycerolipids are lipophilic glycoglycerolipids having a lipophilicity index GL of $1.0 \leq GL \leq 6.0$, wherein the lipophilicity index GL is calculated according to the formula:

$$GL = \frac{\text{Sum of the carbon atoms of the acyl residues}}{\text{Sum of hydroxy and amino groups}}$$

5. The method according to one of claims 1 - 4, wherein the glycoglycerolipids are glycosyldiacylglycerols, glycosylacylalkylglycerols and glycosyldialkylglycerols.

6. The method according to any one of claims 1 - 5 for recovering hydrolysis-poor glycoglycerolipids and glycosphingolipids from a lipoid phase which contains glycoglycerolipids and glycosphingolipids and acylglycerides, comprising

the steps of:

A1) providing a lipoid phase containing glycoglycerolipids, glycosphingolipids and acylglycerides,
B1) adding to the lipoid phase an aqueous phase containing anions of at least one salt which has a solubility of at least 30 g / l in water at 20°C and which upon dissociation in water, forms carbonate ($CO_3^{2-}$), hydrogen carbonate ($HCO_3^-$), metasilicate ($SiO_3^{2-}$), orthosilicate ($SiO_4^{4-}$), disilicate ($Si_2O_5^{2-}$), trisilicate ($Si_3O_7^{2-}$), acetate ($CH_3COO^-$), borate ($BO_3^{3-}$) and / or tartrate ($C_4H_4O_6^{2-}$),
C1) mixing the lipoid phase and the aqueous phase, and
D1) separating the glycoglycerolipid-rich and/or glycosphingolipid-rich aqueous phase and obtaining a glycoglycerolipid-poor and/or glycosphingolipid-poor lipoid phase.

**7.** The method according to claim 6, wherein the glycosphingolipids are lipophilic glycosphingolipids having a lipophilicity index SL of $1.0 \leq SL \leq 7.0$, wherein the lipophilicity index SL is calculated according to the following formula:

$$SL = \frac{\text{Sum of carbon atoms of the ceramide residue}}{\text{Sum of hydroxy and amino and amido groups}}$$

**8.** The method according to any one of claims 1 - 7, wherein the glycoglycerolipids do not contain any carboxylate, sulfate, sulfonate or phosphate group(s).

**9.** The method according to any one of claims 1 - 8, wherein the lipoid phase in step A1) further comprises sterylglycosides which are separated in the aqueous phase in step D1).

**10.** The method according to one of claims 1 - 9, wherein in step B1) an aqueous phase containing cations of a salt which has a solubility of at least 30 g/l in water at 20°C and in the dissociation in water forms $Mg^{2+}$, $Ca^{2+}$, $Ti^{2+}$, $Ti^{4+}$, $Co^{2+}$, $Co^{3+}$, $Ni^{2+}$, $Cu^{2+}$, $Zn^{2+}$, $Sn^{2+}$ or $Sn^{4+}$ ions is added to the lipoid phase.

**11.** The method according to any one of claims 1 - 10, wherein, if phospholipids and/or fatty acids are contained in the lipoid phase, a phospholipid-free and/or fatty acid-free glycoglycerolipid-rich phases can be obtained if the following step A2) or A2') is carried out after step A1) and before step B1)

A2) adding water as aqueous phase to the lipoid phase, followed by mixing the lipoid phase and the aqueous phase and separating the aqueous phase,
or
A2') adding an aqueous carboxylic acid solution or an aqueous solution of an inorganic acid having a pH between 3.0 and 5.0 as the aqueous phase to the lipoid phase, followed by mixing the lipoid phase and the aqueous phase and separating the aqueous phase.

**12.** The method according to one of claims 1 - 11, wherein the lipoid phase and the aqueous phase are intensively mixed in step C1) and/or A2) or A2').

**Revendications**

**1.** Procédé de séparation à faible hydrolyse de glycoglycérolipides à partir d'une phase lipidique contenant des glycoglycérolipides et des acylglycérols, comprenant les étapes suivantes :

A1) préparation d'une phase lipidique contenant des glycoglycérolipides et des acylglycérols,
B1) mélange de la phase lipidique avec une phase aqueuse contenant des anions d'au moins un sel ayant une solubilité d'au moins 30 g/l dans de l'eau à 20 °C et formant du carbonate ($CO_3^{2-}$), de l'hydrogénocarbonate ($HCO_3^-$), du métasilicate ($SiO_3^{2-}$), de l'orthosilicate ($SiO_4^{4}$), du disilicate ($Si_2O_5^{2-}$), du trisilicate ($Si_3O_7^{2-}$), de l'acétate ($CH_3COO^-$), du borate ($BO_3^{3-}$) et/ou du tartrate ($C_4H_4O_6^{2-}$) lors de sa dissociation dans l'eau,
C1) mélange de la phase lipidique et de la phase aqueuse,
D1) et séparation de la phase aqueuse riche en glycoglycérolipides et obtention d'une phase lipidique pauvre en glycoglycérolipides.

**2.** Procédé conforme à la revendication 1, comprenant, après l'étape D1), l'étape D2) suivante :

D2) obtention des glycoglycérolipides issus de la phase aqueuse isolée, riche en glycoglycérolipides.

**3.** Procédé conforme à l'une des revendications 1 ou 2, comprenant, après l'étape D1) ou l'étape D2) le cas échéant, l'étape E1) suivante :

E1) mélange de la phase lipidique pauvre en glycoglycérolipides avec une phase aqueuse comprenant au moins une liaison présentant au moins un groupement amidine et/ou au moins un groupement guanidine, puis mélange de la phase lipidique pauvre en glycoglycérolipides et de la phase aqueuse et séparation de la phase aqueuse.

**4.** Procédé conforme à l'une des revendications 1 à 3, où les glycoglycérolipides sont lipophiles et présentent un indice de lipophilie GL compris entre $1{,}0 \leq GL \leq 6{,}0$ ; ledit indice de lipophilie GL étant calculé selon la formule :

$$GL = \frac{\text{Somme des atomes de carbone du reste des groupements acyles}}{\text{Somme des groupements hydroxyles et amines}}$$

**5.** Procédé conforme à l'une des revendications 1 à 4, où les glycoglycérolipides sont composés de glycosyl-diacylglycérol, de glycosyl-acylalkylglycérol et de glycosyldialkylglycérol.

**6.** Procédé conforme à l'une des revendications 1 à 5 permettant l'obtention de glycoglycérolipides à faible hydrolyse et de glycosphingolipides à partir d'une phase lipidique comportant des glycoglycérolipides, des glycosphingolipides et des acylglycérols, comprenant les étapes suivantes :

A1) préparation d'une phase lipidique contenant des glycoglycérolipides, des glycosphingolipides et des acylglycérols,
B1) mélange de la phase lipidique avec une phase aqueuse contenant des anions d'au moins un sel ayant une solubilité d'au moins 30 g/l dans de l'eau à 20 °C et formant du carbonate ($CO_3^{2-}$), de l'hydrogénocarbonate ($HCO_3^-$), du métasilicate ($SiO_3^{2-}$), de l'orthosilicate ($SiO_4^{4}$), du disilicate ($Si_2O_5^{2-}$), du trisilicate ($Si_3O_7\ ^{2-}$), de l'acétate ($CH_3COO^-$), du borate ($BO_3^{3-}$) et/ou du tartrate ($C_4H_4O_6^{2-}$) lors de sa dissociation dans l'eau,
C1) mélange de la phase lipidique et de la phase aqueuse,
D1) séparation de la phase aqueuse riche en glycoglycérolipides et/ou de la phase riche en glycosphingolipides et obtention d'une phase lipidique pauvre en glycoglycérolipides et/ou pauvre en glycosphingolipides.

**7.** Procédé conforme à la revendication 6, où les glycosphingolipides sont lipophiles et présentent un indice de lipophilie SL compris entre $1{,}0 \leq SL \leq 7{,}0$ ; ledit indice de lipophilie SL étant calculé selon la formule :

$$SL = \frac{\text{Somme des atomes de carbone du reste des céramides}}{\text{Somme des groupements hydroxyles, amines et amides}}$$

**8.** Procédé conforme à l'une des revendications 1 à 7, où les glycoglycérolipides ne présentent pas de groupements carboxylate, sulfate, sulfonate ou phosphate.

**9.** Procédé conforme à l'une des revendications 1 à 8, où la phase lipidique de l'étape A1) comprend également des stérylglucosides qui seront isolés dans la phase aqueuse lors de l'étape D1).

**10.** Procédé conforme à l'une des revendications 1 à 9, où, lors de l'étape B1), la phase lipidique est mélangée à une phase aqueuse contenant des cations d'un sel présentant une solubilité d'au moins 30 g/l dans de l'eau à 20 °C et formant des ions $Mg^{2+}$, $Ca^{2+}$, $Ti^{2+}$, $Ti^{4+}$, $Co^{2+}$, $Co^{3+}$, $Ni^{2+}$, $Cu^{2+}$, $Zn^{2+}$, $Sn^{2+}$ ou $Sn^{4+}$ lors de leur dissociation dans l'eau.

**11.** Procédé conforme à l'une des revendications 1 à 10, où, si la phase lipidique contient des phospholipoïdes et/ou des acides gras, des phases sans phospholipides et/ou sans acides gras sont obtenues si tant est qu'entre l'étape A1) et l'étape B1), l'étape A2) ou A2') explicitée ci-après est effectuée :

A2) mélange de la phase lipidique avec de l'eau en tant que phase aqueuse, puis mélange de la phase lipidique et de la phase aqueuse, et séparation de la phase aqueuse
ou

A2') mélange de la phase lipidique avec une solution d'acide carboxylique aqueuse ou une solution aqueuse d'un acide inorganique dont le pH se situe entre 3,0 et 5,0 en tant que phase aqueuse, puis mélange de la phase lipidique et de la phase aqueuse, et séparation de la phase aqueuse.

**12.** Procédé conforme à l'une des revendications 1 à 11, où la phase lipidique et la phase aqueuse dans l'étape C1) et/ou A2) ou A2') sont mélangées vigoureusement.

Fig. 1

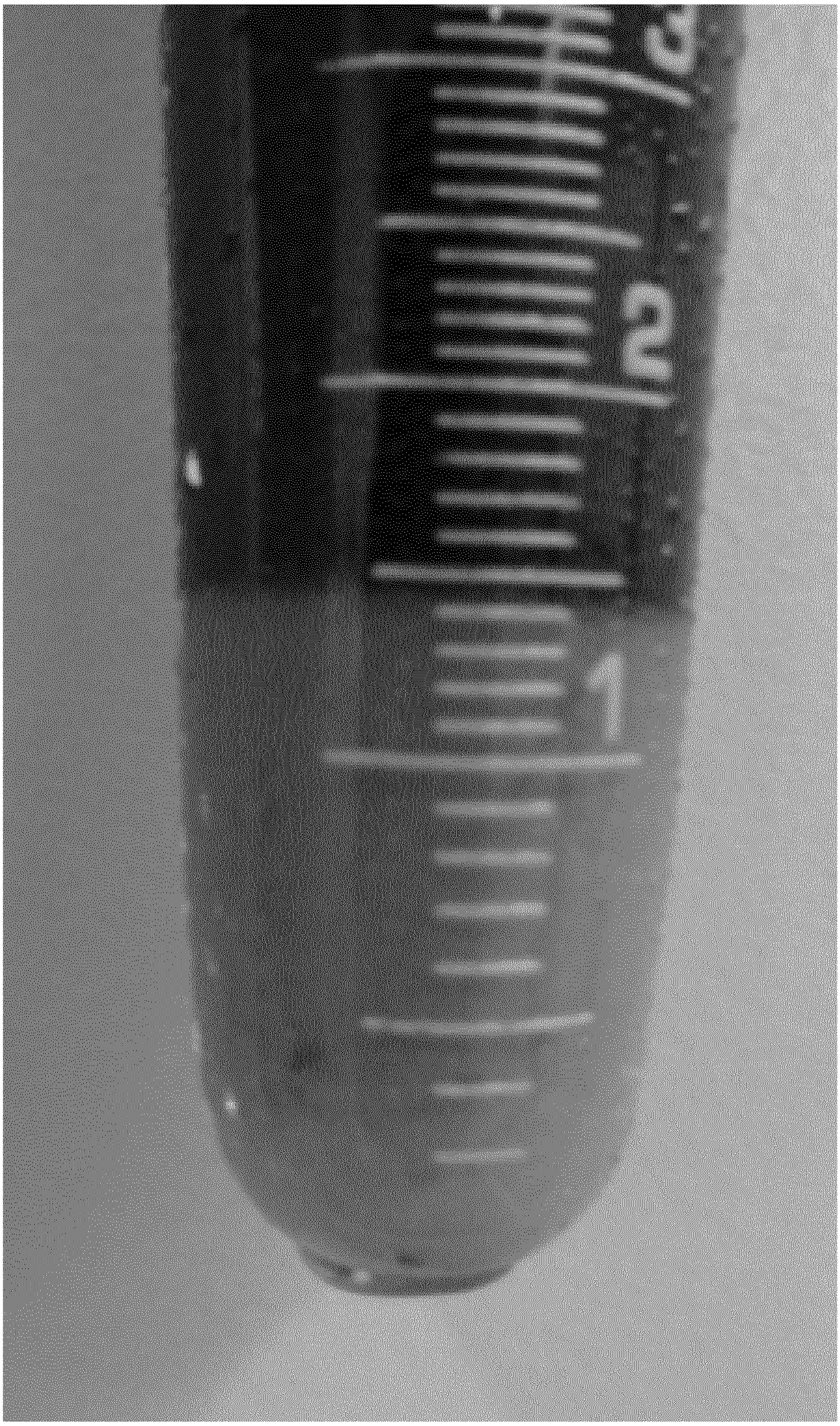

Fig. 2

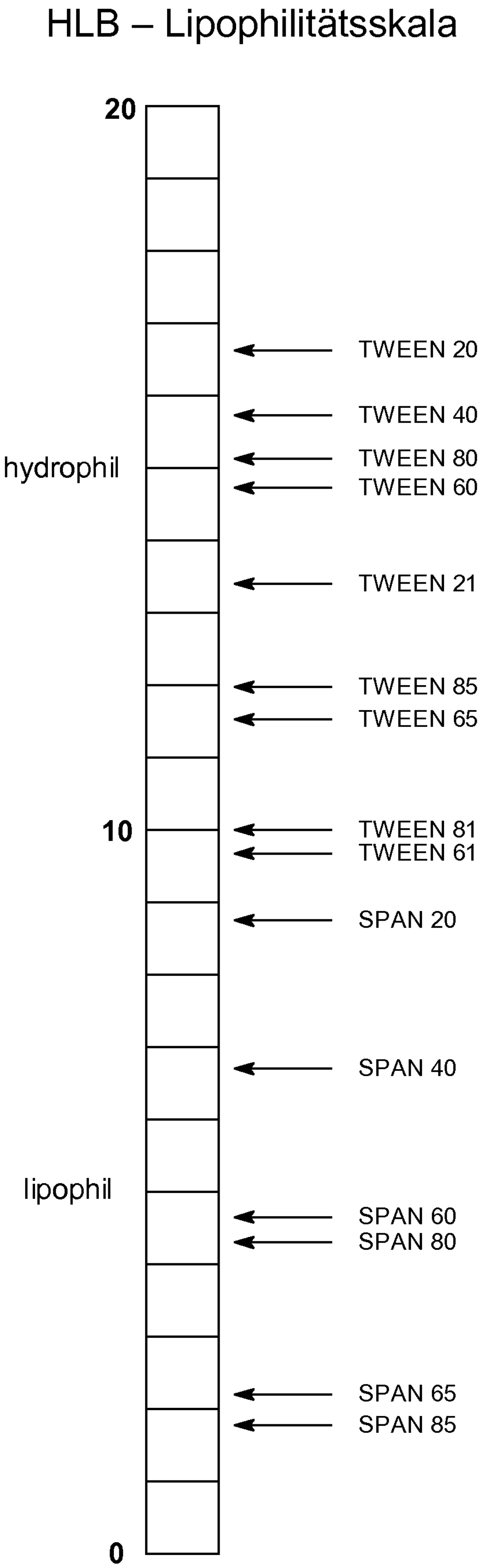
HLB – Lipophilitätsskala
20
hydrophil
10
lipophil
0
TWEEN 20
TWEEN 40
TWEEN 80
TWEEN 60
TWEEN 21
TWEEN 85
TWEEN 65
TWEEN 81
TWEEN 61
SPAN 20
SPAN 40
SPAN 60
SPAN 80
SPAN 65
SPAN 85

Fig. 3

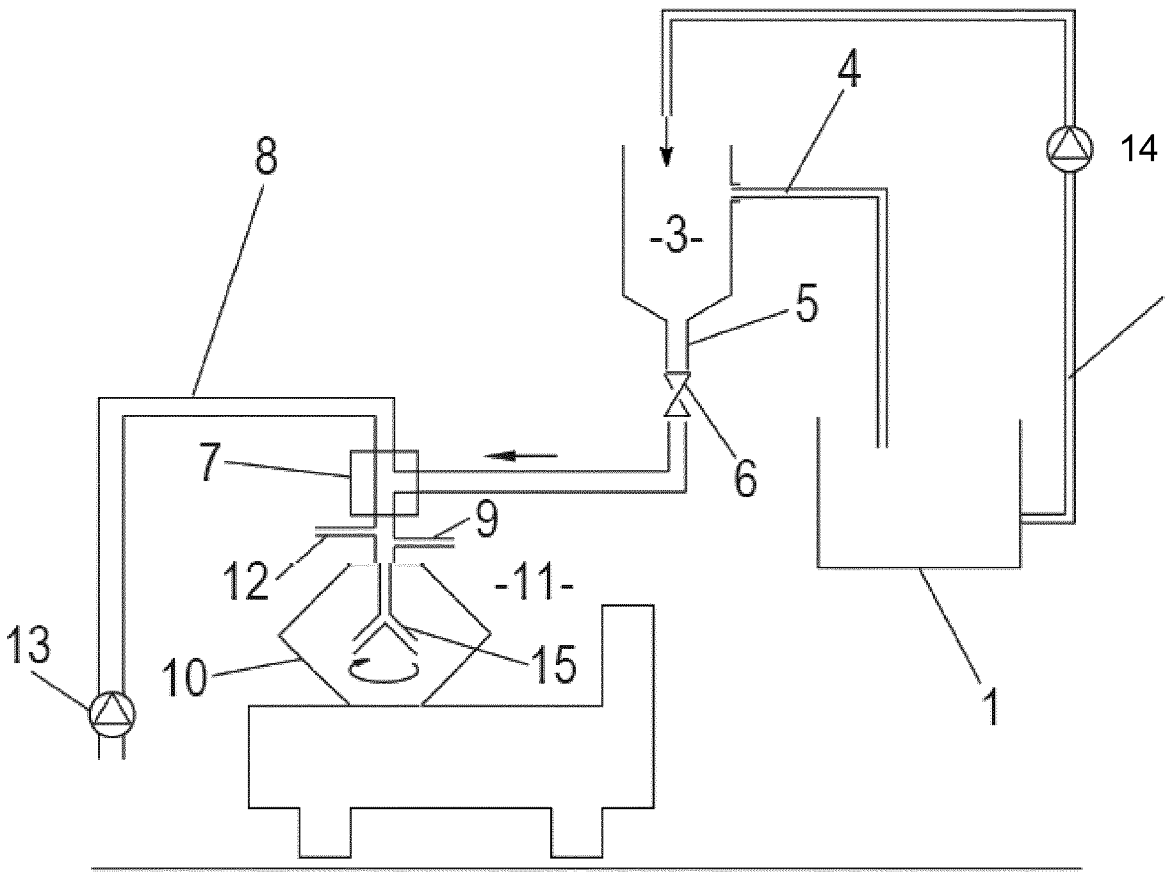

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6953849 B
- WO 2012109642 A1
- EP 2735605 A1
- WO 2011160857 A2